# EUROPEAN PATENT SPECIFICATION

(11) **EP 0 772 601 B1**
(45) Date of publication and mention of the grant of the patent: **18.09.2002**
(21) Application number: 95929327.5
(22) Date of filing: 27.07.1995
(51) Int. Cl.: C07D 233/54, A61K 31/415, C07D 233/84, C07D 401/04, C07D 401/12, C07D 405/04, C07D 409/04

(54) **4,5-SUBSTITUTED IMIDAZOLYL COMPOUNDS FOR THE TREATMENT OF INFLAMMATION**
4,5-SUBSTITUIERTE IMIDAZOLYLVERBINDUNGEN ZUR BEHANDLUNG VON ENTZUNDUNGEN
COMPOSES D'IMIDAZOLYLE SUBSTITUES EN POSITIONS 4 ET 5, CONVENANT AU TRAITEMENT DE L'INFLAMMATION

(30) Priority: 28.07.1994 US 281903
(43) Date of publication of application: 14.05.1997
(60) Divisional application: 02002833.8
(73) Proprietor: G.D. SEARLE & CO., Chicago, IL 60680-5110 (US)
(72) Inventor: WEIER, Richard, M., Lake Bluff, IL 60044 (US); COLLINS, Paul, W., Deerfield, IL 60015 (US); STEALEY, Michael, A., Libertyville, IL 60048 (US); BARTA, Thomas E., Apartment 3-W, Evanston, IL 60602 (US); HUFF, Renee, M., Park Ridge, IL 60068 (US)
(74) Representative: Beil, Hans Christoph, Dr.
(86) International application number: PCT/US95/09505
(87) International publication number: WO 96/003387

(56) References cited:
- EP-A- 0 032 113
- EP-A- 0 044 486
- WO-A-94/15932
- WO-A-95/00501
- CH-A- 561 717
- US-A- 3 901 908
- US-A- 4 175 127
- US-A- 4 503 065

## Description

This invention is in the field of antiinflammatory pharmaceutical agents and specifically related to compounds, compositions and the use thereof for preparing a medicament for treating inflammation and inflammation-associated disorders, such as arthritis.

### BACKGROUND OF THE INVENTION

Prostaglandins play a major role in the inflammation process and the inhibition of prostaglandin production, especially production of PGG₂, PGH₂ and PGE₂, has been a common target of antiinflammatory drug discovery. However, common non-steroidal antiinflammatory drugs (NSAIDs) that are active in reducing the prostaglandin-induced pain and swelling associated with the inflammation process are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved.

Previous MSAIDs have been found to prevent the production of prostaglandins by inhibiting enzymes in the human arachidonic acid/prostaglandin pathway, including the enzyme cyclooxygenase (COX). The recent discovery of an inducible enzyme associated with inflammation (named "cyclooxygenase-2 (COX-2)" or "prostaglandin G/H synthase II") provides a viable target of inhibition which more effectively reduces inflammation and produces fewer and less drastic side effects.

The references below that disclose antiinflammatory activity, show continuing efforts to find a safe and effective antiinflammatory agent. The novel imidazoles disclosed herein are such safe and also effective antiinflammatory agents furthering such efforts. The invention compounds are found to show usefulness *in vivo* as antiinflammatory agents with minimal side effects. The substituted imidazoles disclosed herein preferably selectively inhibit cyclooxygenase-2 over cyclooxygenase-1.

US-A-4,822,805, to Takasugi et al., describes pyridyl-imidazoles as antiinflammatory agents. Specifically, 2-[2-methoxy-4-(methylsulfonyl)phenyl]-4-methyl-5-(3-pyridyl)imidazole is described.

US-A-4,188,397, to Hill, describes 2,2-alkyldiylbis(thio)bis(imidazoles) with substituted phenyl radicals at the 4 and 5 positions of the imidazole rings as having antiinflammatory activity. Specifically, imidazoles having phenyl radicals substituted with methoxy, methylthio, trifluoromethylhalo and methylenedioxy are described.

T. Sharpe et al. [*J.Med. Chem.*, **28**, 1188 (1985)] describe antiarthritic activity of 4,5-diaryl-2-(substituted thio)-1H-imidazoles.

US-A-4,686,231, to Bender et al., describes 4,5-diaryl-1H-imidazoles as inhibiting the 5-lipoxygenase pathway for the treatment of arthritis. 1-Methyl-4,5-bis(methoxyphenyl)-2-methylthio-1H-imidazole is specifically described.

AU-A-8665565 describes cyano-2,2-bis(imidazoles) as having antihypertensive agents. WO-A-9314082, published July 22, 1993, describes 1-pyridyl-2-phenyl-imidazole derivatives for the treatment of interleukin-1 mediated diseases.

H. Greenberg et al. [*J.Org.Chem*., **31**, 3951 (1966)] describe 4-(2-oxo-5-phenyl-4-imidazolin-4-yl)benzenesulfonamide in a study of the bromination reaction thereof.

T. van Es and O. Backeberg [*J. Chem. Soc*., 1363 (1963)] describe the synthesis of 4,4'-imidazol-4,5-diyl]bis(benzenesulfonamide) for use in a study of substitution reactions on phenyl radicals.

EP-A-0 372,445, published June 13, 1990, describes 4,5-diaryl-1H-imidazoles as having antihypercholesterolemic activity. N-[[5-(4-Methylsulfonylphenyl)-4-phenyl-1H-imidazol-2-yl]thio]pentyl-N-octyl-N-heptylurea is specifically described. US-A-5,364,875, to Wilde, describes substituted imidazoles for the treatment of atherosclerosis. US-A-5,358,946, to Wilde, describes substituted imidazoles for the treatment of atherosclerosis. US-A-5,310,748, to Billheimer et al., describes substituted imidazoles for the treatment of atherosclerosis. US-A-5,166,214, to Billheimer et al., describes substituted imidazoles for the treatment of atherosclerosis. T. Maduskuie et al., [*J. Med. Chem*., **38,** 1067 (1995)] describes substituted imidazoles as acyl-CoA:Cholesterol Acyltransferase inhibitors

US-A-4,503,065, to Wilkerson, describes 4,5-diaryl-2-halo-1H-imidazoles as being antiinflammatory. Specifically, 1-(1-ethoxyethyl)-2-fluoro-4,5-bis(4-methylsulfonylphenyl)-1H-imidazole is described.

J. Lombardino (*J. Med. Chem*., **17**, 1182 (1974)) describes trisubstituted imidazoles as being antiinflammatory, and specifically 4,5-bis(4-methoxyphenyl)-2-trifluoromethyl-1H-imidazole. Similarly, US-A-3,707,475, to Lombardino, describes antiinflammatory 4,5-diarylimidazoles. Specifically, 4-chlorophenyl-5-(4-methylthiophenyl)-2-trifluoromethyl-lH-imidazole is described.

US-A-4,472,422, to Whitney, describes 4,5-diaryl-1H-imidazole-2-methanamines as having antiinflammatory activity. Specifically, 5-(4-fluorophenyl)-4-(4-methylsulfonylphenyl)-α,α-bis(trifluoromethyl)-1H-imidazole-2-methanamine is described.

US-A-4,372,964, to Whitney, describes 4,5-diaryl-1H-imidazole-2-methanols as having antiinflammatory activity. Specifically, 5-(4-fluorophenyl)-4-(4-methylsulfonylphenyl)-α,α-bis[trifluoromethyl)-1H-imidazole-2-methanol is described. Additionally, Whitney describes 1-[4,5-diaryl-1H-imidazol-2-yl]-2,2,2-trifluoro-1-ethanones as having antiinflammatory activity. Specifically, 1-[5-(4-fluorophenyl)-4-(4-methylsulfonylphenyl)-1H-imidazol-2-yl]-2,2,2-trifluoro-1-ethanone is described.

US-A-4,576,958, to Wexler, describes 4-phenyl-5-(4-methylsulfonylphenyl)-1H-imidazoles as having antiinflammatory activity. Specifically, 4-(4-fluorophenyl)-5-(4-methylsulfonylphenyl)-α,α -bis(trifluoromethyl)-1H-imidazole-2-methanol and 4-(4-fluorophenyl)-5-(4-methylsulfonylphenyl)-α,α -bis(trifluoromethyl)-1H-imidazole-2-methanol, acetate is described. US-A-4,632,930, to Carini et al., claims cycloalkyl substituted imidazoles, and specifically 4-cyclopentyl-5-(4-methylsulfonyl)-α,α-bis(trifluoromethyl)-1H-imidazole-2-methanol, as having antihypertensive properties.

US-A-3,901,908, to Fitzi, et al., describes 2-alkyl-4,5-bis(substituted phenyl)-1H-imidazoles. Specifically, 2-*tert*-butyl-4-(4-methylsulfonylphenyl)-5-phenyl-1H-imidazole is described. FR-A-2,081,407 describes 4,5-phenyimidazoles as antiinflammatory agents.

4,5-Diarylimidazoles have been described in WO-A-9500501, published January 5, 1995, as having antiinflammatory activity.

The invention's imidazolyl compounds are found to show usefulness *in vivo* as antiinflammatory agents with minimal side effects.

The US-A-4175127 discloses pyridyl-substituted 2,3-Dihydroimidazo[2,1-b]-thiazoles which have anti-arthritic activity.

The CH-A-561717 describes a process for preparing pyridyl or pyrazolyles-substituted imidazoles which are useful as anti-inflammatory agents.

The EP-A-0044486 is concerned with anti-inflammatory-4,5-diaryl-alpha-polyfluoroalkyl-1H-imidazole-2-methanamines.

WO-A-9415932 discloses 3,4-diarylthiophene/furane- and pyrrole-compounds which exhibit a COX-2-selectivity.

### Description of the invention

A class of substituted imidazolyl compounds useful in preparing a medicament for treating inflammation-related disorders is defined by Formula I 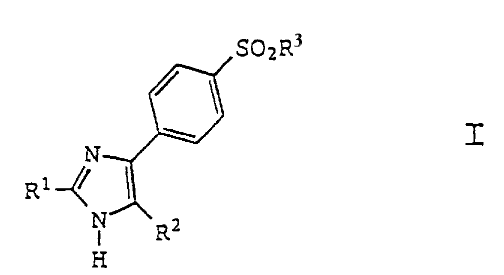 wherein R¹ is selected from C₁-C₁₀-alkyl, C₁-C₆-haloalkyl, aryl-C₁-C₆-alkyl, heterocyclic-C₁-C₆-alkyl, heteroaryl-C₁-C₆-alkyl, formyl, cyano, mercapto, C₁-C₆-alkoxy, C₁-C₆-alkylthio, C₁-C₆-alkylthio-C₁-C₆-alkyl, C₁-C₆-alkylsulfonyl, C₁-C₆-haloalkylsulfonyl, halo, C₁-C₆-hydroxyalkyl, C₁-C₆-alkoxy-C₁-C₆-alkyl, C₂-C₆-alkenyloxy-C₁-C₆-alkyl, C₁-C₆-alkylcarbonyl, arylcarbonyl, aryl-C₁-C₆-alkylcarbonyl, cyano-C₁-C₆-alkyl, aryl-C₂-C₆-alkenyl, C₁-C₆-aminoalkyl, C₁-C₆-alkylamino-C₁-C₆-alkyl, N-arylamino-C₁-C₆-alkyl, N-C₁-C₆-alkyl-N-arylamino-C₁-C₆-alkyl, carboxy-C₁-C₆-alkyl, C₁-C₆-alkoxycarbonyl-C₁-C₆-alkyl, C₁-C₆-alkoxycarbonyl, C₁-C₆-haloalkylcarbonyl, carboxyl, aminocarbonyl, C₁-C₆-alkylaminocarbonyl, mono or die C₁-C₆-alkylaminocarbonyl-C₁-C₆-alkyl, aryl-C₁-C₆-alkoxy, aryl-C₁-C₆-alkylthio, phenylsulfonyl, heteroaryl-C₁-C₆-alkoxy, heteroaryl-C₁-C₆-alkylthio, heteroaryl-C₁-C₆-alkoxy-C₁-C₆-alkyl, heteroaryloxy-C₁-C₆-alkyl, heteroarylthio-C₁-C₆-alkyl, heteroaryl-C₁-C₆-alkylthio-C₁-C₆-alkyl, heteroaryloxy, heteroarylthio, aryl-C₁-C₆-thioalkyl, aryloxy-C₁-C₆-alkyl, arylthio, aryloxy, aryl-C₁-C₆-alkylthio-C₁-C₆-alkyl, aryl-C₁-C₆-alkoxy-C₁-C₆-alkyl, C₁-C₆-alkoxyaryl-C₁-C₆-alkoxy-C₁-C₆-alkyl, aryl and heteroaryl, wherein the aryl and heteroaryl radicals are optionally substituted at a substitutable position with one or more radicals selected from halo, C₁-C₆-alkylthio, C₁-C₆-alkylsulfinyl, C₁-C₁₀-alkyl, cyano, C₁-C₆-haloalkyl, hydroxyl, C₁-C₆-alkoxy, C₁-C₆-hydroxyalkyl and C₁-C₆-haloalkoxy; wherein R² is selected from heteroaryl, and aryl, wherein the heteroaryl, and aryl radicals are substituted with one or more radicals selected from hydrido, halo, C₁-C₆-alkylthio, C₁-C₆-alkylsulfinyl, C₁-C₆-alkyl, cyano, carboxyl, C₁-C₆-alkoxycarbonyl, C₁-C₆-haloalkyl, hydroxyl, C₁-C₆-alkoxy, C₁-C₆-hydroxyalkyl, C₁-C₆-alkoxy-C₁-C₆-alkyl, C₁-C₆-haloalkoxy, amino, mono or di C₁-C₆-alkylamino, mono or di arylamino and nitro; and wherein R³ is selected from C₁-C₁₀-alkyl and amino;
wherein aryl means phenyl or naphthyl
wherein heterocyclic/heterocyclo means saturated, partially saturated or unsaturated heteroatom containing 5 or 6 membered radicals where the heteroatom is selected from nitrogen, sulfur and oxygen;
wherein heteroaryl is a fully unsaturated heterocyclic radical; provided R¹ is not C₁-C₁₀-alkyl when R³ is methyl and when R² is phenyl or phenyl substituted with methyl, methoxy or chloro; further provided R¹ is not propylsulfonyl or trifluoroacetyl when R³ is methyl; or a pharmaceutically-acceptable salt thereof.

Preferred compounds are those of the above formula wherein R¹ is selected from heterocyclic-C₁-C₆-alkyl, heteroaryl-C₁-C₆-alkyl, formyl, cyano,mercapto, C₁-C₆-alkoxy, C₁-C₆-alkylthio, C₁-C₆-alkylthio-C₁-C₆-alkyl, C₁-C₆-alkylsulfonyl, C₁-C₆-haloalkylsulfonyl, arylsulfonyl, halo, C₂-C₆-alkenyloxy-C₁-C₆-alkyl, C₁-C₆-alkylcarbonyl, benzoyl, aryl-C₁-C₆-alkylcarbonyl, C₁-C₆-cyanoalkyl, aryl-C₂-C₆-alkenyl, C₁-C₆-aminoalkyl, C₁-C₆-alkylamino-C₁-C₆-alkyl, N-arylamino-C₁-C₆-alkyl, N-C₁-C₆-alkyl-N-arylaminoalkyl, C₁-C₆-alkoxycarbonyl, C₁-C₆-haloalkylcarbonyl, aminocarbonyl, C₁-C₆-alkylaminocarbonyl, C₁-C₆-alkylaminocarbonyl-C₁-C₆-alkyl, aryl-C₁-C₆-alkoxy, aryl-C₁-C₆-alkylthio, phenylsulfonyl, heteroaryl-C₁-C₆-alkoxy, heteroaryloxy, heteroarylthio, arylthio-C₁-C₆-alkyl, aryloxy-C₁-C₆-alkyl, arylthio, aryloxy, aryl-C₁-C₆-alkylthio-C₁-C₆-alkyl, aryl-C₁-C₆-alkoxy-C₁-C₆-alkyl, C₁-C₆-alkoxyaryl-C₁-C₆-alkoxy-C₁-C₆-alkyl, aryl and heteroaryl, wherein the aryl and heteroaryl radicals are optionally substituted at a substitutable position with one or more radicals selected from halo, C₁-C₆-alkylthio, C₁-C₆-alkylsulfinyl, C₁-C₆-alkyl, cyano, C₁-C₆-haloalkyl, hydroxyl, C₁-C₆-alkoxy, C₁-C₆-hydroxyalkyl and C₁-C₆-haloalkoxy; wherein R² and R³ are defined as above; or a pharmaceutically-acceptable salt thereof.

A further group of preferred compounds are those wherein R¹ is selected from C₁-C₆-alkyl, C₁-C₆-haloalkyl, C₁-C₆-hydroxyalkyl, C₁-C₆-alkoxy-C₁-C₆-alkyl, aryl-C₁-C₆-alkylsulfonyl, arylthio-C₁-C₆-alkyl, heteroaryl-C₁-C₁₀-alkoxy-C₁-C₆-alkyl, heteroaryloxy-C₁-C₆-alkyl, heteroarylthio-C₁-C₆-alkyl, heteroaryl-C₁-C₆-alkylthio-C₁-C₆-alkyl, aryl-C₁-C₆-alkylthio, and aryl-C₁-C₆-alkoxy; wherein R² is selected from heteroaryl, and aryl, wherein the heteroaryl, and aryl radicals are substituted at a substitutable position with one or more radicals selected from hydrido, halo, C₁-C₆-alkylthio, C₁-C₆-alkylsulfinyl, C₁-C₆-alkyl, cyano, carboxyl, C₁-C₆-alkoxycarbonyl, C₁-C₆-haloalkyl, hyrdoxyl, C₁-C₆-alkoxy, C₁-C₆-hydroxyalkyl, C₁-C₆-alkoxy-C₁-C₆-alkyl, C₁-C₆-haloalkoxy, amino, C₁-C₆-alkylamino, phenylamino and nitro; and wherein R³ is defined as above;or a pharmaceutically-acceptable salt thereof.

The phrase "further provided", as used in the above description, is intended to mean that the denoted proviso is not to be considered conjunctive with any of the other provisos.

Compounds of Formula I would be useful for, for preparing a medicament for the treatment of inflammation in a subject, and for treatment of other inflammation- associated disorders, such as, as an analgesic in the treatment of pain and headaches, or as an antipyretic for the treatment of fever. For example compounds of the invention would be useful to treat arthritis, including but not limited to rheumatoid arthritis, spondyloarthopathies, gouty arthritis, osteoarthritis, systemic lupus erythematosus and juvenile arthritis. Such compounds of the invention would be useful in the treatment of asthma, bronchitis, menstrual cramps, tendinitis, bursitis, and skin related conditions such as psoriasis, eczema, burns and dermatitis. Compounds of the invention also would be useful to treat gastrointestinal conditions such as inflammatory bowel disease, Crohn's disease, gastritis, irritable bowel syndrome arid ulcerative colitis and for the prevention of colorectal cancer. Compounds of the invention would be useful in treating inflammation in such diseases as vascular diseases, migraine headaches, periarteritis nodosa, thyroiditis, aplastic anemia, Hodgkin's disease, sclerodoma, rheumatic fever, type I diabetes, myasthenia gravis, multiple sclerosis, sarcoidosis, nephrotic syndrome, Behcet's syndrome, polymyositis, gingivitis, hypersensitivity, conjunctivitis, swelling occurring after injury, myocardial ischemia, and the like. The compounds were also be useful in the treatment of ophthalmic diseases such as retinitis, retinopathies, uveitis, and of acute injury to the eye tissue. The compounds would also be useful for the treatment of certain central nervous system disorders such as Alzheimer's disease and dementia. The compounds of the invention are useful as anti-inflammatory agents, such as for the treatment of arthritis, with the additional benefit of having significantly less harmful side effects. These compounds would also be useful in the treatment of allergic rhinitis, respiratory distress syndrome, endotoxin shock syndrome, atherosclerosis and central nervous system damage resulting from stroke, ischemia and trauma.

Besides being useful for human treatment, these compounds are also useful for treatment of mammals,

including horses, dogs, cats, rats, mice, sheep, pigs, etc.

The compound can especially be used for preparing a medicament for treating arthritis, pain or fever.

The present compounds may also be used in co-therapies, partially or completely, in place of other conventional antiinflammatories, such as together with steroids, NSAIDs, 5-lipoxygenase inhibitors, LTB₄ antagonists and LTA₄ hydrolase inhibitors.

Suitable LTB₄ inhibitors include, among others, ebselen, Bayer Bay-x-1005, Ciba Geigy compound CGS-25019C, Leo Denmark compound ETH-615, Lilly compound LY-293111, Ono compound ONO-4057, Terumo compound TMK-688, Lilly compounds LY-213024, 264086 and 292728, ONO compound ONO-LB457, Searle compound SC-53228, calcitrol, Lilly compounds LY-210073, LY223982, LY233469, and LY255283, ONO compound ONO-LB-448, Searle compounds SC-41930, SC-50605 and SC-51146, and SK&F compound SKF-104493. Preferably, the LTB₄ inhibitors are selected from ebselen, Bayer Bay-x-1005, Ciba Geigy compound CGS-25019C, Leo Denmark compound ETH-615, Lilly compound LY-293111, Ono compound ONO-4057, and Terumo compound TMK-688.

Suitable 5-LO inhibitors include, among others, masoprocol, tenidap, zileuton, pranlukast, tepoxalin, rilopirox, flezelastine hydrochloride, enazadrem phosphate, and bunaprolast.

As, illustrated, the imidazoles of Formula I and I' are magnetically and structurally equivalent because of the prototropic tautomeric nature of the acidic hydrogen [A.R. Katritzky and C.W. Rees, "*Imidazoles and their Benzo Derivatives*" **Comprehensive Heterocyclic Chemistry**, Vol. 5, 363-365 (1984)]:

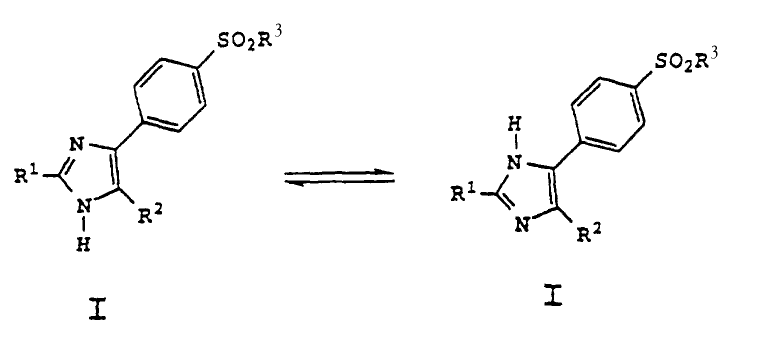

The present invention preferably includes compounds which selectively inhibit cyclooxygenase. II over cyclooxygenase I. Preferably, the compounds have a cyclooxygenase II IC₅₀ of less than about 0.5 µM, and also have a selectivity ratio of cyclooxygenase II inhibition over cyclooxygenase I inhibition of at least 5, and more preferably of at least 100. Even more preferably, the compounds have a cyclooxygenase I IC₅₀ of greater than about 2.5 µM, and more preferably of greater than 50 µM. Such preferred selectivity may indicate an ability to reduce the incidence of common NSAID-induced side effects.

A family of specific compounds cf particular interest within Formula I consists of compounds and pharmaceutically-acceptable salts thereof as follows:
5-(3-chlorophenyl)-4-(4-methylsulfonylphenyl)-2-trifluoromethyl-1H-imidazole;
5-(3-chloro-4-methylphenyl)-4-(4-methylsulfonylphenyl)-2-trifluoromethyl-1H-imidazole;
5-(3-fluorophenyl)-4-(4-methylsulfonylphenyl)-2-trifluoromethyl-1H-imidazole;
5-(3-chloro-4-methoxyphenyl)-4-(4-methylsulfonylphenyl)-2-trifluoromethyl-1H-imidazole;
5-(2,4-dichlorophenyl)-4-(4-methylsulfonylphenyl)-2-trifluoromethyl-1H-imidazole;
5-(4-bromophenyl)-4- (4-methylsulfonylphenyl) -2-trifluoromethyl-1H-imidazole;
4-(4-methylsulfonylphenyl)-2-trifluoromethyl-5-(4-trifluoromethylphenyl)-1H-imidazole;
4-(4-methylsulfonylphenyl)-2-trifluoromethyl-5-(4-trifluoromethoxyphenyl)-1H-imidazole;
5-(4-ethylphenyl)-4-(4-methylsulfonylphenyl)-2-trifluoromethyl-1H-imidazole;
5-(4-butylphenyl)-4-(4-methylsulfonylphenyl)-2-trifluoromethyl-1H-imidazole;
5-(4-butoxyphenyl)-4-(4-methylsulfonylphenyl)-2-trifluoromethyl-1H-imidazole;
4-(4-methylsulfonylphenyl)-5-(4-methylthiophenyl)-2-trifluoromechyl-1H-imidazole;
5-(3,5-dichloro-4-methoxyphenyl)-4-(4-methylsulfonylphenyl)-2-trifluoromethyl-1H-imidazole;
5-(3,5-dichloro-4-methylphenyl)-4-(4-methylsulfonylphenyl)-2-trifluoromethyl-1H-imidazole;
5-[4-(4-methylsulfonylphenyl)-2-trifluoromethyl-1H-imidazol-5-yl]-1,3-benzodioxole;
2-(4-chlorophenoxy)methyl-5-(4-chlorophenyl)-4-(4-methylsulfonylphenyl)-1H-imidazole;
5-(4-chlorophenyl)-2-[(4-fluorophenoxy)methyl]-4-(4-methylsulfonylphenyl)-1H-imidazole;
5-(4-chlorophenyl)-4-(4-methylsulfonylphenyl)-2-(phenylthiomethyl)-1H-imidazole;
5-(4-chlorophenyl)-2-[(4-methoxybenzyloxy)methyl]-4-(4-methylsulfonylphenyl)-1H-imidazole;
2-benzyl-5-(4-chlorophenyl)-4-(4-methylsulfonylphenyl)-1H-imidazole;
5-(4-chlorophenyl)-4-(4-methylsulfonylphenyl)-2-(phenylethyl)-1H-imidazole;
5-(4-chlorophenyl)-4-(4-methylsulfonylphenyl)-2-(phenylcarbonyl)-1H-imidazole;
5-(4-chlorophenyl)-4-(4-methylsulfonylphenyl)-2-phenoxymethyl-1H-imidazole;
5-(4-chlorophenyl)-4-(4-methylsulfonylphenyl)-2- (2-phenyl-*trans*-eth-1-ene)-1H-imidazole;
2-(2-benzofuryl)-5-(4-chlorophenyl)-4-(4-methylsulfonylphenyl)-1H-imidazole;
5-(4-chlorophenyl)-4-(4-methylsulfonylphenyl)-2-(2-furyl)-1H-imidazole;
2-benzylthio-5-(4-chlorophenyl)-4-[4-(methylsulfonyl)phenyl]imidazole;
5-(4-chlorophenyl)-4-[4-(methylsulfonyl)phenyl]-2-(trifluoromethyl)-1H-imidazole;
5-(4-chlorophenyl)-2-(3-furyl)-4-[4-(methylsulfonyl)phenyl]-1H-imidazole;
4-(4-methylsulfonylphenyl)-5-phenyl-2-phenoxymethyl-1H-imidazole;
4-(4-methylsulfonylphenyl)-5-phenyl-2-(2-phenyl-*trans*-eth-1-ene)-1H-imidazole;
2-(2-benzofuryl)-4-(4-methylsulfonylphenyl)-5-phenyl-1H-imidazole;
2-(2-furyl)-4-(4-methylsulfonylphenyl)-5-phenyl-1H-imidazole;
2-benzylthio-4-[4-(methylsulfonyl)phenyl]-5-phenylimidazole;
4-[4-(methylsulfonyl)phenyl]-5-phenyl-2-(trifluoromethyl)-1H-imidazole;
2-(3-furyl)-4-[4-(methylsulfonyl)phenyl]-5-phenyl-1H-imidazole;
4-[5-(3-chlorophenyl)-2-trifluoromethyl-1H-imidazol-4-yl]benzenesulfonamide;
4-[5-(3-chloro-4-methylphenyl)-2-trifluoromethyl-1H-imidazol-4-yl]benzenesulfonamide;
4-[5-(3-fluorophenyl)-2-trifluoromethyl-1H-imidazol-4-yl]benzenesulfonamide;
4-[5-(3-chloro-4-methoxyphenyl)-2-trifluoromethyl-1H-imidazol-4-yl]benzenesulfonamide;
4-[5-(2,4-dichlorophenyl)-2-trifluoromethyl-1H-imidazol-4-yl]benzenesulfonamide;
4-[5-(4-bromophenyl)-2-trifluoromethyl-1H-imidazol-4-yl]benzenesulfonamide;
4-[2-trifluoromethyl-5-(4-trifluoromethylphenyl)-1H-imidazol-4-yl]benzenesulfonamide;
4-[2-trifluoromethyl-5-(4-trifluoromethoxyphenyl)-1H-imidazol-4-yl]benzenesulfonamide;
4-[5-(4-ethylphenyl)-2-trifluoromethyl-1H-imidazol-4-yl]benzenesulfonamide;
4-[5-(4-butylphenyl)-2-trifluoromethyl-lH-imidazol-4-yl]benzenesulfonamide;
4-[5-(4-butoxyphenyl)-2-trifluoromethyl-1H-imidazol-4-yl]benzenesulfonamide;
4-[5-(4-methylthiophenyl)-2-trifluoromethyl-1H-imidazol-4-yl]benzenesulfonamide;
4-[5-(3,5-dichloro-4-methoxyphenyl)-2-trifluoromethyl-1H-imidazol-4-yl]benzenesulfonamide;
5-(3,5-dichloro-4-methylphenyl)-2-trifluoromethyl-1H-imidazol-4-yl]benzenesulfonamide;
5-[4-(4-aminosulfonylphenyl)-2-trifluoromethyl-1H-imidazol-5-yl]-1,3-benzodioxole;
4-[2-(4-chlorophenoxy)methyl-5-(4-chlorophenyl)-1H-imidazol-4-yl]benzenesulfonamide;
4-[5-(4-chlorophenyl)-2-[(4-fluorophenoxy)methyl]-1H-imidazol-4-yl]benzenesulfonamide;
4-[5-(4-chlorophenyl)-2-(phenylthiomethyl)-1H-imidazol-4-yl]benzenesulfonamide;
4-[5-(4-chlorophenyl)-2-[(4-methoxybenzyloxy)methyl]-1H-imidazol-4-yl]benzenesulfonamide;
4-[2-benzyl-5-(4-chlorophenyl)-1H-imidazol-4-yl]benzenesulfonamide;
4-[5-(4-chlorophenyl)-2-(phenylethyl)-1H-imidazol-4-yl]benzenesulfonamide ;
4-[5-(4-chlorophenyl)-2-(phenylcarbonyl)-1H-imidazol-4-yl]benzenesulfonamide;
4-[5-(4-chlorophenyl)-2-phenoxymethyl-1H-imidazol-4-yl]benzenesulfonamide;
4-[5-(4-chlorophenyl)-2-(2-phenyl-*trans*-eth-1-ene)-1H-imidazol-4-yl]benzenesulfonamide;
4-[2-(2-benzofuryl)-5-(4-chlorophenyl)-1H-imidazol-4-yl]benzenesulfonamide;
4-[5-(4-chlorophenyl)-2-(2-furyl)-1H-imidazol-4-yl]benzenesulfonamide;
4-[2-benzylthio-5-(4-chlorophenyl)-1H-imidazol-4-yl]benzenesulfonamide;
4-[5-(4-chlorophenyl)-2-(trifluoromethyl)-1H-imidazol-4-yl]benzenesulfonamide;
4-[5-(4-chlorophenyl)-2-(3-furyl)-1H-imidazol-4-yl]benzenesulfonamide;
4-[5-phenyl-2-phenoxymethyl-1H-imidazol-4-yl]benzenesulfonamide;
4-[5-phenyl-2-(2-phenyl*-trans*-eth-1-ene)-1H-imidazol-4-yl]benzenesulfonamide;
4-[2-(2-benzofuryl)-5-phenyl-1H-imidazol-4-yl]benzenesulfonamide;
4-[2-(2-furyl)-5-phenyl-1H-imidazol-4-yl]benzenesulfonamide;
4-[2-benzylthio-5-phenyl-imidazol-4-yl]benzenesulfonamide;
4-[5-phenyl-2-(trifluoromethyl)-1H-imidazol-4-yl]benzenesulfonamide;
4-[2-(3-furyl)-5-phenyl-1H-imidazol-4-yl]benzenesulfonamide;
5-(4-fluorophenyl)-4-(4-methylsulfonylphenyl)-2-trifluoromethyl-1H-imidazole;
5-(4-fluorophenyl)-4-(4-methylsulfonylphenyl)-2-phenoxymethyl-1H-imidazole;
5-(4-fluorophenyl)-4-(4-methylsulfonylphenyl)-2-(2-phenyl*-trans*-eth-1-ene)-1H-imidazole;
2-(2-benzofuryl)-5-(4-fluorophenyl)-4-(4-methylsulfonylphenyl)-1H-imidazole;
5-(4-fluorophenyl)-2-(2-furyl)-4-(4-methylsulfonylphenyl)-1H-imidazole;
5-(4-fluorophenyl)-2-methyl-4-(4-methylsulfonylphenyl)-1H-imidazole;
5-(4-fluorophenyl)-2-isopropyl-4-(4-methylsulfonylphenyl)-1H-imidazole;
5-(4-fluorophenyl)-2-hydroxymethyl-4-(4-methylsulfonylphenyl)-1H-imidazole;
2-benzylthio-5-(4-fluorophenyl)-4-[4-(methylsulfonyl)phenyl]imidazole;
5-(3-fluoro-4-methoxyphenyl)-4-[4-(methylsulfonyl)phenyl]-2-(trifluoromethyl)-1H-imidazole;
5-(4-chlorophenyl)-4-[4-(methylsulfonyl)phenyl]-2-(trifluoromethyl)-1H-imidazole;
5-(2-chlorophenyl)-4-[4-(methylsulfonyl)phenyl]-2-(trifluoromethyl)-1H-imidazole;
5-(3,4-dichlorophenyl)-4-[4-(methylsulfonyl)phenyl]-2-(trifluoromethyl)-1H-imidazole;
4-[4-(methylsulfonyl)phenyl]-5-(2-naphthyl)-2-(trifluoromethyl)-1H-imidazole;
5-(4-methoxyphenyl)-4-[4-(methylsulfonyl)phenyl]-2-(trifluoromethyl)-1H-imidazole;
5-(4-methylphenyl)-4-[4-(methylsulfonyl)phenyl]-2-(trifluoromethyl)-1H-imidazole;
5-(2,4-difluorophenyl)-4-(4-(methylsulfonyl)phenyl]-2-(trifluoromethyl)-1H-imidazole;
5-(3,4-difluorophenyl)-4-[4-(methylsulfonyl)phenyl]-2-(trifluoromethyl)-1H-imidazole;
4-[4-(methylsulfonyl)phenyl]-5-phenyl-2-(trifluoromethyl)-1H-imidazole;
5-(4-fluorophenyl)-2-(3-furyl)-4-(4-methylsulfonylphenyl)-1H-imidazole;
4-[5-(4-fluorophenyl)-2-(trifluoromethyl)-1H-imidazol-4-yl]benzenesulfonamide;
4-[5-(4-fluorophenyl)-2-phenoxymethyl-1H-imidazol-4-yl]benzenesulfonamide;
4-[5-(4-fluorophenyl)-2-(2-phenyl-*trans*-eth-1-ene)-1H-imidazol-4-yl]benzenesulfonamide;
4-[5-(4-fluorophenyl)-2-(2-benzofuryl)-1H-imidazol-4-yl]benzenesulfonamide;
4-[5-(4-fluorophenyl)-2-isopropyl-1H-imidazol-4-yl]benzenesulfonamide;
4-[5-(4-fluorophenyl)-2-methyl-1H-imidazol-4-yl]benzenesulfonamide ;
4-[5-(4-fluorophenyl)-2-(2-furyl)-1H-imidazol-4-yl]benzenesulfonamide;
4-(2-benzylthio-5-(4-fluorophenyl)-1H-imidazol-4-yl]benzenesulfonamide;
4-[5-(4-fluorophenyl)-2-hydroxymethyl-1H-imidazol-4-yl]benzenesulfonamide;
4-[5-(3-fluoro-4-methoxyphenyl)-2-(trifluoromethyl)-1H-imidazol-4-yl]benzenesulfonamide;
4-[5-(4-chlorophenyl)-2-(trifluoromethyl)-1H-imidazol-4-yl]benzenesulfonamide;
4-[5-(2-chlorophenyl)-2-(trifluoromethyl)-1H-imidazol-4-yl]benzenesulfonamide;
4-[5-(3,4-dichlorophenyl)-2-(trifluoromethyl)-1H-imidazol-4-yl]benzenesulfonamide;
4-[5-(2-naphthyl)-2-(trifluoromethyl)-1H-imidazol-4-yl]benzenesulfonamide;
4-[5-(4-methoxyphenyl)-2-(trifluoromethyl)-1H-imidazol-4-yl]benzenesulfonamide;
4-[5-(4-methylphenyl)-2-(trifluoromethyl)-1H-imidazol-4-yl]benzenesulfonamide;
4-[5-(2,4-difluorophenyl)-2-(trifluoromethyl)-1H-imidazol-4-yl]benzenesulfonamide;
4-[5-(3,4-difluorophenyl)-2-(trifluoromethyl)-1H-imidazol-4-yl]benzenesulfonamide;
4-[5-phenyl-2-(trifluoromethyl)-1H-imidazol-4-yl]benzenesulfonamide;
4-[5-(4-fluorophenyl)-2-(3-furyl)-1H-imidazol-4-yl]benzenesulfonamide;
5-(4-fluorophenyl)-1-methyl-4-[4-(methylsulfonyl)phenyl]-2-(trifluoromethyl)-1H-imidazole;

5-(3,5-dimethylphenyl)-4-[4-(methylsulfonyl)phenyl]-2-(trifluoromethyl)-1H-imidazole;
5-(5-bromothien-2-yl)-4-[4-(methylsulfonyl)phenyl]-2-(trifluoromethyl)-1H-imidazole;
N,N-dimethyl-4-[4-[4-(methylsulfonyl)phenyl]-2-(trifluoromethyl)-1H-imidazol-5-yl]benzenamine;
4-(4-fluorophenyl)-5-[4-(mechylsulfonyl)phenyl]-1H-imidazole-2-thiol;

5-(3,5-dimethyl-4-methoxyphenyl)-4-[4-(methylsulfonyl)phenyl)-2-(trifluoromethyl)-1H-imidazole;
5-[3-fluoro-4-(methylthio)phenyl]-4-[4-(methylsulfonyl)phenyl]-2-(trifluoromethyl)-1H-imidazole;
5-(3-chloro-5-methylphenyl)-4-[4-(methylsulfonyl)phenyl]-2-(trifluoromethyl)-1H-imidazole;
5-[3-chloro-4-(methylthio)phenyl]-4-[4-(methylsulfonyl)phenyl]-2-(trifluoromethyl)-1H-imidazole;
5-(3-fluoro-4-methylphenyl)-4-[4-(methylsulfonyl)phenyl]-2-(trifluoromethyl)-1H-imidazole;
1-[5-(3-chloro-5-methylphenyl)-4-[4-(methylsulfonyl)phenyl]-2-(trifluoromethyl)-1H-imidazol-1-yl]ethanone;
5-(3-methylphenyl)-4-[4-(methylsulfonyl)phenyl] -2-trifluoromethyl-1H-imidazole;
5-(3-methylphenyl)-4-[4-(sulfonamido)phenyl]-2-trifluoromethyl-1H-imidazole;

4-[4-(methylsulfonyl)phenyl]-5-(3-pyridyl)-2-(trifluoromethyl)-1H-imidazole;

5-(2-fluorophenyl)-4-[4-(methylsulfonyl)phenyl]-2-(trifluoromethyl)-1H-imidazole;
5-(3-methoxyphenyl)-4-[4-(methylsulfonyl)phenyl]-2-(trifluoromethyl)-1H-imidazole,
5-(2-methylphenyl)-4-[4-(methylsulfonyl)phenyl]-2-(trifluoromethyl)-1H-imidazole;
5-(cyclohexyl)-4-[4-(methylsulfonyl)phenyl]-2-(trifluoromethyl)-1H-imidazole;
4-[4-(methylsulfonyl)phenyl]-5-(2-thiophenyl)-2-(trifluoromethyl)-1H-imidazole;
5-(4-chloro-3-methylphenyl)-4-[4-(methylsulfonyl)phenyl]-2-(trifluoromethyl)-1H-imidazole;
5-(4-cycloheptyl)-4-[4-(methylsulfonyl)phenyl]-2-(trifluoromethyl)-1H-imidazole;
5-(4-fluoro-3-methylphenyl)-4-[4-(methylsulfonyl)phenyl]-2-(trifluoromethyl)-1H-imidazole;
5-(cyclopentyl)-4-[4-(methylsulfonyl)phenyl]-2-(trifluoromethyl)-1H-imidazole;
4-[4-(methylsulfonyl)phenyl]-5-(3-thiophenyl)-2-(trifluoromethyl)-1H-imidazole;
5-(3-fluoro-2-methylphenyl)-4-[4-(methylsulfonyl)phenyl]-2-(trifluoromethyl)-1H-imidazole;
5-(4-chloro-3-methoxyphenyl)-4-[4-(methylsulfonyl)phenyl]-2-(trifluoromethyl)-1H-imidazole;
5-(4-fluorophenyl)-2-(methoxymethyl)-4-[4-(methylsulfonyl)-phenyl]-1H-imidazole;
1-[5-(4-fluorophenyl)-4-[4-(methylsulfonyl)phenyl]-1H-imidazol-2-yl]-2-phenylethanone;
[5-(4-fluorophenyl)-4-[4-(methylsulfonyl)phenyl]-1H-imidazol-2-yl]phenylmethanone;
1-[5-(4-fluorophenyl)-4-[4-(methylsulfonyl)phenyl]-1H-imidazol-2-yl]-4-phenyl-1-butanone;

Compounds of Formula I where R² is heteroaryl would also be capable of inhibiting cytokines, such as TNF, IL-1, IL-6, and IL-8. As such, the compounds can be used in the manufacture of a medicament or in a method for the treatment for the prophylactic or therapeutic treatment of diseases mediated by cytokines, such as TNF, IL-1, IL-6, and IL-8.

The term "hydrido" denotes a single hydrogen atom (H). This hydrido radical may be attached, for example, to an oxygen atom to form a hydroxyl radical or two hydrido radicals may be attached to a carbon atom to form a methylene (-CH₂-) radical. Where used, either alone or within other terms such as "haloalkyl", "alkylsulfonyl", "alkoxyalkyl" and "hydroxyalkyl", the term "alkyl" embraces linear or branched radicals having the range of carbon atoms indicated in claim 1 or, preferably, one to six carbon atoms. Examples of such radicals include methyl, ethyl, n-propyl, isopropyl, n-butyl, isobutyl, sec-butyl, *tert*-butyl, pentyl, isoamyl, hexyl. The term "halo" means halogens such as fluorine, chlorine, bromine or iodine. The term "haloalkyl" embraces radicals wherein any one or more of the alkyl carbon atoms is substituted with halo as defined above. Specifically embraced are monohaloalkyl, dihaloalkyl and polyhaloalkyl radicals. A monohaloalkyl radical, for one example, may have either an iodo, bromo, chloro or fluoro atom within the radical. Dihalo and polyhaloalkyl radicals may have two or more of the same halo atoms or a combination of different halo radicals. Examples of haloalkyl radicals include fluoromethyl, difluoromethyl, trifluoromethyl, chloromethyl, dichloromethyl, trichloromethyl, trichloromethyl, pentafluoroethyl, heptafluoropropyl, difluorochloromethyl, dichlorofluoromethyl, difluoroethyl, difluoropropyl, dichloroethyl and dichloropropyl. The term "hydroxy C₁₋₆ alkyl" embraces linear or branched alkyl radicals having one to six carbon atoms any one of which may be substituted with one or more hydroxyl radicals. Examples of such radicals include hydroxymethyl, hydroxyethyl, hydroxypropyl, hydroxybutyl and hydroxyhexyl. The terms "C₁₋₆ alkoxy" and "C₁₋₆ alkoxy C₁₋₆ alkyl" embrace linear or branched oxy-containing radicals each having alkyl portions of one to six carbon atoms. Examples of such radicals include methoxy, ethoxy, propoxy, butoxy and *tert*-butoxy. The term "C₁₋₆ alkoxy C₁₋₆ alkyl" embraces alkyl radicals having one or more C₁₋₆ alkoxy radicals attached to the alkyl radical, that is, to form monoalkoxyalkyl and dialkoxyalkyl radicals. More preferred alkoxyalkyl radicals are "lower alkoxyalkyl" radicals having one to six carbon atoms and one or two alkoxy radicals. Examples of such radicals include methoxymethyl, methoxyethyl, ethoxyethyl, methoxybutyl and methoxypropyl. The "alkoxy" or "alkoxyalkyl" radicals may be further substituted with one or more halo atoms, such as fluoro, chloro or bromo, to provide "haloalkoxy" or "haloalkoxyalkyl" radicals. More preferred haloalkoxy radicals are "lower haloalkoxy" radicals having one to six carbon atoms and one or more halo radicals. Examples of such radicals include fluoromethoxy, chloromethoxy, trifluoromethoxy, trifluoroethoxy, fluoroethoxy and fluoropropoxy. The term "cyano C₁₋₆ alkyl" embraces radicals having a cyano or nitrile (-CN) radical attached to an alkyl radicals as described above. Examples of such cyanoalkyl radicals include cyanomethyl, cyanopropyl, cyanoethyl and cyanobutyl. The term "aryl" means phenyl and naphthyl. The terms "heterocyclic" and "heterocyclo" means saturated, partially saturated and unsaturated heteroatom-containing ring-shaped radicals, where the heteroatoms may be selected from nitrogen, sulfur and oxygen having 5 or 6 ring members. Examples or saturated heterocyclic radicals include e.g. pyrrolidinyl, imidazolidinyl, piperidino, piperazinyl, morpholinyl; thiazolidinyl. Examples of partially saturated heterocyclic radicals include dihydrothiophene, dihydropyran, dihydrofuran and dihydrothiazole.

The term "heteroaryl" are unsaturated heterocyclic radicals. Examples of unsaturated heterocyclic radicals, also termed "heteroaryl" radicals include for example, pyrrolyl, pyrrolinyl, imidazolyl, pyrazolyl, pyridyl, pyrimidyl, pyrazinyl, pyridazinyl, triazolyl [e.g., 4H-1,2,4-triazolyl, 1H-1,2,3-triazolyl, 2H-1,2,3-triazolyl,] tetrazolyl [e.g. 1H-tetrazolyl, 2H-tetrazolyl,]; oxazolyl, isoxazolyl, oxadiazolyl [e.g., 1,2,4-oxadiazolyl, 1,3,4-oxadiazolyl, 1,2,5-oxadiazolyl].

The term "heterocyclic C₁₋₆ alkyl" are heterocyclic-substituted C₁₋₆ alkyl radicals. e.g. pyrrolidinylmethyl.

The term "C₁₋₆-alkylthio" embraces radicals containing a linear or branched alkyl radical, of one to six are carbon atoms attached to a divalent sulfur atom. Examples of such alkylthio radicals are methylthio, ethylthio, propylthio, butylthio and hexylthio. The term "C₁₋₆ alkylthio C₁₋₆ alkyl" embraces C₁₋₆ alkylthio radicals attached to an C₁₋₆ alkyl radical. e.g. methylthiomethyl. The term "arylthio" embraces radicals containing an aryl radical, attached to a divalent sulfur atom, such as a phenylthio radical. The term "arylthio C₁₋₆ alkyl" embraces arylthio radicals attached to a C₁₋₆ alkyl radical. Examples of such radicals include phenylthiomethyl. The term "C₁₋₆ alkylsulfinyl" embraces radicals containing a linear or branched alkyl radical, of one to six carbon atoms, attached to a divalent -S(=O)- radical. Examples of such C₁₋₆-alkylsulfinyl radicals include methylsulfinyl, ethylsulfinyl, butylsulfinyl and hexylsulfinyl. The term "sulfonyl", whether used alone or linked to other terms such as alkylsulfonyl, denotes respectively divalent radicals -SO₂-. C₁₋₆ Alkylsulfonyl" embraces alkyl radicals attached to a sulfonyl radical, where alkyl is defined as above. Examples of such C₁₋₆ alkylsulfonyl radicals include methylsulfonyl, ethylsulfonyl and propylsulfonyl. The "C₁₋₆ alkylsulfonyl" radicals may be further substituted with one or more halo atoms, such as fluoro, chloro or bromo, to provide "halo C₁₋₆ alkylsulfonyl" radicals. Examples of such halo C₁₋₆ alkylsulfonyl radicals include fluoromethylsulfonyl, trifluoromethylsulfonyl and chloromethylsulfonyl. The terms "carboxy" or "carboxyl", whether used alone or with other terms, such as "carboxyalkyl", denotes -CO₂H. The term "carbonyl", whether used alone or with other terms, such as "alkoxycarbonyl", denotes -(C=O)-. The term "alkoxycarbonyl" means a radical containing an alkoxy radical, as defined above, attached via an oxygen atom to a carbonyl radical. The alkoxy radicals have one to six carbon atoms. Examples of such "C₁₋₆ alkoxycarbonyl" ester radicals include methoxycarbonyl, ethoxycarbonyl, propoxycarbonyl, butoxycarbonyl and hexyloxycarbonyl. The term "ar C₁₋₆ alkyl" embraces aryl-substituted C₁₋₆ alkyl radicals. Examples of such radicals include benzyl, diphenylmethyl, triphenylmethyl, phenylethyl and diphenylethyl. The aryl in said aralkyl may be additionally substituted as indicated above. The terms benzyl and phenylmethyl are interchangeable. The term "ar C₁₋₆ alkenyl" embraces aryl-substituted C₁₋₆ alkenyl radicals. Examples of such radicals include phenylethenyl and diphenylethenyl. The aryl in said aralkenyl may be additionally substituted as indicated above. The terms "C₁₋₆ alkylcarbonyl", "arylcarbonyl" and "ar C₁₋₆ alkylcarbonyl" include radicals having C₁₋₆ alkyl, aryl and ar C₁₋₆ alkyl radicals, respectively, as defined above, attached via an oxygen atom to a carbonyl radical. Examples of such C₁₋₆ alkylcarbonyl radicals include methylcarbonyl and ethylcarbonyl. Examples of ar C₁₋₆ alkylcarbonyl radicals include benzylcarbonyl. An example of an arylcarbonyl radical is phenylcarbonyl. The term "C₁₋₆-alkoxycarbonyl C₁₋₆ alkyl" embraces radicals having "C₁₋₆ alkoxycarbonyl", as defined above substituted to a C₁₋₆ alkyl radical. Examples of such alkoxycarbonylalkyl radicals include methoxycarbonylmethyl. The term "halo C₁₋₆ alkylcarbonyl" embraces radicals having a halo C₁₋₆ alkyl radical as described above attached to a carbonyl radical. The term "carboxy C₁₋₆ alkyl" embrace radicals having a carboxy radical as defined above, attached to an C₁₋₆ alkyl radical. The alkanoyl radicals may be substituted or unsubstituted, such as formyl, acetyl, propionyl, butyryl, isobutyryl, valeryl, isovaleryl, pivaloyl, hexanoyl, trifluoroacetyl or the like, in which the preferable one is formyl, acetyl, propionyl or trifluoroacetyl. The term "heteroar C₁₋₆ alkyl" embraces heteroaryl-substituted C₁₋₆ alkyl radicals. Examples of such radicals include pyridylmethyl, thienylmethyl and, furylethyl. The heteroaryl in said heteroaralkyl may be additionally substituted as indicated above. The term "aryloxy" embraces aryl radicals, as defined above, attached to an oxygen atom. The aryl in said aryloxy may be additionally substituted as indicated above. Examples of such radicals include phenoxy. The term "heteroaryloxy" embraces heteroaryl radicals as defined above attached to an oxygen radical. The term "ar C₁₋₆ alkoxy" embraces oxy-containing ar C₁₋₆ alkyl radicals attached through an oxygen atom to other radicals. The term "ar C₁₋₆ alkoxy C₁₋₆ alkyl" embraces C₁₋₆ alkyl radicals having an aralkoxy radical attached to the alkyl radical, that is, to form monoaralkyloxyalkyl. The "aralkoxy" or "aralkoxyalkyl" radicals may be further substituted on the aryl as indicated above. Examples of aralkoxyalkyl radicals include benzyloxymethyl. The term "heteroarylthio" embraces radicals having heteroaryl radicals attached to a sulfur radical. Examples of such radicals include 2-furylthio, 2-thienylthio, 3-thienylthio, 4-pyridylthio and 3-pyridylthio. The term "C₁₋₆ alkoxyar C₁₋₆ alkoxy C₁₋₆ alkyl" embraces C₁₋₆ alkoxy substituted ar C₁₋₆ alkoxy C₁₋₆ alkyl radicals. The terms "heteroar C₁₋₆ alkylthio" and "heteroar C₁₋₆ alkylthio" denote radicals having an heteroaryl radical attached to an C₁₋₆ alkylthio radical; e.g. furylmethylthiomethyl. The term "heteroaryl C₁₋₆ alkylthio C₁₋₆ alkyl" denotes radicals having an heteroaryl radical attached to an C₁₋₆ alkylthio radical further attached through the sulfur atom to a C₁₋₆ alkyl radical; e.g. furylmethylthiomethyl. The term "heteroarylthio C₁₋₆ alkyl" denotes radicals having an heteroaryl radical attached to a sulfur atom further attached through the sulfur atom to a C₁₋₆ alkyl radical. Examples of such radicals include thienylthiomethyl and pyridylthiohexyl. The term "ar C₁₋₆ alkylthio" embraces radicals having ar C₁₋₆ alkyl radicals attached to a bridging sulfur atom. six carbon atoms. Examples of such radicals include benzylthio and phenylethylthio. The term "ar C₁₋₆ alkylthio C₁₋₆ alkyl" embraces radicals having ar C₁₋₆ alkyl radicals attached to C₁₋₆ alkyl radicals through a bridging sulfur atom. Examples of such radicals include benzylthiomethyl and phenylethylthiomethyl. The term "heteroaryloxy C₁₋₆ alkyl" denotes radicals having an heteroaryl radical attached to an oxygen atom further attached through the oxygen atom to an C₁₋₆ alkyl radical. Examples of such radicals include furylbutoxyethyl, pyridyloxymethyl and thienyloxyhexyl. The term "amino C₁₋₆ alkyl" embraces C₁₋₆ alkyl radicals substituted with amino radicals. Examples include aminomethyl, aminoethyl and aminobutyl. The term "C₁₋₆ alkylamino C₁₋₆ alkyl" embraces amino C₁₋₆ alkyl radicals having the nitrogen atom substituted with at least one C₁₋₆ alkyl radical. The term "mono or di C₁₋₆ alkylamino" denotes amino groups which have been substituted with one or two C₁₋₆ alkyl radicals. E.g. as N-methylamino, N-ethylamino, N,N-dimethylamino, N,N-diethylamino or the like. The term "C₁₋₆ alkylaminocarbonyl" embraces alkylamino radicals, as described above, to a carbonyl radical. E.g. N-methylaminocarbonyl and N,N-dimethylcarbonyl. The term "mono or di arylamino" denotes amino groups which have been substituted with one or two aryl radicals, such as N-phenylamino. The "arylamino" radicals may be further substituted on the aryl ring portion of the radical as indicated above. The terms "N-arylamino C₁₋₆ alkyl" and "N-aryl-N-C₁₋₆ alkyl-amino C₁₋₆ alkyl" denote amino groups which have been substituted with one aryl radical or one aryl and one C₁₋₆ alkyl radical, respectively, and having the amino group attached to a C₁₋₆ alkyl radical. Examples of such radicals include N-phenylaminomethyl and N-phenyl-N-methylaminomethyl. The term "aminocarbonyl" denotes an amide group of the formula -C(=O)NH₂. The term "mono or di C₁₋₆ alkylaminocarbonyl C₁₋₆ alkyl" denotes an aminocarbonyl group which has been substituted with one or two C₁₋₆ alkyl radicals. The term "aryloxy C₁₋₆ alkyl" embraces alkyl radicals having an aryloxy radical, attached to the alkyl radical. E.g. phenoxymethyl. The terms "heteroar C₁₋₆ alkoxy C₁₋₆ alkyl" and "heteroaryl C₁₋₆ alkoxy C₁₋₆ alkyl" embrace alkyl radicals having a heterocyclic radical attached to an C₁₋₆ alkoxy radical, further attached to the C₁₋₆ alkyl radical. E.g. 2-thienylmethoxymethyl.

The present invention comprises a pharmaceutical composition comprising a therapeutically-effective amount of a compound of Formula I in association with at least one pharmaceutically-acceptable carrier, adjuvant or diluent.

The present invention also comprises the use of compounds of Formula I for preparing a medicament for treating inflammation or inflammation- associated disorders in a subject, the use comprising administering to the subject having or susceptible to such inflammation or disorder a therapeutically-effective amount of a compound of Formula I.

Also included in the family of compounds of Formula I are the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds of Formula I may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, salicylic, *p*-hydroxybenzoic, phenylacetic, mandelic, embonic (pamoic), methanesulfonic, ethylsulfonic, benzenesulfonic, pantothenic, toluenesulfonic, 2-hydroxyethanesulfonic sulfanilic, stearic, cyclohexylaminosulfonic, algenic, β-hydroxybutyric, salicylic, galactaric and galacturonic acid. Suitable pharmaceutically-acceptable base addition salts of compounds of Formula I include metallic salts made from aluminum, calcium, lithium, magnesium, potassium, sodium and zinc or organic salts made from N,N'-dibenzylethylenediamine, chloroprocaine, choline, diethanolamine, ethylenediamine, meglumine (N-methylglucamine) and procaine. All of these salts may be prepared by conventional means from the corresponding compound of Formula I by reacting, for example, the appropriate acid or base with the compound of Formula I.

### GENERAL SYNTHETIC PROCEDURES

The compounds of the invention can be synthesized according to the following procedures of Schemes I-VII, wherein the R¹-R⁵ substituents are as defined for Formula I-III, above, except where further noted.

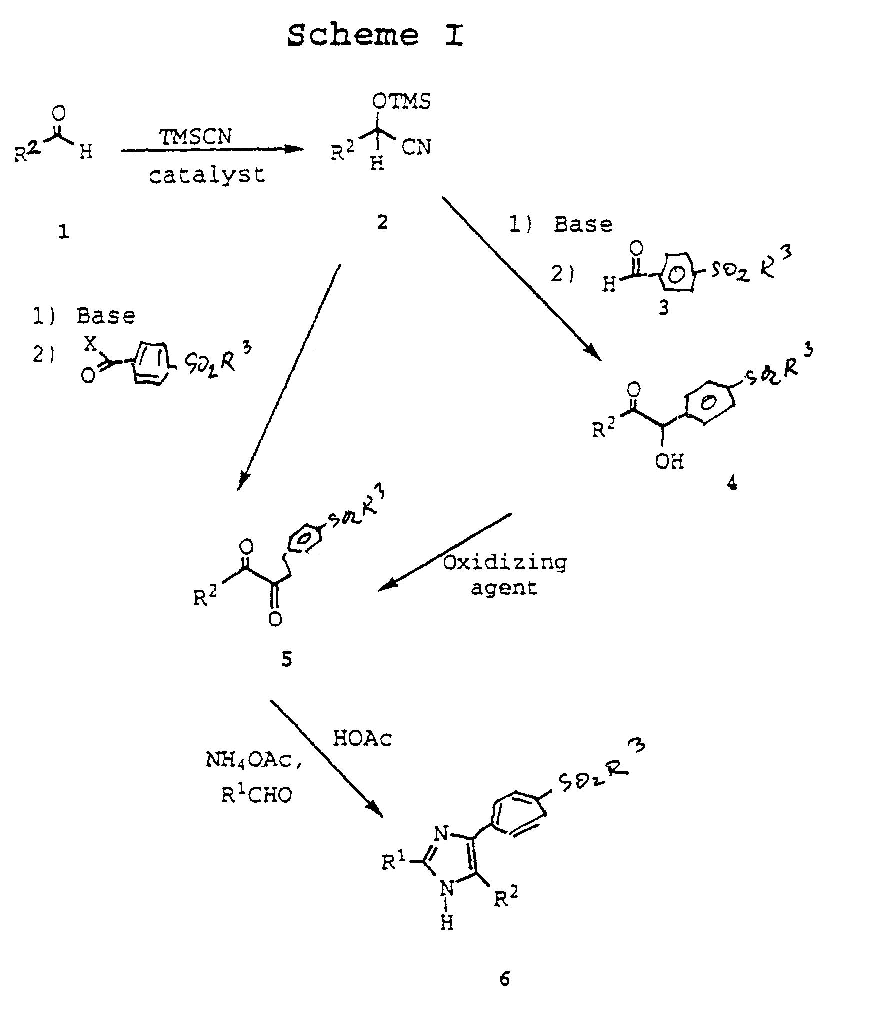

The subject imidazole compounds **6** of this invention may be synthesized according to the sequence outlined in Scheme I. Aldehyde **1** may be converted to the protected cyanohydrin **2** by reaction with a trialkylsilyl cyanide, such as trimethylsilyl cyanide (TMSCN) in the presence of a catalyst such as zinc iodide (ZnI₂) or potassium cyanide (KCN). Reaction of cyanohydrin **2** with a strong base followed by treatment with aldehyde **3** and using both acid and base treatments, in that order, on workup gives enol **4.**

Examples of strong bases suitable for this reaction are lithium diisopropylamide (LDA) and lithium hexamethyldisilazane. Enol **4** may be converted to diketone **5** by reaction with a suitable oxidizing agent, such as bismuth oxide or manganese dioxide, or by a Swern oxidation using dimethyl sulfoxide (DMSO) and trifluoroacetic anhydride. Diketone **5** may be obtained directly by reaction of the anion of cyanohydrin **2** with a substituted acid halide (where X is halo). Any of compounds **4** and **5** may be used as intermediates for conversion to imidazoles 6 according to chemical procedures known by those skilled in the art and described by M. R. Grimmett, *"Advances in Imidazole Chemistry"* in **Advances in Heterocyclic Chemistry, 12,** 104 (1970). The conversion of **5** to imidazoles **6** is-carried out by reaction with ammonium acetate and an appropriate aldehyde (R¹CHO) in acetic acid. Enol **4** may be converted to imidazoles **6** by reaction with formamide. In addition, enol **4** may be converted to imidazoles by first acylating with an appropriate acyl group (R¹CO-) and then treating with ammonium hydroxide.

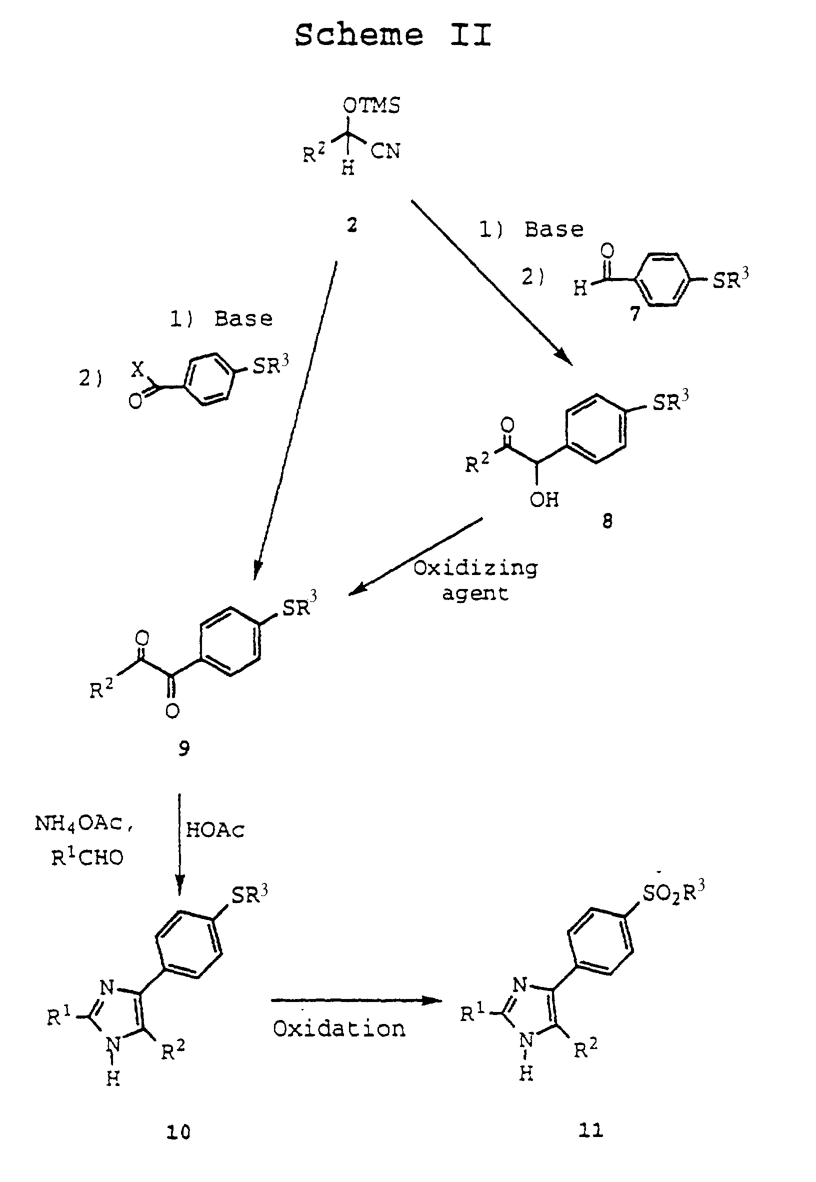

The subject imidazole compounds **11** of this invention may be synthesized according to the sequence outlined in Scheme II. Reaction of cyanohydrin **2** with a strong base followed by treatment with benzaldehyde **7** (where R³ is alkyl) and using both acid and base treatments, in that order, on workup gives benzoin **8.** Examples of strong bases suitable for this reaction are lithium diisopropylamide (LDA) and lithium hexamethyldisilazane. Benzoin **8** may be converted to benzil **9** by reaction with a suitable oxidizing agent, such as bismuth oxide or manganese dioxide, or by a Swern oxidation using dimethyl sulfoxide (DMSO) and trifluoroacetic anhydride. Benzil **9** may be obtained directly by reaction of the anion of cyanohydrin **2** with a substituted benzoic acid halide (where X is halo). Any of compounds **8** and **9** may be used as intermediates for conversion to imidazoles **10** (where R⁵ is alkyl) according to chemical procedures known by those skilled in the art and described by M. R. Grimmett, "*Advances in Imidazole Chemistry"* in **Advances in Heterocyclic Chemistry, 12,** 104 (1970). The conversion of **9** to imidazoles **10** is carried out by reaction with ammonium acetate and an appropriate aldehyde (R¹CHO) in acetic acid. Benzoin **8** may be converted to imidazoles **10** by reaction with formamide. In addition, benzoin **8** may be converted to imidazoles by first acylating with an appropriate acyl group (R¹CO-) and then treating with ammonium hydroxide. Those skilled in the art will recognize that the oxidation of the sulfide (where R⁵ is methyl) to the methylsulfone (-SO₂CH₃) may be carried out at any point along the way beginning with compounds **8**, and including oxidation of imidazoles **10**, using, for examples, reagents such as hydrogen peroxide in acetic acid, *m*-chloroperoxybenzoic acid (MCPBA) and potassium peroxymonosulfate (OXONE®).

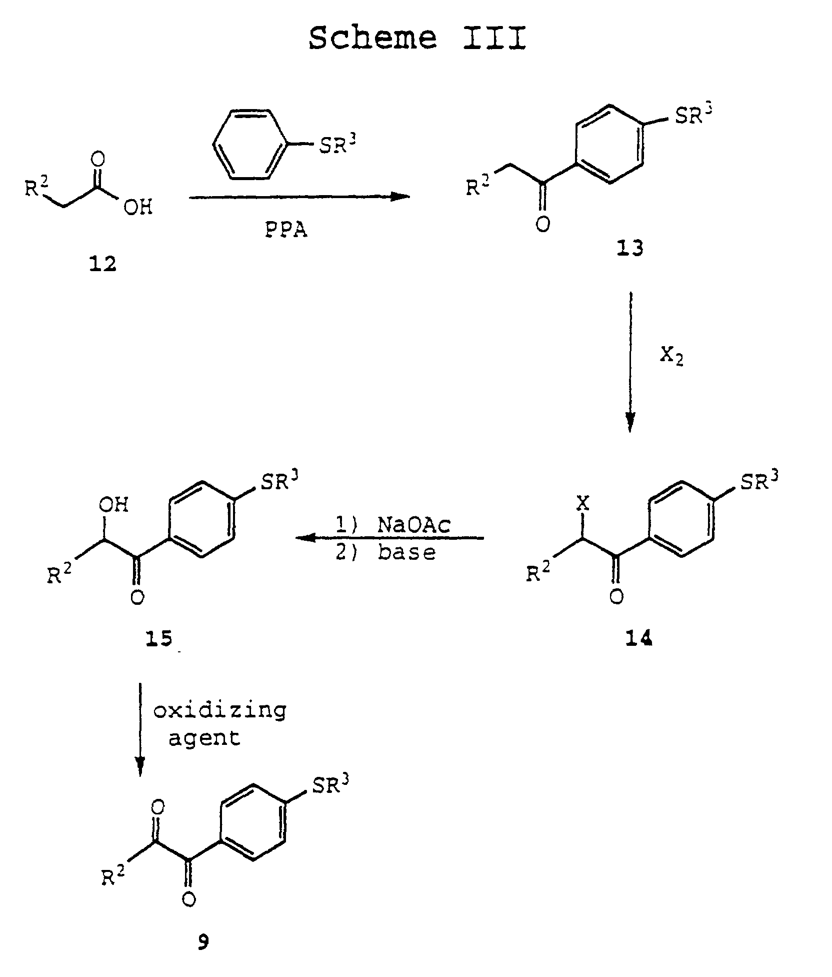

Alternative syntheses of benzoins and benzils may be carried out as described in Scheme III. Acylation of a thiobenzene derivative with an appropriately substituted acetic acid **12** using an acidic catalyst yields desoxybenzoin **13**. Some suitable acidic catalysts include polyphosphoric acid (PPA), sulfuric acid, titanium tetrachloride, ferric chloride and stannic chloride. The resulting desoxybenzoin **13** may be halogenated to give haloketone **14** (where X is halo}. Treatment of compound **14** with either water in a suitable co-solvent such as acetone, or with a carboxylate salt, followed by saponification with base, yields benzoin **15.** Examples of bases suitable for saponification include sodium bicarbonate, sodium carbonate, potassium carbonate, sodium hydroxide and ammonium hydroxide. Examples of appropriate carboxylate salts include sodium acetate and sodium benzoate. Compound **15** is converted to benzil **9** by reaction with a suitable oxidizing agent such as bismuth oxide or manganese dioxide. Alternatively, benzil **9** may be synthesized directly from desoxybenzoin **13** by treatment with an appropriate oxidizing agent, such as selenious acid (H₂SeO₃).

Haloketone **14** may be converted to imidazoles **10** by reaction either with formamide or with amidines.

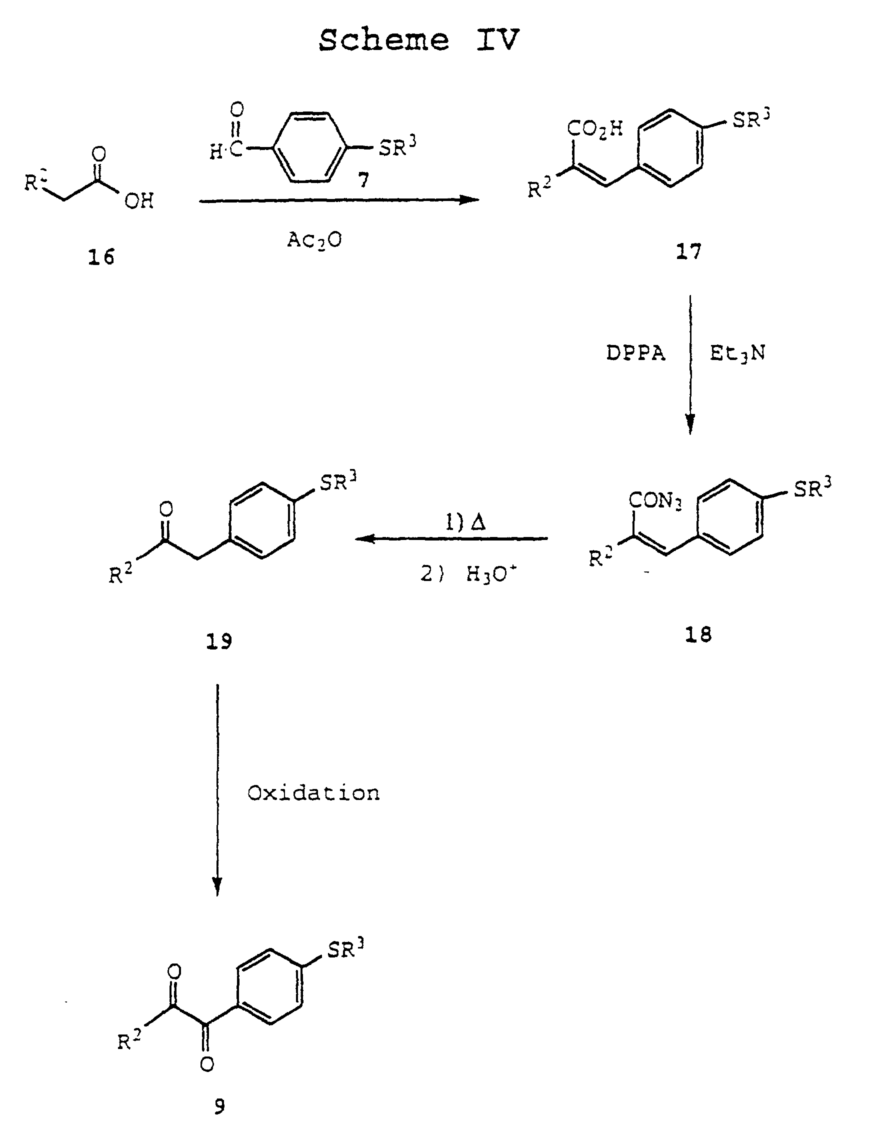

Scheme III outlines yet another method for the synthesis of benzil **9**. Reaction of a suitable acetic acid derivative 16 with an aromatic aldehyde **7** in acetic anhydride yields unsaturated acid **17**. Acid **17** is converted to acyl azides **18** by reaction with diphenylphosphoryl azide (DPPA) in the presence of a base such as triethylamine (Et₃N) or by reaction of an activated carboxyl derivative of **17,** such as an acid chloride or anhydride, with sodium azide. Decomposition of the acyl azide **18** by thermolysis, followed by hydrolysis with aqueous acid yields desoxybenzoin **19**. Compound **19** may be converted to benzil **9** by oxidation according to procedures discussed in Schemes II-III.

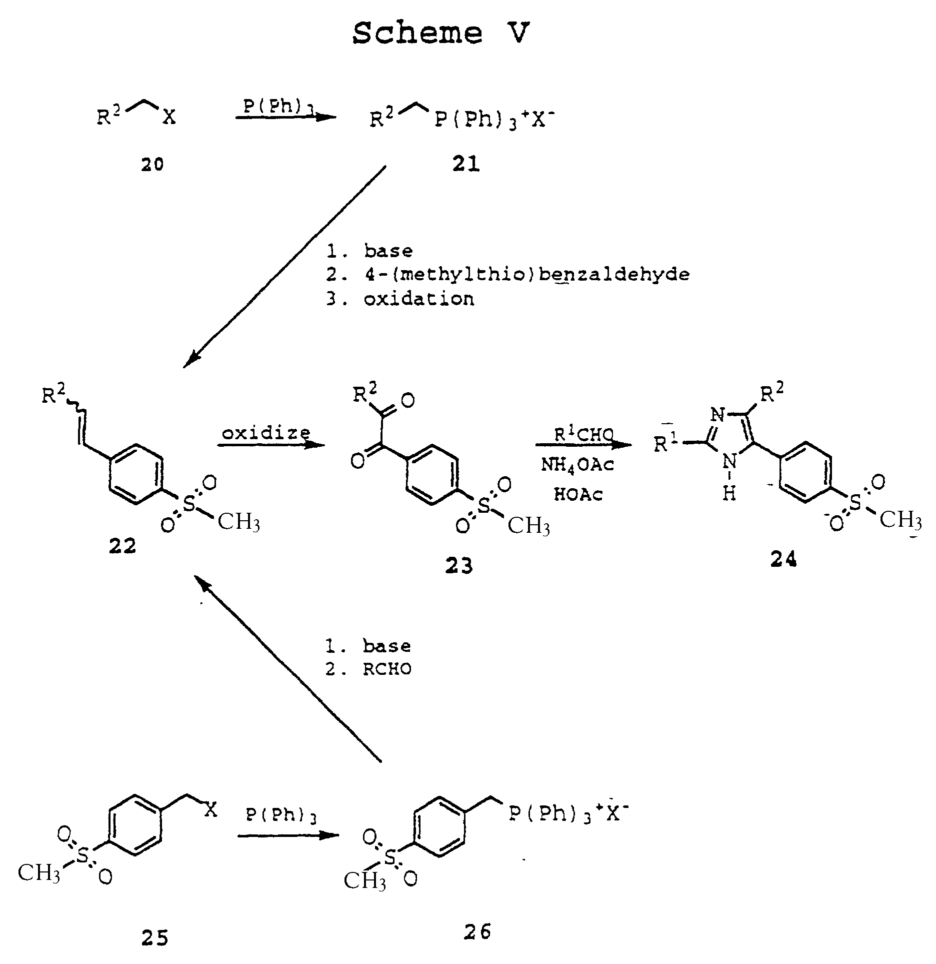

The compounds of the invention can be prepared according to the procedures in Scheme V. The subject imidazoles **24** are obtained from the condensation of diketones **23** with aldehydes R¹CHO in the presence of acetic acid and ammonium acetate, as previously described. Scheme V differs from previous synthetic routes in that it employs an α-diketone **23** that is obtained by oxidation of the olefinic intermediate **22**. This transformation is conveniently accomplished in one pot subjecting the olefin to potassium permanganate oxidation in acetic anhydride, according to a method of stilbene oxidation developed by Sharpless, *et .al.* [*J. Am. Chem. Soc.*, **93**, 3303 (1971)]. One may reduce the requirement for acetic anhydride by using a cosolvent, such as dioxane, ether, or methylene chloride. Oxidative sequences involving catalytic permanganate; catalytic osmium tetroxide followed by Swern oxidation; or epoxidation followed by oxidative rearrangement present alternatives to the conditions described.

Olefin **22** may be obtained using Wittig technology or other coupling protocols, such as transition metal mediated cross-coupling, silicon-based (Peterson) olefination, or sulfone-based (Julia) coupling. The commercial intermediate **25** (X=Cl) in the present scheme is transformed into an activated phosphorus compound, for example, a triphenylphosphonium salt. The phosphorus species **26** is deprotonated with a strong base, lithium ethoxide, generated *in situ* from n-butyllithium and ethanol. Other bases, such as sodium hydroxide or potassium t-butoxide present alternatives. A primarily aprotic medium could be employed, such as potassium *tert*-butoxide in tetrahydrofuran or sodium amide in dioxane. R²CHO represents an aryl aldehyde, such as benzaldehyde; an alkyl or cycloalkyl aldehyde, such as cyclohexanecarboxaldehyde; or a heterocyclic aldehyde, such as thiophene carboxaldehyde. It is recognized that the intermediate methyl sulfone **25** could be replaced with a different (e.g. ethyl sulfone) or sulphonamide with similar results.

Olefin **22** may also be obtained by allowing a suitable phosphonium salt **21** (X=Cl, Br, OTs, or I) to react in the presence of strong base, such as lithium ethoxide with 4-(thiomethyl)benzaldehyde. Oxidation of the sulfur atom to the sulfone is conveniently accomplished with Oxone® at this stage, but could be accomplished with other reagents, such as hydrogen peroxide or m-chloroperbenzoic acid, or could be effected at another place in the sequence. The oxidation step could be entirely eliminated by employing 4-(methylsulphonyl)benzaldehyde in the place of 4-(thiomethyl)benzaldehyde.

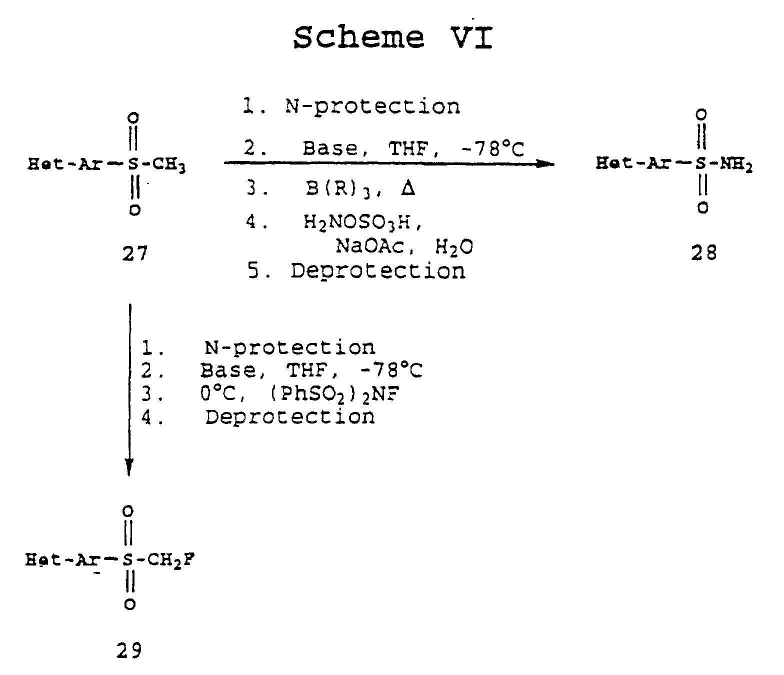

Synthetic Scheme VI shows the three step procedure used to prepare sulfonamide antiinflammatory agents **28** and the two step procedure used to prepare fluoromethyl sulfone antiinflammatory agents **29** from their corresponding methyl sulfones **27**. In step one, THF solutions of the methyl sulfones **27** at -78°C are treated with an alkyllithium reagent, e.g., methyllithium, n-butyllithium, lithium diisopropylamide (LDA), etc. In step two, the anions generated in step one are treated with an organoborane, e.g., triethylborane, tributylborane, etc., at -78°C then allowed to warm to ambient temperature prior to stirring at reflux. An alternative to the boron chemistry involves room temperature alkylation, such as with trimethylsilylmethylhalides, followed by treatment with tetrabutylammonium fluoride (1M in THF). In step three, an aqueous solution of sodium acetate and hydroxylamine-O-sulfonic acid is added to provide the corresponding sulfonamide antiinflammatory agents **28** of this invention. Alternatively, the anion solutions generated in step one may be warmed to 0°C and treated with N-fluorodibenzenesulfonamide to provide the corresponding fluoromethyl sulfone antiinflammatory agents **29** of this invention.

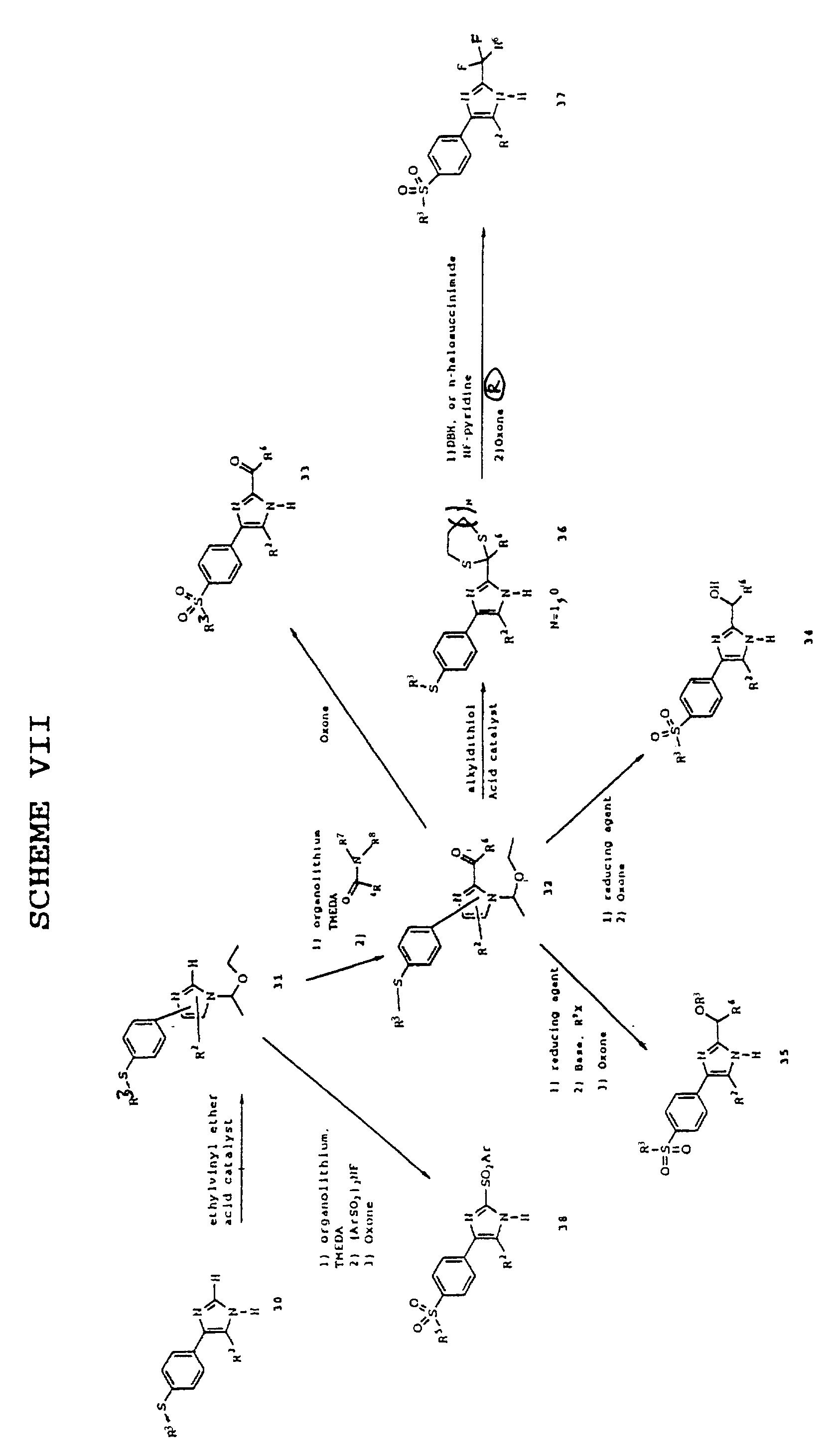

Synthetic Scheme VII outlines further medications at position 2 of the imidazole compounds of this invention. Compound **3**0 may be obtained via the condensation of benzoin **8** with formamide as discussed above. The parent imidazole **30,** maybe converted to the ethoxyethyl derivatives **31** by treatment with ethylvinylether in the presence of an acid catalyst. Examples of suitable acid catalysts are dichloroacetic acid, p-toluenesulfonic acid, methanesulfonic acid and hydrogen chloride. Alternatively the ethoxyethyl group may be incorporated by chemical procedures known to those skilled in the art and described by T.S. Manoharan and R.S. Brown in *J. Org. Chem.*, **53,** 1107-1110 (1988). Due to the tautomeric nature of the nitrogen atom, two compounds **31** are obtained at the end of this process. Both compounds **31** are carried on to subsequent steps as a mixture because this group is ultimately removed and upon removal one compound is obtained from the reaction sequence of interest. A series of acyl derivatives can be obtained by conversion of **31** into **32** and then **33** by lithiation via the process of Manoharan and Brown, followed by treatment of the resulting lithium anion with amides such as dimethylformamide (R⁶=H, R⁷ and R⁸=CH₃), dimethyl acetamide(R⁶,R⁷, R⁸=CH₃) or with amides where R⁷ and R⁸ = OMe and CH₃ and wherein R⁶ may include hydrogen, lower alkyl, aryl, aralkyl, and haloalkyl. Subsequent treatment with Oxone® results in compounds **33,** where R⁶ is as defined above. The preparation of these amides involves the treatment of the appropriate carboxylic acid with N,O-dimethylhydroxyamine chloride in the presence of triethylamine, and 2-chloromethylpyridinium iodide at room temperature in methylene chloride. The preparation of the amide where R⁷ and R⁸ are OMe and methyl, and R⁶ is CF₃ can be accomplished by those skilled in art by following the published procedure in *J. Org. Chem*,. **56,** 4260 (1991). Finally, the lithium anion may be treated with other electrophiles, for example, trifluoroacetone or N-fluorodibenzenesulfonamide resulting in compounds where imidazole position 2 is [CH₃(OH)CF₃] or phenylsulfonyl **38,** respectively. If further modification of the acyl group in compounds **32** is desired, the imidazole nitrogen at position 1 is left protected. This process is exemplified by the conversion of compounds **32** into **34, 35, 36, 37** and **38** where R⁶ is defined as stated above. The conversion of **32** into **34** can be performed by treatment of compound **32** with a reducing agent followed by treatment with Oxone®. Some examples of reducing agents are sodium borohydride, lithium borohydride, zinc borohydride, lithium triethylborohydride, RED-AL, borane, alane, and diisopropylaluminum hydride. To synthesize ether compounds **35** (R⁹ is lower alkyl, alkenyl or aralkyl), ketones **32** are reduced to the corresponding alcohols using a suitable reducing agent as defined above. The resulting alcohol is treated with a strong base, such as sodium hydride or lithium hydride in a suitable solvent, such as dimethylformamide, dimethylsulfoxide or tetrahydrofuran, and then reacted with the appropriate alkyl, aralkyl or alkenyl halide. Oxidation of the sulfide to the methyl sulfone results in concomitant loss of the nitrogen protecting group to produce compounds **35.** The conversion of compound **32** to **37** may be carried out by those that are skilled in the art using procedures described by Susan C. Sondej and John A. Katzenellenbogen [*J.Org Chem*., **51**, 3508-3513 (1986).

The following examples contain detailed descriptions of the methods of preparation of compounds of Formula I. These detailed descriptions fall within the scope, and serve to exemplify, the above described General Synthetic Procedures which form part of the invention. These detailed descriptions are presented to illustrate the invention. All parts are by weight and temperatures are in Degrees centigrade unless otherwise indicated. All compounds showed NMR spectra consistent with their assigned structures.

### Example 1

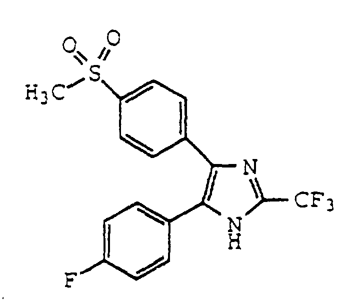

### 5-(4-Fluorophenyl)-4-(4-methylsulfonylphenyl)-2-trifluoromethyl-1H-imidazole

### Method A

### Step 1: Preparation of 4-fluorophenyl-4'-methylthiophenyl-α-carboxystilbene (cis and trans)

4-Fluorophenylphenylacetic acid (23.5 g, 0.152 mol), *p*-methylthiobenzaldehyde (23.2 g, 0.152 mol), and triethylamine (Et₃N) (16.2 g, 0.16 mol) were dissolved in acetic anhydride (100 ml) and heated to reflux for 8 hours. After cooling, the contents were poured into water (500 ml) and stirred for 2 hours. The mixture was extracted with methylene chloride, dried (MgSO₄) and concentrated *in vacuo.* The residue was dissolved in methanol (300 ml), 2N KOH (potassium hydroxide) (100 ml) was added, and the contents were placed on a steam bath for 30 minutes. The mixture was cooled in ice and conc. HCl was added to reach pH 3, forming a precipitate. The precipitate was filtered, air dried and recrystallized from ethanol (EtOH) to give 24 g (50%) of 4-fluorophenyl-4'-methylthiophenyl-α-carboxystilbene (cis and trans) in the first crop: Anal. Calc'd. for C₁₆H₁₃FO₂S (M.W. = 288.34): C, 66.65; H, 4.54. Found: C, 66.71; H, 4.49.

### Step 2 Preparation of 1-(4-fluorophenyl)-2-(4-methylthiophenyl)-1-ethanone

A solution of the stilbene carboxylic acid (11.8 g, 41 mmol) from step 1, diphenylphosphoryl azide (PhO)₂PON₃ (12.5 g, 1.1 equiv.) and Et₃N (5 g, 1.2 equiv.) in toluene (200 ml) was stirred for 1 hour at 25°C. The contents were poured into water (1 L), the layers separated and the organic layer was washed with brine. After filtration through MgSO₄, the toluene solution was heated at reflux for 1.5 hours under a nitrogen atmosphere. After cooling, the solvent was concentrated *in vacuo*, and the residue was heated to reflux with 2:1 acetic acid:water (50 ml) for 2 hours. After cooling, water (50 ml) was added and the precipitate was filtered, air dried and recrystallized from EtOH to give the ketone (7 g, 65%): m.p. 139-140°C. Anal. Calc'd. for C₁₅H₁₃FOS (M.W. 260.33): C, 69.21; H, 5.03; S, 12.32. Found: C, 68.94; H, 5.09; S, 12.15.

### Step 3 Preparation of 1-(4-fluorophenyl)-2-(4-methylsulfonylphenyl)-1-ethanone

A solution of 1-(4-fluorophenyl)-2-(4-methylthiophenyl)-1-ethanone from step 2 (15.7 g, 60 mmol) in methanol (500 ml) and tetrahydrofuran (200 ml) was stirred at 25°C while a solution of OXONE® (40 g, excess) in water (150 ml) was added over 45 minutes. The entire reaction mixture was stirred at 40°C for 3 hours. Water (500 ml) was added and the precipitate was filtered and air dried. The desired ketone was recrystallized by dissolving in warm chloroform and adding hexane until cloudy. The precipitate formed by cooling the solution in a refrigerator was filtered and air dried to give the titled ketone (15 g, 80%): m.p. 185-187°C. Anal. Calc'd. for C₁₅H₁₃FO₃S (M.W. 292.33) : C, 61.63; H, 4.48; S, 10.97. Found: C, 61.72; H, 4.34; S, 11.01.

### Step 4 Preparation of 1-(4-methylsulfonylphenyl)-2-(4-fluorophenyl)-ethane-1,2-dione

A mixture of ketone from step 3 (1 g, 3.5 mmol), selenious acid (H₂SeO₃) (550 mg, 1.25 equiv.), dioxane (10 ml) and water (1 ml) were heated to reflux for 4 hours and cooled. The mixture was filtered and the filtrate evaporated. The residue was triturated with cold methanol and filtered to give 620 mg (60%) of the dione: m.p. 174-175°C. Anal. Calc'd. for C₁₅H₁₁FO₄S (M.W. 306.31): C, 58.82; H, 3.62; S, 10.47. Found: C, 58.70; H, 3.60; S, 10.70.

### Step 5 Preparation of 5-(4-fluorophenyl)-4-(4-methylsulfonylphenyl)-2-trifluoromethyl-1H-imidazole

A mixture of dione from step 4 (1 g, 4.2 mmol), ammonium acetate (2.2 g, 5 equiv.), trifluoroacetaldehyde ethyl hemiacetal (2 g, 5 equiv.). and acetic acid (20 ml) was heated to reflux for 16 hours under an argon atmosphere. After cooling, the contents were poured into water (100 ml) and neutralized with ammonium hydroxide (NH₄OH) to pH 6.5. The precipitate was filtered, dried in an oven at 80°C and recrystallized from ethyl acetate and hexane to give 5-(4-fluorophenyl)-4-(4-methylsulfonylphenyl) -2-trifluoromethyl-1H-imidazole (550 mg, 34%): m.p. 249-250°C (dec.). Anal. Calc'd. for C₁₇H₁₂F₄N₂O₂S (M.W. 384.35): C, 53.13; H, 3.15; N, 7.29; S, 8.34. Found: C, 52.91; H, 2.99; N, 6,97; S, 8.56.

### Method B

### Step 1 Preparation of 1-(4-fluorophenyl)-2-(4-methylsulfonylphenyl)-2-bromoethane-1-one

A suspension of 1-(4-fluorophenyl)-2-(methylsulfonylphenyl)-ethane-1-one (Method A, step 3) (24 g, 82 mmol) in acetic acid (500 ml) was warmed to 80°C with stirring, and a solution of bromine (14.38 g, 0.09 mol) in acetic acid (50 ml) was added over 15 minutes. The reaction mixture became homogeneous, was cooled and stirred an additional 3 hours at 25°. The contents were poured into water (3 L) and extracted with ethyl acetate. The organic phase was washed with aqueous NaHCO₃, brine and dried (MgSO₄). The dried solution was concentrated *in vacuo* to give a solid which was triturated with cold ether to produce 26.5 g (87%) of the desired haloketone: m.p. 144-145°C. Anal. Calc'd. for C₁₅H₁₂BrFO₃S: C, 48.53; H, 3.26; Br, 21.52; S, 8.65. Found: C, 48.34; H, 3.28; Br, 21.19; S, 8.42.

### Step 2 Preparation of 5-(4-fluorophenyl)-4-(4-methylsulfonylphenyl)-2-trifluoromethyl-1H-imidazole

A mixture of 1-(4-fluorophenyl)-2-(4-methylsulfonylphenyl)-2-bromo-ethane-1-one from step one (3 g, 8.2 mmol), trifluoroacetamidine (925 mg, 8.2 mmol), NaHCO₃ (690 mg, 8.2 mmol) and n-butanol (30 ml) was heated to reflux for 18 hours, cooled and concentrated. The residue was dissolved in water and methylene chloride, and the organic phase was separated, dried (Na₂SO₄) and concentrated. The residue was purified by chromatography on silica gel using methanol/toluene (5/95) and the crude product was recrystallized from ethyl acetate and hexane to give 700 mg (22%) of purified material which was identical to the material obtained in Method A, step 5.

### Method C

### Step 1 Preparation of 4-fluoro-α-(trimethylsilyl)benzeneacetonitrile

To a solution of 50.0 g (403 mmol) of 4-fluorobenzaldehyde in methylene chloride (100 ml) stirring in an ice bath under a nitrogen atmosphere, was added dropwise 54 ml (40 g, 403 mmol) of trimethylsilyl cyanide. After the addition was complete, anhydrous zinc iodide (10 mg) was,added, and stirring continued overnight while the mixture warmed to room temperature. The solvent was removed by distillation under reduced pressure, and continued distillation under high vacuum gave the title compound (82.2 g) as a very pale straw yellow liquid: bp. 98-100°C at 0.8 mm Hg.

### Step 2 Preparation of 1-(4-fluorophenyl)-2-(4-methylthiophenyl)-2-hydroxy-ethane-1-one

To a cold (-70°C) stirred solution of lithium bis(trimethylsilylamide) (11 ml of 1.0 M in tetrahydrofuran, 11 mmol) in tetrahydrofuran (20 ml) under an argon atmosphere was added a solution of trimethylsilane (TMS) protected cyanohydrin of 4-fluorobenzaldehyde from step 1 (2.2 g, 0.01 mole) over 2 minutes and the reaction mixture was stirred cold (-70°C) for 15 minutes. A mixture of 4-thiomethylbenzaldehyde (1.52 g, 0.01 mol) in tetrahydrofuran (10 ml) was added and the reaction mixture was warmed to -50°C for 1 hour. Saturated ammonium chloride (NH₄Cl) (20 ml) was added and the mixture was warmed to 25°C. Water (20 ml) and methylene chloride (CH₂Cl₂) (25 ml) were added and the organic phase was separated and dried (Na₂SO₄). The organic solvent was evaporated and the residue was dissolved in methanol (10 ml) and treated with 5% H₂SO₄ (5 ml) at 25°C overnight. Ether (25 ml) and water (50 ml) were added and the ether layer was separated. The ether layer was then stirred vigorously with 0.5N NaOH (30 ml) for 15 minutes. The ether layer was dried (Na₂SO₄), evaporated, and the residue was purified by chromatography on silica gel with ethyl acetace/hexane (20/80) to yield 1.8 g (67%) of the titled ketone. Anal. Calc'd. for C₁₅H₁₃FO₂S (M.W. 276.33): C, 65.20; H, 4.74; S, 11.60. Found: C, 65.10; H, 4.80; S, 11.75.

### Step 3 Preparation of 1-(4-methylthiophenyl)-2-(4-fluorophenyl)-ethane-1,2-dione

A mixture of ketone from step 2 (1.25 g, 4.5 mmol) and bismuth oxide (Bi₂O₃) (2.7 g, 1.2 equiv.) in acetic acid (20 ml) was warmed to 80°C for 30 minutes. After cooling, the mixture was filtered through Celite® filter agent and the filtrate was concentrated. The residue was dissolved in hot methanol and filtered. The solvent was concentrated under a nitrogen stream, placed in the refrigerator and the precipitate formed was filtered to give 1 g. (83%) of titled dione: m.p. 96-97°C. Anal. Calc'd. for C₁₅H₁₁FO₂S (M.W. 274.31): C, 65.68; H, 4.04; S, 11.69. Found: C, 65.42; H, 3.85; S, 11.42.

### Step 4 Preparation of 5-(4-fluorophenyl)-4-(4-methylthiophenyl)-2-trifluoromethyl-1H-imidazole

Following the procedure of Example 1, Method A, step 5, and using the dione from step 3 above (1g, 3.6 mmol), ammonium acetate (2.2 g, 5 equiv.), trifluoroacetaldehyde ethyl hemiacetal (1.25 g, 1.75 equiv.) and acetic acid (30 ml) gave a crude imidazole which was purified by chromatography on silica gel with toluene to afford 570 mg (45%) of the title compound.

### Step 5 Preparation of 5-(4-fluorophenyl)-4-(4-methylsulfonylphenyl)-2-trifluoromethyl-1H-imidazole

A solution of imidazole from step 4 above (550 mg, 1.6 mmol) in methanol (10 ml) was stirred at 25°C while a solution of OXONE® (1.6 g) in water (3 ml) was added. The reaction mixture was stirred for 3 hours at room temperature, poured into water (50 ml) and extracted with methylene chloride. After drying the organic layer (Na₂SO₄) and concentration, the residue was created with cold ethyl acetate, filtered, and air dried to give 490 mg (80%) of the titled material which was identical with Examples 1 Method A and Method 3.

### Example 2

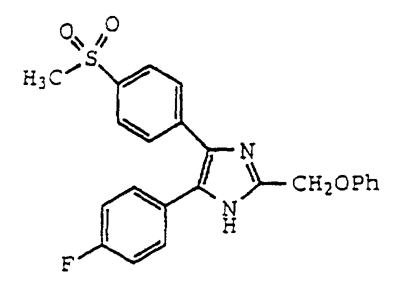

### 5-(4-Fluorophenyl)-4-(4-methylsulfonylphenyl)-2-phenoxymethyl-1H-imidazole

In a manner similar to Example 1, 1-(4-methylsulfonylphenyl)-2-(4-fluorophenyl)-ethane-1,2-dione (Example 1, Method A, step 4) (500 mg, 1.6 mmol), ammonium acetate (1.1 g, 5 equiv.), phenoxyacetaldehyde dimethyl acetal (800 mg, 2.5 equiv.) and acetic acid (20 ml) gave 4-(4-methylsulfonylphenyl)-5-(4-fluorophenyl)-2-phenoxymethyl-1H-imidazole (110 mg, 12%) after chromatography on silica gel using ethyl acetace/toluene (30/70) as the eluent and recrystallization from ethyl acetate and hexane: m.p. 189-190°C. Anal. Calc'd. for C₂₃H₁₉N₂O₃FS•1/4 H₂O (M.W. 426.98): C, 64.70; H, 4.60; N, 6.56; S, 7.51. Found: C, 64.62; H, 4.29; N, 6.22; S, 7.88.

### Example 3

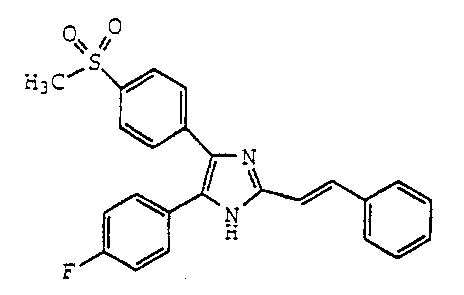

### 5-(4-Fluorophenyl)-4-(4-methylsulfonylphenyl)-2-(2-phenyl-trans-eth-1-ene)-1H-imidazole

Following the procedure of Example 1, 1-(4-methylsulfonylphenyl)-2-(4-fluorophenyl)-ethane-1,2-dione (Example 1, Method A, step 4) (500 mg, 1.6 mmol), NH₄OAc (1.1 g, 14.3 mmol), trans-cinnamaldehyde (250 mg, 1.89 mmol) and acetic acid (20 ml) gave 121 mg (18%) of the desired imidazole after chromatography on silica gel with methanol:toluene (3:97) as the eluent and recrystallization from cyclohexane: m.p. 221-224°C. Anal. Calc'd. for C₂₄H₁₉N₂O₂FS (M.W. 418.49): C, 68.88; H, 4.58; N, 6.69; S, 7.66. Found: C, 68.44; H, 4.57; N, 6.45; S, 7.61.

### Example 4

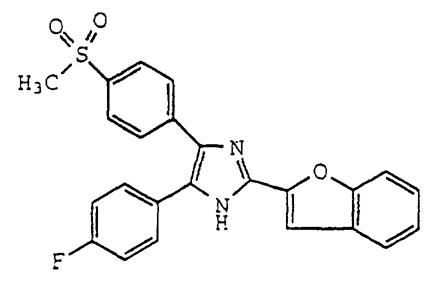

### 2-(2-Benzofuryl)-5-(4-fluorophenyl)-4-(4-methylsulfonylphenyl)-1H-imidazole

Following the procedure of Example 1, Method A, 1-(4-methylsulfonylphenyl)-2-(4-fluorophenyl)-ethane-1,2-dione (500 mg, 1.6 mmol), NH₄OAc (1.1 g, 14.3 mmol), benzofuran-2-carboxaldehyde (280 mg, 1.92 mmol) and acetic acid (20 ml) gave 2-(2-benzofuryl)-5-(4-fluorophenyl)-4-(4-methylsulfonylphenyl)-1H-imidazole (575 mg, 76%) after recrystallization from isopropanol and water: m.p. 235-237°C. Anal. Calc'd. for C₂₄H₁₇N₂O₃FS•2H₂O (M.W. 468.51): C, 61.53; H, 4.52; N, 5.98; S, 6.84. Found: C, 61.39; H, 4.27; N, 5.79; S, 6.82.

### Example 5

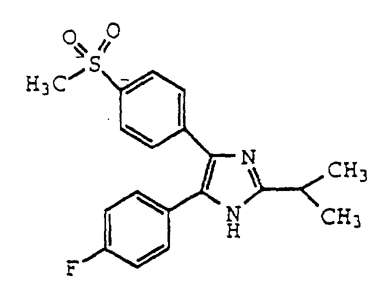

### 5-(4-Fluorophenyl)-2-isopropyl-4-(4-methylsulfonylphenyl)-1H-imidazole

Following the procedure of Example 1, Method A, 1-(4-methylsulfonylphenyl)-2-(4-fluorophenyl)-ethane-1,2-dione (Example 1, Method A, step 4) (500 mg, 1.6 mmol), ammonium acetate (1.1 g, 5 equiv.), isobutyraldehyde (140 mg, 1.25 equiv.) and acetic acid (20 ml) gave 4-(4-methylsulfonylphenyl)-5-(4-fluorophenyl)-2-isopropyl-1H-imidazole (380 mg, 66%) after recrystallization from ethyl acetate and hexane: m.p. 214-215°C. Anal. Calc'd. for C₁₉H₁₉N₂O₂FS (M.W. 358.44): C, 63.67; H, 5.34; N, 7.82; S, 8.95. Found: C, 63.49; H, 5.41; N, 7.62; S, 8.97.

### Example 6

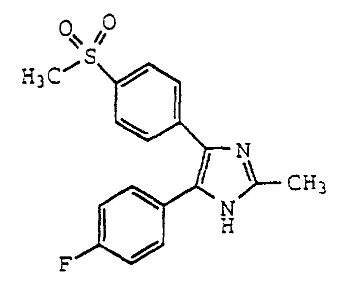

### 5-(4-Fluorophenyl)-2-methyl-4-(4-methylsulfonylphenyl)-1H-imidazole

### Step 1 Preparation of 1-(4-methylthiophenyl)-2-(4-fluorophenyl)-1-ethanone

A mixture of polyphosphoric acid (160 g), 4-fluorophenylacetic acid (10 g, 65 mmol) and thioanisole (10 g, 1.2 equiv.) was heated and mechanically stirred to 125°C for 20 minutes and cooled to 50°C. Ice was added rapidly with external cooling so as to maintain the internal temperature at about 50°C. After excess ice was added, the reaction was stirred for 30 minutes, filtered, and the filter cake air dried. The crude ketone was recrystallized from EtOH to give 10 g (60%) of ketone: Anal. Calc'd. for C₁₅H₁₃FOS (M.W. 260.33): C, 69.21; H, 5.03; S, 12.32. Found: C, 69.14; H, 5.00; S, 12.23.

### Step 2 Preparation of 1-(4-methylthiophenyl)-2-bromo-2-(4-fluorophenyl)-1-ethanone

A mixture of 1-(4-methylthiophenyl)-2-(4-fluorophenyl)-1-ethanone from step 1 (9.48 g, 36 mmol) and acetic acid (200 ml) was stirred at 15°C and a solution of bromine (5.80 g, 0.036 mol) in acetic acid (10 ml) was added dropwise over 5 minutes. The reaction mixture was stirred at 25°C for an additional 2 hours. The mixture was poured into water (400 ml) with stirred for one hour. The precipitate formed was filtered, air dried and dried *in vacuo* at ambient temperature to give 11.4 g (93%) of the bromoketone: Anal. Calc'd, for C₁₅H₁₂BrFOS (M.W. 339.23): C, 53.11; H, 3.57; S, 9.45; Br, 23.55. Found: C, 51.34; H, 3.34; S, 9.62; Br, 23.40.

### Step 3 Preparation of 1-(4-methylthiophenyl)-2-(4-fluorophenyl)-1-ethanone-2-acetate

A mixture of 1-(4-methylthiophenyl)-2-bromo-2-(4-fluorophenyl)-1-ethanone from step 2 (7.26 g, 21 mmol), sodium acetate (NaOAc) (8.6 g, 5 equiv.), dimethoxyethane (105 ml) and water (70 ml) was heated to reflux for 4 hours, cooled, poured into water (400 ml) and extracted with methylene chloride. The organic-extract was dried (MgSO₄), filtered, concentrated *in vacuo* and the residue was purified by chromatography on silica gel with toluene to give 5 g (74%) of the acetate which slowly crystallized upon standing: Anal. Calc'd. for C₁₇H₁₅FO₃S (M.W. 318.37): C, 64.14; H, 4.75; S, 10.07. Found: C, 63.89; H, 4.68; S, 9.81.

### Step 4 Preparation of 4-(4-methylthiophenyl)-5-(4-fluorophenyl)-2-methyl-1H-imidazole

The 1-(4-methylthiophenyl)-2-(4-fluorophenyl)-1-ethanone-2-acetate from step 3 (700 mg, 2.2 mmol) was heated to 180°C in formamide (10 ml) under a nitrogen atmosphere for 2 hours. After cooling, the mixture was poured into water (50 ml) and extracted with methylene chloride. The extract was dried (Na₂SO₄), evaporated, and the residue was purified by chromatography on silica gel with toluene/methanol (95/5) containing 0.5% NH₄OH co give 132 mg (20%) or imidazole: Anal. Calc'd. for C₁₇H₁₅N₂FS (M.W. 298.38): C, 68.43; H, 5.07; N, 9.39; S, 10.17. Found: C, 68.14; H, 5.18; N, 9.09; S, 10.31.

### Step 5 Preparation of 4-(4-methylsulfonylphenyl)-5-(4-fluorophenyl)-2-methyl-1H-imidazole

In a manner similar to Example 1, Method C, step 4, 4-(4-methylthiophenyl)-5-(4-fluorophenyl)-2-methyl-1H-imidazole (114 mg, 0.4 mmol) from step 4 was converted to 5-(4-fluorophenyl)-2-methyl-4-(4-mechylsulfonylphenyl)-1H-imidazole (107 mg, 95%): Anal. Calc'd. for C₁₇H₁₅N₂O₂FS (M.W. 330.38): C, 61.80; H, 4.58; N, 8.48; S, 9.71. Found: C, 61.90; H, 4.61; N, 68.35; S, 9.39.

### Example 7

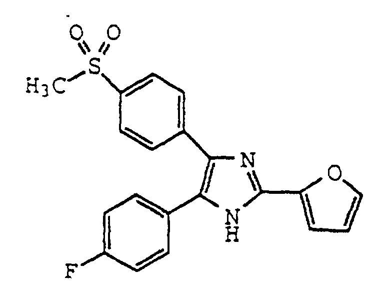

### 5-(4-Fluorophenyl)-2-(2-furyl)-4-(4-methylsulfonylphenyl)-1H-imidazole

In a manner similar to Example 1, Method A, 1-(4-methylsulfonylphenyl)-2-(4-fluorophenyl)-ethane-1,2-dione (Example 1, Method A, step 4) (500 mg, 1.6 mmol), ammonium acetate (1.1 g, 5 equiv.), 2-furaldehyde (170 mg, 1.1 equiv.) and acetic acid (20 ml) gave 308 mg (50%) of product after chromatography on silica gel with ethyl acetate/toluene (50/50) and recrystallization from isopropanol: m.p. 244-246°C (dec.). Anal. Calc'd. for C₂₀H₁₅N₂O₃FS (M.W. 382.42): C, 62.82; H, 3.95; N, 7.33; S, 8.38. Found: C, 62.63; H, 4.06; N, 7.13; S, 8.39.

### Example 8

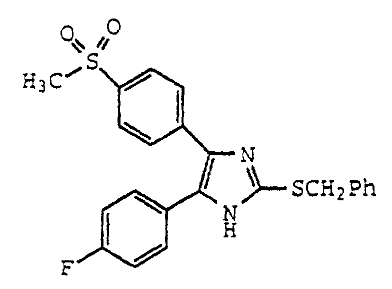

### 2-Benzylthio-5-(4-fluorophenyl)-4-[4-(methylsulfonyl)phenyl]-1H-imidazole

A mixture of S-benzylisothiourea hydrochloride (300 mg, 1.5 mmol), 1-(4-fluorophenyl)-2-(4-methylsulfonylphenyl)-2-bromoethan-1-one (Example 1, Method B, step 1) (500 mg, 1.3 mmol) and NaHCO₃ (500 mg, 6 mmol) in EtOH (10 ml) was heated to reflux for 3 hours. After evaporation of the solvent, the residue was treated with water (25 ml⁻) and extracted with methylene chloride (25 ml). After drying (MgSO₄), the organic phase was evaporated. The residue was purified by chromatography on silica gel, eluting with ethyl acetate/toluene (25/75). The crude material was recrystallized from toluene and hexane to give 100 mg (17%) of titled product: m.p. 155-156°C. Anal. Calc'd. for C₂₃H₁₉FN₂O₂S₂: C, 62.99; H, 4.37; N, 6.39; S, 14.62. Found: C, 62.93; H, 4.41; N, 5.98; S, 14.33.

### Example 9

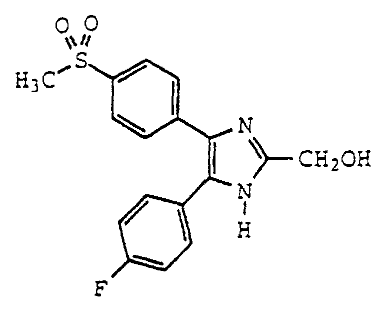

### 5-(4-Fluorophenyl)-2-hydroxymethyl-4-[4-(methylsulfonyl)phenyl]imidazole

### Step 1 Preparation of 5-(4-fluorophenyl)-4-(4-methylthiophenyl)-1H-imidazole

1-(4-Fluorophenyl) -2-(4-methylthiophenyl)-2-hydroxy-ethane-1-one (Example 1, Method C, step 1) (12.25 g, 44 mmol) was heated at 200°C in formamide (200 ml) for 4 hours under a nitrogen atmosphere. After cooling, the reaction was poured into 1 L of ice water and stirred rapidly for 30 minutes. The precipitate was filtered and air dried. The crude material was recrystallized from EtOH to give 7.5 g (60%) of imidazole: m.p. 195-196°C (dec.). Anal. Calc'd. for C₁₆H₁₃N₂FS: C, 67.58; H, 4.61; N, 9.85; S, 11.28. Found: C, 67.51; H, 4.51; N, 9.68; S, 11.33.

### Step 2 Preparation of 5-(4-fluorophenyl)-4-(4-methylthiophenyl)-1-(1-methyl-1-ethoxymethyl)-1H-imidazole

A mixture of the imidazole described in step 1 (500 mg, 1.7 mmol), ethyl vinyl ether (250 mg, 3.4 mmol) and dichloroacetic acid (200 mg, 1.5 mmol) was heated to reflux in toluene (5 ml) for 6 hours. After cooling, the mixture was stirred with 1N NaOH (2 ml) for 30 minutes. The organic layer was separated and dried over K₂CO₃ (anhyd.) and the residue, after evaporation, was purified by chromatography on silica gel, eluting with toluene/methanol (95/5), to give 520 mg (86%) of the titled compound as an oil: Anal. Calc'd. for C₂₀H₂₁N₂OFS: C, 67.39; H, 5.94; N, 7.86; S, 8.99. Found: C, 67.51; H, 5.88; N, 7.55; S, 9.05.

### Step 3 Preparation of 5-(4-fluorophenyl)-4-(4-methylthiophenyl)-1H-imidazole-2-carboxaldehyde

A solution of the compound from step 2 (500 mg, 1.4 mmol) and tetramethylethylenediamine (TMEDA) (186 mg, 1.6 mmol) in tetrahydrofuran (8 ml) was cooled to -70°C under an argon atmosphere. n-Butyllithium (1.2 ml of 1.6M solution in hexane, 1.9 mmol) was added and the solution stirred at -70°C for 15 minutes. Dimethylformamide (DMF) (140 mg, 1.9 mmol) was added and the solution was warmed to 0°C. The reaction was quenched by the addition of saturated NaHCO₃ solution (2 ml) and extracted with diethyl ether. The organic phase was separated and dried (Na₂SO₄). The drying agent was filtered and the filtrate concentrated *in vacuo*. The residue was treated with 2N HCl (5 ml) in EtOH (10 ml) at room temperature for 24 hours. The reaction solution was made basic with NaHCO₃, diluted with water (20 ml) and extracted with methylene chloride. After evaporation, the residue was purified by chromatography on silica gel, eluting with toluene/methanol (90/10), to give 350 mg (70%) of titled compound which was used immediately without further purification.

### Step 4 Preparation of 5-(4-fluorophenyl)-2-hydroxymethyl-4-(4-methylthiophenyl)-1H-imidazole

A solution of compound from step 3 (350 mg, 1 mmol) and NaBH₄ (75 mg, 2 eq.) in methanol (10 ml) was stirred for 1 hour at 25°C. The reaction was acidified with 2N HCl (4 ml), stirred for 2 hours and placed on a steam bath for 2 minuces. After cooling, the mixture was neutralized with aqueous NaHCO₃ solution, extracted with methylene chloride, dried (MgSO₄) and evaporated. The residue was purified by chromatography on silica gel, eluting with toluene/methanol (90/10). The crude product was recrystallized from ethyl acetate/hexane to give 240 mg (78%) of pure 5-(4-fluorophenyl)-2-hydroxymethyl-4-(4-methylthiophenyl)-1H-imidazole: m.p. 108-110°C. Anal. Calc'd. for C₁₇H₁₅N₂OFS: C, 64.95; H, 4.81; N, 8.91; S, 10.20. Found: C, 64.69; H, 4.92; N, 8.79; S, 10.41.

### Step 5 Preparation of 5-(4-fluorophenyl)-2-hydroxymethyl-4-[4-(methylsulfonyl)phenyl]imidazole

A mixture of 2-hydroxymethyl-imidazole from step 4 (150 mg, 0.4 mmol), OXONE® (450 mg, excess), water (2 ml), methanol (5 ml) and tetrahydrofuran (3 ml) was stirred at 25°C for 2 hours. The reaction mixture was diluted with water (20 ml) and extracted with methylene chloride. After removal of solvent, the crude material was recrystallized from toluene to give 80 mg (70%) of 4-(4-fluorophenyl)-2-hydroxymethyl-5-(4-methylsulfonylphenyl)-1H-imidazole: m.p. 115-117°C. Anal. Calc'd. for C₁₇H₁₅N₂O₃FS•H₂O: C, 56.03; 4.70; N, 7.69; S, 8.80. Found: C, 56.02; H, 4.64; N, 7.45; S, 8.90.

### Example 10

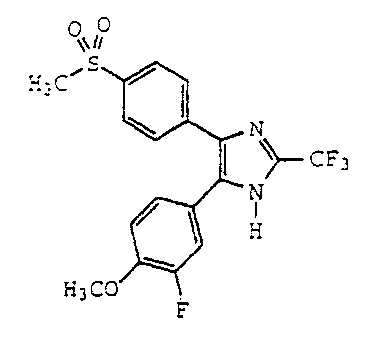

### 5-(3-Fluoro-4-methoxyphenyl)-4-[4-(methylsulfonyl)phenyl]-2- (trifluoromethyl)-1H-imidazole

### Step 1 Preparation of 3-fluoro-4-methoxy-α -[(trimethylsilyl)oxy]benzeneacetonitrile

3-Fluoro-*p*-anisaldehyde (15 mmol, 2.31 g) and trimethylsilyl cyanide (16 mmol, 2.13 ml) were added to ZnI₂ (15 mg). The mixture was stirred for 3 hours at room temperature and vacuum distilled (ca. 1 torr, 105°C) affording the title compound as an oil (3.38 g, 89%).

### Step 2 Preparation of 1-(3-fluoro-4-methoxyphenyl)-2-hydroxy-2-[4-(methylthio)phenyl]ethanone

A dry flask under an argon atmosphere containing tetrahydrofuran (25 ml) was cooled to -78°C. Lithium hexamethyldisilazide (1M in tetrahydrofuran, 15 ml) was introduced, followed by 3-fluoro-4-methoxy-α -[(trimethylsilyl)oxy]benzeneacetonitrile from step 1 (13. 4 mmol, 3.38 g), which was transferred using ca. 10 ml of tetrahydrofuran. After 20 minutes, (4-methylthio)benzaldehyde (14.4 mmol, 1.92 ml) was added. The reaction was quenched after an hour by addition of 5% HCl (60 ml) and KHF₂ (22 mmol, 1.75 g). The mixture was stirred for 1.5 hours at room temperature and extracted into ethyl acetate (150 ml). The organic 4-(3-Fluoro-4-methoxyphenyl)-5-[4-(methylthio)phenyl]-2-(trifluoromethyl)-1H-imidazole (516 mg, 1.35 mmol) from step 4 was dissolved in methanol (10 ml) and cooled to 0°C. OXONE® (2.46 g, 4 mmol) in water (10 ml) was added. After the addition, the ice bath was removed and the reaction was stirred at ambient temperature for 3 hours. The mixture was extracted with chloroform (3 x 50 ml). The combined organic phases were dried (MgSO₄) and concentrated *in vacuo*. The residue was purified by chromatography, affording 4-(3-fluoro-4-methoxyphenyl)-5-[4-(methylsulfonyl)phenyl]-2-(trifluoromethyl)-1H-imidazole as a solid (98 mg): m.p. 228-234°C (DSC). The structure assignment was supported by NMR. Mass spectrum (EI, m/e): 414. Anal. Calc'd. for C₁₈H₁₄N₂F₄O₃S: C, 52.17; H, 3.41; N, 6.76. Found: C, 52.30; H, 3.62; N, 6.53.

### Example 11

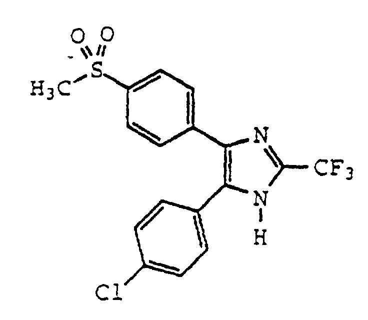

### 5-(4-Chlorophenyl)-4-[4-(methylsulfonyl)phenyl]-2-(trifluoromethyl)-1H-imidazole

### Step 1 Preparation of 4-chloro-α -(trimethylsilyl)oxy]benzeneacetonitrile

The title compound was prepared from 4-chlorobenzaldehyde (2.81 g) by the method of Example phase was separated and stirred for 1 hour in the presence of aqueous NaOH (1.20 g in 50 ml). The organic layer was separated, dried over MgSO₄, concentrated *in vacuo* and subjected to chromatography, affording the title compound (2.00 g, 50%) as a waxy solid.

### Step 3 Preparation of 1-(3-fluoro-4-methoxyphenyl)-2-[4-(methylthio)phenyl]ethane-1,2-dione

1-(3-Fluoro-4-methoxyphenyl)-2-hydroxy-2-[4-(methylthio)phenyl]ethanone from step 2 (6.5 mmol, 2.00 g) was diluted with glacial acetic acid (30 ml). Bismuth oxide (11.4 mmol, 5.30 g) was added and the mixture was heated at 90°C for 2 hours. The crude mixture was filtered through Celite® filter agent, lyophilized and subjected to chromatography, affording the title compound as a yellow solid (642 mg, 32%).

### Step 4 Preparation of 5-(3-fluoro-4-methoxyphenyl)-4-[4-(methylthio)phenyl]-2-(trifluoromethyl)-1H-imidazole

To 1-(3-fluoro-4-methoxyphenyl)-2-[4-(methylthio)phenyl]ethane-1,2-dione from step 3 (2.1 mmol, 638 mg) was added ammonium acetate (12 mmol, 0.925 g), glacial acetic acid (10 ml) and trifluoroacetaldehyde ethyl hemiacetal (5.5 mmol, 0.64 ml). The mixture was brought to reflux. After 6 hours, additional hemiacetal (0.2 ml) was added and the mixture was heated an additional 16 hours. Lyophilization followed by chromatography (1:20 methanol in toluene) afforded the imidazole as a glass (526 mg).

### Step 5 Preparation of 5-(3-fluoro-4-methoxyphenyl)-4-[4-(methylsulfonyl)phenyl]-2-(trifluoromethyl)-1H-imidazole

10, seep 1 (distilled at ca, 1 torr, 98-100°C), affording an oil (4.53 g, 94%).

### Step 2 Preparation of 1-(4-chlorophenyl)-2-hydroxy-2-[4-(methylthio)phenyl]ethanone

The title compound was prepared from 4-chloro-α -[(trimethylsilyl)oxy]benzeneacetonitrile from step 1 (18.9 mmol, 4.53 g) by the method of Example 10, step 2, affording a solid (3.05 g, 55%).

### Step 3 Preparation of 1-(4-chlorophenyl)-2-[4-(methylthio)phenyl]ethane-1,2-dione

The title compound was prepared from 1-(4-chlorophenyl)-2-hydroxy-2-[4-(methylthio)phenyl]ethanone from step 2 (4.5 mmol, 1.31 g) by the method of Example 10, step 3, affording a yellow solid (929 mg, 71%).

### Step 4 Preparation of 5-(4-chlorophenyl)-4-[4-(methylthio)phenyl]-2-(trifluoromethyl)-1H-imidazole

The title compound was prepared from 1-(4-chlorophenyl)-2-[4-(methylthio)phenyl]ethane-1,2-dione of step 3 (3.0 mmol, 870 mg) by the method of Example 10, step 4, affording a foam (516 mg, 47%).

### Step 5. Preparation of 5-(4-chlorophenyl)-4-[4-(methylsulfonyl)phenyl]-2-(trifluoromethyl)-1H-imidazole

The title compound was prepared from 4-[4-(methylthio)phenyl]-5-(4-chlorophenyl)-2-(trifluoromethyl)-1H-imidazole from step 4 (488 mg, 1.3 mmol) by the method of Example 10, seep 5, affording a solid (103 mg). The structure assignment was supported by NMR. Mass spectrum (EI, m/e): 400. Anal. Calc'd. for C₁₇H₁₂N₂ClF₃O₂S•0.5 H₂O: C. 49.82; H, 3.20; N, 6.84. Found: C, 49.52; H, 2.98; N, 6.46.

### Example 12

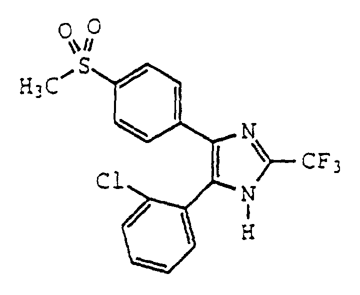

### 5-(2-Chlorophenyl)-4-[4-(methylsulfonyl)phenyl]-2-(trifluoromethyl)-1H-imidazole

### Step 1 Preparation of 2-chloro-α -[(trimethylsilyl)oxy]benzeneacetonitrile

The title compound was prepared from 2-chlorobenzaldehyde (2.81 ml) by the method of Example 10, step 1 (distilled at ca. 1 torr, 92°C), affording an oil (5.61 g, 94%).

### Step 2 Preparation of 1-(2-chlorophenyl)-2-hydroxy-2-[4-(methylthio)phenyl]ethanone

The title compound was prepared from 2-chloro-α -[(trimethylsilyl)oxy]benzeneacetonitrile (23.5 mmol, 5.61 g) from step 1 by the method of Example 10, step 2, affording a solid (1.54 g, 22%).

### Step 3 Preparation of 1-(2-chlorophenyl)-2-hydroxy-2-[4-(methylthio)phenyl]ethanone

The title compound was prepared from 1-(2-chlorophenyl)-2-[4-(methylthio)phenyl]ethane-1,2-dione from step 2 (5.27 mmol, 1.54 g) by the method of Example 10, step 3, affording a yellow solid (374 mg, 24%).

### Step 4 Preparation of 5-(2-chlorophenyl)-4-[4-(methylthio)phenyl]-2-(trifluoromethyl)-1H-imidazole

The title compound was prepared from 1-(2-chlorophenyl)-2-hydroxy-2-[4-(methylthio)phenyl]ethanone from step 3 (1.27 mmol, 370 mg) by the method of Example 10, step 4, affording a white solid (291 mg, 62%). Mass spectrum (EI, m/e): 368. Anal. Calc'd. for C₁₇H₁₂N₂ClF₃S: C, 55.36; H, 3.28; N, 7.60. Found: C, 55.46; H, 3.13; N, 7.28.

### Step 5 Preparation of 5-(2-chlorophenyl)-4-[4-(methylsulfonyl)phenyl]-2-(trifluoromethyl)-1H-imidazole

4-[4-(Methylthio)phenyl]-5-(2-chlorophenyl)-2-(trifluoromethyl)-1H-imidazole from step 4 (285 mg, 0.77 mmol) was dissolved in acetic acid (6 ml). Hydrogen peroxide (30%, 0.23 ml) was added and the solution was heated over a steam bath for 40 minutes. Lyophilization followed by chromatography afforded the title compound as a white foam (133 mg). The-structure assignment was supported by NMR. Mass spectrum (EI, m/e): 400. Anal. Calc'd. for C₁₇H₁₂N₂ClF₃O₂S: C, 50.80; H, 2.92; N, 6.76. Found: C, 50.94; H, 3.02; N, 6.99.

### Example 13

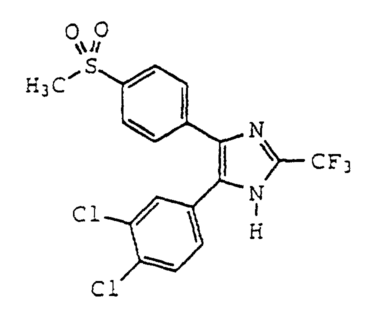

### 5-(3,4-Dichlorophenyl)-4-[4-(methylsulfonyl)phenyl]-2-(trifluoromethyl)-1H-imidazole

### Step 1 Preparation of 3,4-dichloro-α -[(trimethylsilyl)oxy]benzeneacetonitrile

The title compound was prepared from 3,4-dichlorobenzaldehyde (25 mmol, 4.375 g) by the method of Example 10, step 1 (distilled at ca. 1 torr, 115°C), affording the title compound as an oil (6.49 g, 95%).

### Step 2 Preparation of 1-(3,4-dichlorophenyl)-2-hydroxy-2-[4-(methylthio)phenyl]ethanone

A dry flask under an argon atmosphere containing tetrahydrofuran (50 ml) was cooled to -78°C. Lithium hexamethyldisilazide (1M in tetrahydrofuran, 25 ml) was introduced, followed by a solution of 3,4-dichloro-α -[(trimethylsilyl)oxy]benzeneacetonitrile from step 1 (23.7 mmol, 6.49 g) in tetrahydrofuran (10 ml). After 20 minutes, (4-methylthio)benzaldehyde (25 mmol, 3.33 ml) was added. The reaction was quenched after an hour by addition of 10% HCl (100 ml) and KHF₂ (40 mmol, 3.12 g). The mixture was stirred 45 minutes at room temperature, then extracted into ethyl acetate (200 ml). The organic phase was separated, washed with saturated NaCl solution (50 ml), then stirred for 1 hour in 'the presence of aqueous NaOH (1.14 g in 50 ml). The organic layer was separated, dried over MgSO₄, concentrated *in vacuo* and subjected to chromatography on silica gel (2:1 hexane/ethyl acetate), affording the title compound (2.32 g, 30%) as a waxy solid.

### Step 3 Preparation of 1-(3,4-dichlorophenyl)-2-[4-(methylthio)phenyl]ethane-1,2-dione

Dimethyl sulfoxide (16.3 mmol, 1.20 ml) was added to methylene chloride (70 ml) and the solution was cooled to -65°C. Trifluoroacetic acid anhydride (13.1 mmol, 1.84 ml) was added over 2 minutes and the cold solution was stirred an additional 10 minutes. A solution of 1-(3,4-dichlorophenyl)-2-hydroxy-2-[4-(methylthio)phenyl]ethanone from step 2 (7.10 mmol, 2.32 g) in methylene chloride (ca. 10 ml) was introduced and after 30 minutes, triethylamine (32.6 mmol, 4.53 ml) was added. The mixture was warmed to 0°C over 30 minutes, diluted with water (60 ml) and extracted with ethyl acetate (200 ml). The organic phase was separated, dried over magnesium sulfate, filtered through silica gel, and concentrated *in vacuo*. The residue was subjected to chromatography on silica gel using mixtures of hexane and ethyl acetate as eluents, affording the title compound as a yellow solid (2.035 g, 88%).

### Step 4 Preparation of 5-(3,4-dichlorophenyl)-4-[4-(methylthio)phenyl]-2-(trifluoromethyl)-1H-imidazole

The title compound was prepared from 1-(3,4-dichlorophenyl)-2-[4-(methylthio)phenyl]ethane-1,2-dione from step 3 (6.26 mmol, 2.035 g) by the method of Example 10, step 4, affording a viscous oil (1.374 g, 54%).

### Step 5 Preparation of 5-(3,4-dichlorophenyl)-4-[4-(methylsulfonyl)phenyl]-2-(trifluoromethyl)-1H-imidazole

The title compound was prepared from 5-(3,4-dichlorophenyl)-4-[4-(methylthio)phenyl]-2-(trifluoromethyl)-1H-imidazole from step 4 (3.41 mmol, 1.374 g) by the method of Example 12, step 5, affording, after two recrystallizations from ethyl acetate and hexane, a solid (395 mg): m.p. 249-251°C (DSC). The structure assignment was supported by NMR. Anal. Calc'd. for C₁₇H₁₁Cl₂F₃O₂S: C, 46.91; H, 2.55; N, 6.44. Found: C, 47.15; H, 2.93; N, 5.91.

### Example 14

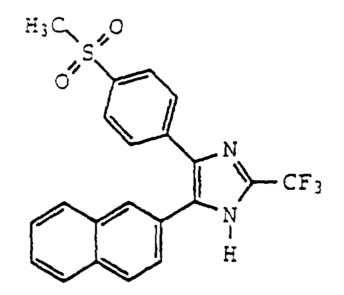

### 4-[4-(Methylsulfonyl)phenyl]-3-(2-naphthyl)-2-(trifluoromethyl)-1H-imidazole

### Step 1 Preparation of α -[(trimethylsilyl)oxy]-2-naphthylacetonitrile

The title compound was prepared from 2-naphthaldehyde (25 mmol, 3.90 g) by the method of Example 10, step 1, (distilled at ca. 1 torr, 110°C), affording an oil (4.896 g, 77%).

### Step 2 Preparation of 2-hydroxy-2-[4-(methylthio)phenyl]-1-(2-naphthyl)-ethanone

The title compound was prepared from α-[(trimethylsilyl)oxy]-2-naphthylacetonitrile from step 1 (19.2 mmol, 4.89 g) by the method of Example 13, step 2, affording a solid (3.28 g, 55%).

### Step 3 Preparation of 2-[4-(methylthio)phenyl]-1-(2-naphthyl)ethane-1,2-dione

The title compound was prepared from 2-hydroxy-2-[4-(methylthio)phenyl]-1-(2-naphthyl)-ethanone from step 2 above (6.50 mmol, 2.00 g) using the method of Example 10, step 3, affording a yellow solid (968 mg, 49%).

### Step 4 Preparation of 4-[4-(methylthio)phenyl]-5-(2-naphthyl)-2-(trifluoromethyl)-1H-imidazole

The title compound was prepared from 2-[4-(methylthio)phenyl]-1-(2-naphchyl)ethane-1,2-dione (3.14 mmol, 960 mg) from step 3 above by the method of Example 10, step 4, affording a white solid (675 mg, 56%).

### Step 5 Preparation of 4-[4-(methylsulfonyl)phenyl]-5-(2-naphthyl)-2-(trifluoromethyl)-1H-imidazole

The title compound was prepared from 4-[4-(methylthio)phenyl]-5-(2-naphthyl)-2-(trifluoromethyl)-1H-imidazole of step 4 above (1.77 mmol, 675 mg) by the method of Example 12, step 5, affording, after recrystallization from acetone and hexane, a solid (393 mg, 54%). The solid was diluted with acetone (ca. 1 ml) and concentrated *in vacuo* to remove traces of hexane: m.p. 228-230°C (DSC). The structure assignment was supported by NMR. Mass spectrum (EI, M/e): 416. Anal. Calc'd. for C₂₁H₁₅F₃N₂O₂S: C, 60.57; H, 3.63; N, 6.73. Found: C, 60.13; H, 3.97; N, 6.34.

### Example 15

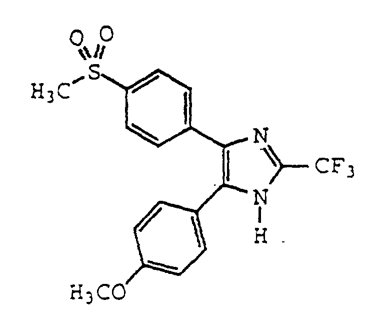

### 5-(4-Methoxyphenyl)-4-[4-(methylsulfonyl)phenyl]-2-(trifluoromethyl)-1H-imidazole

### Step 1 Preparation of 4-methoxy-α -[(trimethylsilyl)oxy]benzeneacetonitrile

The title compound was prepared from *p*-anisaldehyde (25 mmol, 3.03 ml) by the method of Example 10, step 1 (distilled at ca. 1 torr, 102°C), affording an oil (5.74 g, 98%).

### Step 2 Preparation of 2-hydroxy-1-(4-methoxyphenyl)-2-[4-(methylthio)phenyl]ethanone

The title compound was prepared from 4-methoxy-α -[(trimethylsilyl)oxy]benzeneacetonitrile from step 1 above (24.4 mmol, 5.74 g) by the method of Example 13, step 2, affording a solid (5.53 g, 79%).

### Step 3 Preparation of 1-(4-methoxyphenyl)-2-[4-(methylthio)phenyl]ethane-1,2-dione

The title compound was prepared from 2-hydroxy-1-(4-methoxyphenyl)-2-[4-(methylthio)phenyl)ethanone from step 2 (5.64 mmol, 1.63 g) using the method of Example 10, step 3, affording a yellow solid (968 mg, 60%).

### Step 4 Preparation of 5-(4-methoxyphenyl)-4-[4-(methylthio)phenyl]-2-(trifluoromethyl)-1H-imidazole

The title compound was prepared from 1-(4-methoxyphenyl)-2-(4-(methylthio)phenyl]ethane-1,2-dione from step 3 (3.33 mmol, 952 mg) by the method of Example 10, step 4, affording a viscous oil (411 mg, 34%).

### Step 5 Preparation of 5-(4-methoxyphenyl)-4-[4-(methylsulfonyl)phenyl]-2-(trifluoromethyl)-1H-imidazole

The title compound was prepared from 5-(4-methoxyphenyl)-4-[4-(methylthio)phenyl]-2-(trifluoromethyl)-1H-imidazole from step 4 (1.12 mmol, 0.408 g) by the method of Example 12, step 5, affording, after recrystallization from ethyl acetate and hexane, a solid (236 mg, 53%). The solid was diluted with acetone (ca. 1 ml) and concentrated *in* *vacuo* to remove traces of hexane. The structure assignment was supported by NMR. Anal. Calc'd. for C₁₈H₁₅F₃N₂O₃S: C, 54.54; H, 3.81; N, 7.07. Found: C, 54.83; H, 3.83; N, 6.62.

### Example 16

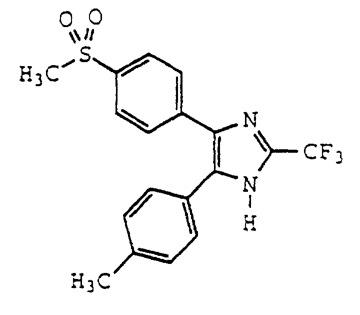

### 5-(4-Methylphenyl)-4-[4-(methylsulfonyl)phenyl]-2-(trifluoromethyl)-1H-imidazole

### Step 1 Preparation of 4-methyl-α -[(trimethylsilyl)oxy]benzeneacetonitrile

The title compound was prepared from p-tolualdehyde (25 mmol, 2.94 ml) by the method of Example 10,step 1 (distilled at ca. 1 torr, 96°C), affording an oil (5.23 g, 96%).

### Step 2 Preparation of 2-hydroxy-1-(4-methylphenyl)-2-[4-(methylthio)phenyl]-ethanone

The title compound was prepared from 4-methyl-α - [(trimethylsilyl)oxy]benzeneacetonitrile from step 1 (23.7 mmol, 5.20 g) by the method of Example 13, step 2, affording a solid (5.53 g, 70%).

### Step 3 Preparation of 1-(4-methylphenyl)-2-[4-(methylthio)phenyl]ethane-1,2-dione

The title compound was prepared from 2-hydroxy-1-(4-methoxyphenyl)-2-[4-(methylthio)phenyl]ethanone from step 2 (5.64 mmol, 1.63 g) using the method of Example 10, step 3, affording a yellow solid (968 mg, 60%).

### Step 4 Preparation of 5-(4-methylphenyl)-4-[4-(methylthio)phenyl]-2-(trifluoromethyl)-1H-imidazole

The title compound was prepared from 1-(4-methylphenyl)-2-[4-(methylthio)phenyl]ethane-1,2-dione from step 3 (3.29 mmol, 889 mg) by the method of Example 10, step 4, affording a viscous oil (345 mg, 30%).

### Step 5 Preparation of 5-(4-methylphenyl)-4-[4-(methylsulfonyl)phenyl]-2-(trifluoromethyl)-1H-imidazole

The title compound was prepared from 5-(4-methylphenyl)-4-[4-(methylthio)phenyl]-2-(trifluoromethyl)-1H-imidazole from step 4 (1.00 mmol, 0.345 mg) by the method of Example 12, step 5, affording, after recrystallization from acetone and hexane, a solid (95 mg, 25%): m.p. 234-238°C (DSC). The structure assignment was supported by NMR. Anal. Calc'd. _for C₁₈H₁₅F₃N₂O₂S: C, 56.84; H, 3.97; N, 7.36. Found: C, 56.68; H, 3.98; N, 7.09.

### Example 17

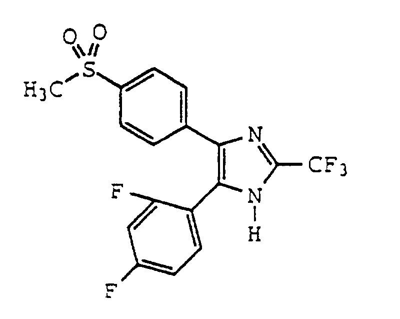

### 5-(2,4-Difluorophenyl)-4-[4-(methylsulfonyl)phenyl]-2-(trifluoromethyl)-1H-imidazole

### Step 1 Preparation of 2,4-difluoro-α - [(trimethylsilyl)oxy]benzeneacetonitrile

The title compound was prepared from 2,4-difluorobenzaldehyde (25 mmol, 2.73 ml) by the method of Example 10, step 1 (distilled at ca. 1 torr, 80°C), affording an oil (5.83 g, 97%)

### Step 2 Preparation of 1-(2,4-difluorophenyl)-2-hydroxy-2-[4-(methylthio)phenyl]-ethanone

The title compound was prepared from 2,4-difluoro-α -[(trimethylsilyl)oxy]benzeneacetonitrile from step 1 (24.2 mmol, 5.85 g) by the method of Example 13, step 2, affording a solid (3.63 g, 51%).

### Step 3 Preparation of 1-(2,4-difluorophenyl)-2-[4-(methylthio)phenyl]ethane-1,2-dione

The title compound was prepared from 1-(2,4-difluorophenyl)-2-hydroxy-2-[4-(methylthio)phenyl]-ethanone from step 2 (12.3 mmol, 3.63 g) using the method of Example 10, step 3, affording a yellow solid (1.57 g, 44%).

### Step 4 Preparation of 5-(2,4-difluorophenyl)-4-[4-(methylthio)phenyl)-2-(trifluoromethyl)-1H-imidazole

The title compound was prepared from 1-(2,4-difluorophenyl)-2-[4-(methylthio)phenyl]ethane-1,2-dione from step 3 (5.38 mmol, 1.57 g) by the method of Example 10, step 4, affording a viscous oil (938 mg, 47%).

### Step 5 Preparation of 5-(2,4-difluorophenyl)-4-[4-(methylsulfonyl)phenyl]-2-(trifluoromethyl)-1H-imidazole

The title compound was prepared from 5-(2,4-difluorophenyl)-4-[4-(methylthio)phenyl]-2-(trifluoromethyl)-1H-imidazole from step 4 (2.53 mmol, 0.938 g) by the method of Example 12, step 5, affording, after recrystallization from ethyl acetate and hexane, a solid (487 mg, 48%). The solid was diluted with acetone (ca. 1 ml) and concentrated *in vacuo* to remove traces of hexane. The structure assignment was supported by NMR. Mass Spectrum (EI, M/e) : 402. Anal. Calc'd. for C₁₇H₁₁F₅O₂S: C, 50.75; H, 2.76; N, 6.96. Found: C, 50.83; H, 2.59; N, 6.79.

### Example 18

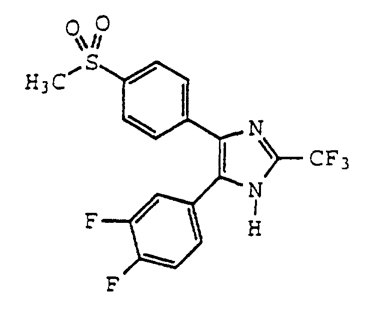

### 5-(3,4-Difluorophenyl)-4-[4-(methylsulfonyl)phenyl]-2-(trifluoromethyl)-1H-imidazole

### Step 1 Preparation of 3,4-difluoro-α -[(trimethylsilyl)oxy]benzeneacetonitrile

The title compound was prepared from 3,4-difluorobenzaldehyde (25 mmol, 2.73 ml) by the method of Example 10, step 1 (distilled at ca. 1 torr, 80°C), affording an oil (6.02 g, 100%).

### Step 2 Preparation of 1-(3,4-difluorophenyl)-2-hydroxy-2-[4-(methylthio)phenyl]-ethanone

The title compound was prepared from 3,4-difluoro-α -[(trimethylsilyl)oxy]benzeneacetonitrile from step 1 above (25 mmol, 6.02 g) by the method of Example 13, step 2, affording a solid (1.83 g, 25%).

### Step 3 Preparation of 1-(3,4-difluorophenyl)-2-[4-(methylthio)phenyl]ethane-1,2-dione

The title compound was prepared from 1-(3,4-difluorophenyl)-2-hydroxy-2-(4-(methylthio)phenyl]-ethanone from step 2 (6.23 mmol, 1.83 g) using the method of Example 10, step 3, affording a yellow solid (985 g, 54%).

### Step 4 Preparation of 5-(3,4-difluorophenyl)-4-[4-(methylthio)phenyl]-2-(trifluoromethyl)-1H-imidazole

The title compound was prepared from 1-(3,4-difluorophenyl)-2-[4-(methylthio)phenyl]ethane-1,2-dione from step 3 (3.37 mmol, 985 mg) by the method of Example 10, step 4, affording a viscous oil (606 mg, 49%).

### Step 5 Preparation of 5-(3,4-difluorophenyl)-4-[4-(methylsulfonyl)phenyl]-2-(trifluoromethyl)-1H-imidazole

The title compound was prepared from 5-(3,4-difluorophenyl)-4-[4-(methylthio)phenyl]-2-(trifluoremethyl)-lH-imidazole from step 4 (1.64 mmol, 0.606 g) by the method of Example 12, step 5, affording, after recrystallization from acetone, a solid (313 mg, 47%). The solid was diluted with acetone (ca. 1 ml) and concentrated *in vacuo* to remove traces of hexane: m.p. 249-252°C (DSC). The structure assignment was supported by NMR. Anal. Calc'd. for C₁₇H₁₁F₅O₂S: C, 50.75; H, 2.76; N, 6.96. Found: C, 50.58; H, 2.96; N, 6.66.

### Example 19

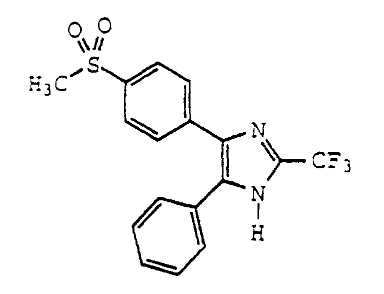

### 4-[4-(Methylsulfonyl)phenyl]-5-phenyl-2-(trifluoromethyl)-1H-imidazole

### Step 1 Preparation of α -(trimethylsilyl)oxy]benzeneacetonitrile

The title compound was prepared from benzaldehyde (25 mmol, 2.55 ml) by the method of Example 10, step 1 (distilled at ca. 1 torr, 86°C), affording an oil (4.52 g, 88%).

### Step 2 Preparation of 2-hydroxy-2-[4-(methylthio)phenyl]-1-phenyl-ethanone

The title compound was prepared from a-(trimethylsilyl)oxy]benzeneacetonitrile from step 1 (22 mmol, 4.52 g) by the method of Example 13, step 2, affording a solid (3.25 g, 57%).

### Step 3 Preparation of 2-(4-(methylthio)phenyl]-1-(phenyl)ethane-1,2-dione

The title compound was prepared from 2-hydroxy-2-[4-(methylthio)phenyl]-1-phenyl-ethanone from step 2 (12.6 mmol, 3.25 g) using the method of Example 10, step 3, affording a yellow solid (1.50 g, 46%).

### Step 4 Preparation of 4-[4-(methylthio)phenyl]-5-phenyl-2-(trifluoromethyl)-1H-imidazole

The title compound was prepared from 2-[4-(methylthio)phenyl]-1-(phenyl)ethane-1,2-dione from step 3 (5.87 mmol, 1.50 g) by the method of Example 10, step 4, affording a viscous oil (918 mg, 47%).

### Step 5 Preparation of 4-[4-(methylsulfonyl)phenyl]-5-phenyl-2-(trifluoromethyl)-1H-imidazole

The title compound was prepared from 4-[4-(methylthio)phenyl]-5-phenyl-2-(trifluoromethyl)-1H-imidazole from step 4 (2.75 mmol, 0.918 g) by the method of Example 12, step 5, affording, after recrystallization from acetone, a solid (489 mg, 45%). The solid was diluted with acetone (ca. 1 ml) and concentrated *in vacuo* to remove traces of hexane: m.p. 228-230°C (DSC). The structure assignment was supported by NMR. Mass spectrum (EI, M/e): 366. Anal. Calc'd. for C₁₇H₁₂F₃O₂S: C, 55.73; H, 3.58; N, 7.65. Found: C, 55.58; H, 3.66; N, 7.31.

### Example 20

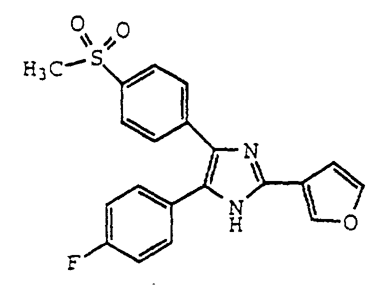

### 5-(4-Fluorophenyl)-2-(3-furyl)-4-(4-methylsulfonylphenyl)-1H-imidazole

In a manner similar to Example 1, Method A, 1-(4-methylsulfonylphenyl)-2-(4-fluorophenyl)-ethane-1,2-dione (Example 1, Method A, step 4) (1 g, 3.3 mmol), ammonium acetate (2 g, 25.9 mmol), 3-furaldehyde (350 mg, 3.64 mmol) and acetic acid (20 ml) gave 500 mg (47%) product after recrystallization from ethanol and water. m.p. 233-235°C (dec.). Anal. Calc'd. for C₂₀H₁₅N₂O₃FS (M.W. 382.42) : C, 62.82; H, 3.95; N, 7.33; S, 8.38. Found: C, 62.50; H, 3.90; N, 7.04; S, 8.25.

### Example 21

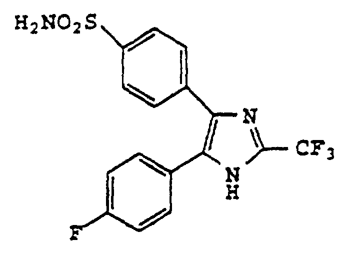

### 4-[5-(4-Fluorophenyl)-2-(trifluoromethyl)-1H-imidazol-4-yl]benzenesulfonamide

### Step 1 Preparation of 5-(4-fluorophenyl)-4-(4-methylsulfonylphenyl)-2-trifluoromethyl-1-(2-trimethylsilylethoxymethyl)-1H-imidazole

A mixture of hexane-washed sodium hydride (NaH) (110 mg of 60%, 2.8 mmol) in dimethylformamide (DMF) (15 ml) was stirred under a nitrogen atmosphere at 25°C and a solution of 5-(4-fluorophenyl)-4-(4-methylsulfonylphenyl)-2-trifluoromethyl-1H-imidazole (Example 11 (970 mg, 2.5 mmol) in DMF (5 ml) was added over 15 minutes. This mixture was stirred an additional 30 minutes. Trimethylsilylethoxymethyl chloride (450 mg, 3 mmol) was added and the reaction mixture was stirred for 16 hours at 25°C. The reaction mixture was poured into 2% aqueous NH₄Cl (150 ml) and extracted with ethyl acetate. After drying (Na₂SO₄) and solvent removal, the protected sulfone imidazole (1.1 g, 95%) was essentially pure and was used without further purification.

### Step 2 Preparation of 4-[5-(4-fluorophenyl)-2-trifluoromethyl-1-(2-trimethylsilylethoxymethyl)-1H-imidazol-4-yl]benzenesulfonamide

To a solution of the protected sulfone imidazole from step 1 (1.1 g, 2.0 mmol) in THF (15 ml) at 0°C under an argon atmosphere, n-butyl magnesium bromide (n-BuMgBr) (2M solution in THF, 4.5 ml) was added and the temperature warmed to 25°C over 30 minutes. Triethylborane (Et₃B) (1M solution in THF, 9 ml) was added and the reaction was heated to reflux for 48 hours. The reaction mixture was cooled to 25°C and a mixture of hydroxylamine-O-sulfonic acid (NH₂OSO₃H) (2 g) and sodium acetate (2 g) in water (8 ml) was added and rapidly stirred at 25°C for 16 hours. Water (50 ml) was added and the product was extracted with ethyl acetate, dried (Na₂SO₄), and evaporated. The residue was purified by chromatography on silica gel using mixtures of ethyl acetate and toluene as the eluent to give 500 mg (45%) of the desired protected sulfonamide. The product was used directly in the next step.

### Step 3: Preparation of 4-[5-(4-fluorophenyl)-2-trifluoromethyl-1H-imidazol-4-yl]benzenesulfonamide

A solution of protected sulfonamide from step 2 (500 mg, 1 mmol) in a solution of 3 N HCl (5 ml) in EtOH (4 ml) was heated to reflux for 1 hour, cooled and diluted with water (20 ml). The resulting mixture was extracted with methylene chloride, dried (Na₂SO₄) and the solvent was evaporated. The residue was recrystallized from toluene containing a small amount of ethyl acetate to give the title product (150 mg, 40%): m.p. 259-260°C. Anal. Calc'd. for C₁₆H₁₁N₃O₂F₄S : C, 49.87; H, 2.88; N, 10.90; S, 8.32. Found: C, 50.30; H, 3.10; N, 10.55; S, 8.73.

The following imidazole derivatives could be prepared by the procedure described in Example 21:
Example 22 4-[5-(4-fluorophenyl)-2-phenoxymethyl-1H-imidazol-4-yl]benzenesulfonamide;
Example 23 4-[5-(4-fluorophenyl)-2-(2-phenyl-*trans*-eth-1-ene)-1H-imidazol-4-yl]benzenesulfonamide;
Example 24 4-[2-(2-benzofuryl)-5-(4-fluorophenyl)-1H-imidazol-4-yl]benzenesulfonamide;
Example 25 4-[5-(4-fluorophenyl)-2-isopropyl-1H-imidazol-4-yl]benzenesulfonamide;
Example 26 4-[5-(4-fluorophenyl)-2-methyl-1H-imidazol-4-yl]benzenesulfonamide;
Example 27 4-[5-(4-fluorophenyl)-2-(2-furyl)-1H-imidazol-4-yl]benzenesulfonamide;
Example 28 4-[2-benzylthio-5-(4-fluorophenyl)-1H-imidazol-4-yl]benzenesulfonamide;
Example 29 4-[5-(4-fluorophenyl)-2-hydroxymethyl-1H-imidazol-4-yl]benzenesulfonamide;
Example 30 4-[5-(3-fluoro-4-methoxyphenyl)-2-(trifluoromethyl)-1H-imidazol-4-yl]benzenesulfonamide;
Example 31 4-[5-(4-chlorophenyl)-2-(trifluoromethyl)-1H-imidazol-4-yl)benzenesulfonamide;
Example 32 4-[5-(2-chlorophenyl)-2-(trifluoromethyl)-1H-imidazol-4-yl]benzenesulfonamide;
Example 33 4-[5-(3,4-dichlorophenyl)-2-(trifluoromethyl)-1H-imidazol-4-yl]benzenesulfonamide;
Example 34 4-[5-(2-naphthyl)-2-(trifluoromethyl)-1H-imidazol-4-yl]benzenesulfonamide;
Example 35 4-[5-(4-methoxyphenyl)-2-(trifluoromethyl)-1H-imidazol-4-yl]benzenesulfonamide;
Example 36 4-[S-(4-methylphenyl)-2-(trifluoromethyl)-1H-imidazol-4-yl]benzenesulfonamide;
Example 37 4-[5-(2,4-difluorophenyl)-2-(trifluoromethyl)-1H-imidazol-4-yl]benzenesulfonamide;
Example 38 4-[5-(3,4-difluorophenyl)-2-(trifluoromethyl)-1H-imidazol-4-yl]benzenesulfonamide;
Example 39 4-[5-phenyl-2-(trifluoromethyl)-1H-imidazol-4-yl]benzenesulfonamide; and
Example 40 4-[5-(4-fluorophenyl)-2-(3-furyl)-1H-imidazol-4-yl]benzenesulfonamide.

### Example 41

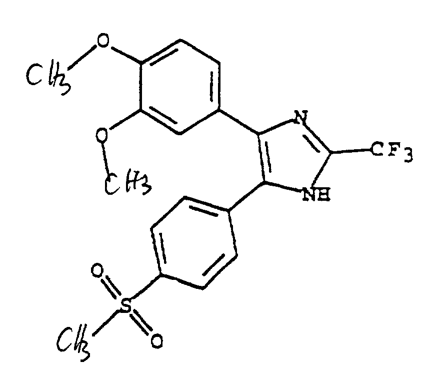

### 4-(3,4-Dimethoxyphenyl)-5-[4-(methylsulfonyl)phenyl]-2-(trifluoromethyl)-1H-imidazole

### Step 1: Preparation of 4-methylthiobenzaldehydetrimethylsilylcyanohydrin

4-Methylthiobenzaldehyde (38.5 g, 0.25 mol) was added dropwise over 30 minutes to a stirred mixture of trimethylsilylcyanide (25 g, 0.25 mol) and zinc iodide (150 mg) at 25°C under a nitrogen atmosphere. After stirring overnight, the reaction mixture was distilled to give 25 g (51%) of desired liquid: bp 120-125°C (0.1 mm Hg). Anal. Calc'd. for C₁₂H₁₇NOSiS (MW 251.43) : C, 57.33; H, 6.82; N, 5.57; S, 12.75. Found: C, 57.11; H, 6.71; N, 5.49; S, 12.99.

### Step 2: Preparation of 4-methylthiophenyl-3',4'-dimethoxyphenyl benzoin

To a cold (-70°C) stirred solution of lithium bis(trimethylsilyl)amide (23 ml of a 1M solution in tetrahydrofuran, 23 mmol) was added a solution of 4-methylthiobenzaldehyde-trimethylsilylcyanohydrin (Step 1) (5 g, 20 mmol) in tetrahydrofuran (25 ml) dropwise over 15 minutes. After stirring for an additional 5 minutes, a solution of 3,4-dimethoxybenzaldehyde (3.3 g, 20 mmol) in tetrahydrofuran (20 ml) was added dropwise over 5 minutes. The reaction was stirred for 30 minutes at -70°C and then warmed to room temperature. After stirring for 3 hours, the reaction was quenched by adding 3N sulfuric acid (50 ml) and warming at 40°C for 3 hours. After cooling, the reaction mixture was partitioned between ethyl ether and water and the layers separated. The organic layer was stirred with 1N sodium hydroxide (100 ml) for 1 hour and the layers were separated. The organic layer was washed with brine and dried over sodium sulfate. The drying agent was filtered and the filtrate concentrated *in vacuo* to give the crude product as an oil. Chromatography of the oil on silica gel using 30% ethyl acetate/hexane gave 2.3 g (35%) of the desired benzoin as an oil that crystallized on standing: mp 88-91°C. Anal. Calc'd. for C₁₇H₁₈O₄S (MW 318.40) : C, 64.13; H, 5.70; S, 10.07. Found: C, 63.92; H, 5.51; S, 10.31.

### Step 3: Preparation of 4-methylthiophenyl-3',4'-dimethoxyphenyl benzil

A mixture of the benzoin from Step 2 (2.2g, 6.9 g mmol) and bismuth oxide (4 g, 8.6 mmol) in acetic acid (25 mL) was heated at 90°C for 45 minutes. The hot reaction mixture was filtered and the filtrate concentrated in vacuo to give 2.0 g (90%) of a solid suitable for use without further purification: mp 109-110°C. Anal. Calc'd. for C₁₇H₁₆O₄S (MW 316.40): C, 64.54; H, 5.10; S, 10.13. Found: C, 64.59; H, 5.02; S, 10.29.

### Step 4: Preparation of 4-methylsulfonylphenyl-3',4'-dimethoxyphenyl benzil

A solution of Oxone® (9.8 g, 16 mmol) in water (50 ml) was added dropwise over 15 minutes to a solution of the benzil from Step 3 (2.0 g, 63 mmol) in a methanol:tetrahydrofuran solution (2:1, 100 ml). The reaction mixture was warmed to 40°C for 2 hours, cooled and poured into water (500ml). The solid precipitate was filtered and air dried to give 2.0 g (95%) of desired product as a solid: mp 181-183°C. Anal. Calc'd. for C₁₇H₁₆O₆S (MW 348.38): C, 58.61; H, 4.63; S, 9.20. Found: C, 58.55; H, 4.49; S, 9.29.

### Step 5: Preparation of 4-(3,4-dimethoxyphenyl)-5-[4-(methylsulfonyl)phenyl]-2-(trifluoromethyl)-1H-imidazole

A mixture of 4-methylsulfonylphenyl-3',4'-dimethoxyphenyl benzil (Step 4) (2 g, 5.7 mmol), ammonium acetate (2.65 g, 34 mmol) and trifluoroacetaldehyde ethyl hemiacetal (1.90 g) in acetic acid was heated at reflux with stirring overnight under a nitrogen atmosphere. The reaction was cooled, poured into water and the resulting precipitate filtered and air dried. The crude product was purified by chromatography on silica gel using 2% methanol in methylene chloride as the eluent to give 1.1 g (51%) of the title product: mp 221-224°C. Anal. Calc'd. for C₁₉H₁₇N₂F₃O₄S (MW 426.41): C, 53.52; H, 4.02; N, 6.57; S, 7.52. Found: C, 53.35; H, 4.07; N, 6.51; S, 7.85.

### Example 42

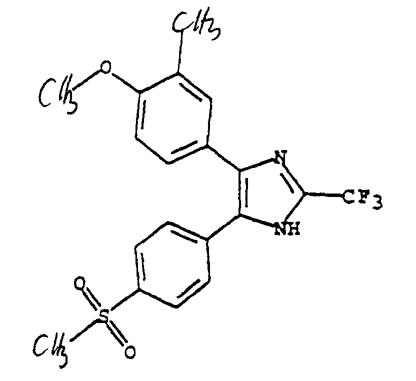

### 4-(4-Methoxy-3-methylphenyl)-5-[4-(methylsulfonyl)phenyl]-2-(trifluoromethyl)-1H-imidazole

### Step 1: Preparation of 4-methylthiophenyl-3'-methyl-4'-methoxyphenyl benzoin

The benzoin was synthesized according to the procedure of Example 41, step 2 using the product of Example 41, Step 1 (5 g, 20 mmol), 3-methyl-4-methoxybenzaldehyde (3 g, 20 mmol) and 1M lithium bis(trimethylsilyl)amide in tetrahydrofuran (23 ml, 23 mmol). The crude product was purified by chromatography on silica gel using 30% ethyl acetate in hexane to give 4.1 g (68%) of the compound as a crystalline solid: mp 101-103°C. Anal. Calc'd. for C₁₇H₁₈O₃S (MW 302.40): C, 67.52; H, 6.00; S, 10.60. Found: C, 67.33; H, 5.89; S, 10.75.

### Step 2: Preparation of 4-methylthiophenyl-3'-methyl-4'-methoxyphenyl benzij

The benzil was synthesized according to the procedure of Example 41, Step 3 using the benzoin of Step 1 (4.1 g, 13.5 mmol) and bismuth oxide (7.9 g, 16.9 mmol) in acetic acid. There was obtained 4 g (95%) of the desired compound as a crystalline solid: mp 134-136°C. Anal. Calc'd. for C₁₇H₁₆O₃S (MW 300.39): C, 67.98; H, 5.37; S, 10.67. Found: C, 68.00; H, 5.20; S, 10.79.

### Step 3: Preparation of 2-trifluoromethyl-4-(4-methylthiophenyl)-5-(3-methyl-4-methoxyphenyl)-1H-imidazole

The imidazole was synthesized according to the procedure of Example 41, Step 5 using the product of Step 2 (1.6 g, 5.3 mmol), ammonium acetate (2.8 g, 37 mmol) and trifluoroacetaldehyde ethyl hemiacetal (1.9 g, 10.6 mmol) in acetic acid (30 ml). The crude product was purified by chromatography on silica gel using 3% methanol in toluene as the eluent give 1 g (55%) of the desired product as a crystalline solid: mp 188-190°C. Anal. Calc'd. for C₁₉H₁₇N₂F₃OS (MW 378.42): C, 60.31; H, 4.53; N, 7.40; S, 8.47. Found: C, 60.11; H, 4.39; N, 7.21; S, 8.63.

### Step 4: Preparation of 4-(4-methoxy-3-methylphenyl)-5-[4-(methylsulfonyl)phenyl]-2-(trifluoromethyl)-1H-imidazole

The title compound was synthesized according to the procedure of Example 41, Step 4 using the product of Step 3 (1 g, 2.6 mmol) and Oxone® (4 g, 6.6 mmol) in methanol (20 ml), tetrahydrofuran (10 ml) and water (5 ml). The crude product was purified by chromatography on silica gel using 50% ethyl acetate in toluene as the eluent to give 610 mg (75%) of the desired product as a crystalline solid: mp 265-267°C. Anal. Calc'd. for C₁₉H₁₇N₂F₃O₃S (MW 410.41): C, 55.60; H, 4.18; N, 6.83; S, 7.81. Found: C, 55.69; H, 4.28; N, 6.70; S, 8.07.

### Example 43

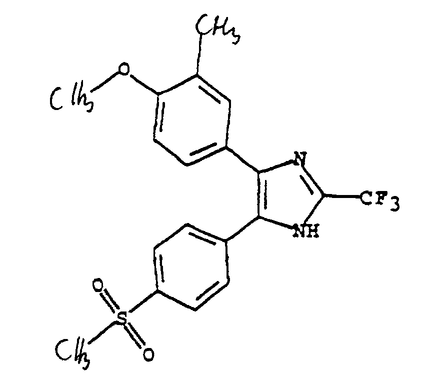

### 4-(4-Methoxy-3-methylphenyl)-5-[4-(methylsulfonyl)phenyl]-2-(trifluoromethyl)-1H-imidazole

### Step 1: Preparation of 4-methylsulfonylphenyl-3'-methyl-4'-methoxyphenyl benzil

The sulfone was synthesized according to the procedure of Example 41, Step 4 using the benzil of Example 42, Step 2 (530 mg, 1.7 mmol) and Oxone® (2.4 g, 4 mmol) in methanol (15 ml), tetrahydrofuran (10 ml) and water (5 ml). There was isolated 545 mg (96%) of the desired sulfone as a crystalline solid: mp 149-151°C. Anal. Calc'd, for C₁₇H₁₆O₅S (MW 332.39): C, 61.43; H, 4.85; S, 9.65. Found: C, 61.40; H, 4.80; S, 9.70.

### Step 2: Preparation of 4-(4-methoxy-3-methylphenyl)-5-[4-(methylsulfonyl)phenyl]-2-(trifluoromethyl)-1H-imidazole

The imidazole was synthesized according to the procedure of Example 41, Step 5 using the benzil of Step 1 (540 mg, 1.6 mmol), ammonium acetate (620 mg, 8 mmol) and trifluoroacetaldehyde ethyl hemiacetal (500 mg, 3.2 mmoi) in acetic acid (10 ml). There was obtained 530 mg (80%) of the desired product.

### Example 44

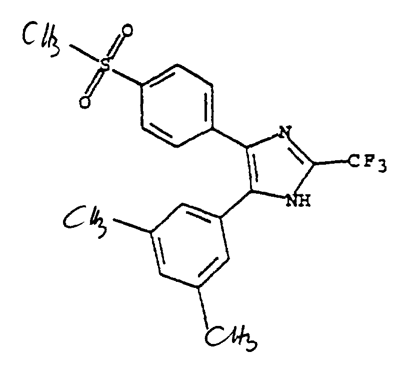

### 5-(3,5-Dimethylphenyl)-4-[4-(methylsulfonyl)phenyl]-2-(trifluoromethyl)-1H-imidazole

### Step 1: Preparation of 3,5-dimethylbenzaldehyde

To a cold (-70°C), stirred solution of oxalyl chloride (5.12 g, 40 mmol) and dimethylsulfoxide (6.3 g, 80 mmol) in methylene chloride (50 ml) was added a solution of 3,5-dimethylbenzyl alcohol (5 g, 36.7 mmol) in methylene chloride (15 ml) over 10 minutes. After stirring for an additional 30 minutes in the cold, triethylamine (8.3 g, 82 mmol) was added and the mixture was warmed to room temperature over 1 hour. The reaction mixture was poured into water and extracted with ethyl ether. The combined organic layers were dried over sodium sulfate, filtered and concentrated to give 4.5 g (88%) of aldehyde suitable for use in the next step without further purification.

### Step 2: Preparation of 4-methylthiophenyl-3',5'-dimethylphenyl benzoin

The benzoin was synthesized according to the procedure of Example 41, Step 2 using the product of Example 41, Step 1 (5 g, 20 mmol), 3, dimethylbenzaldehyde from Step 1 (2.7 g, 20 mmol) and 1M lithium bis(trimethylsilyl)amide in tetrahydrofuran (23 ml, 23 mmol). The crude product was purified by chromatography on silica gel using 20% ethyl acetate in hexane as the eluent to give 2.9 g (50%) of the compound as a crystalline solid: mp 81-83°C. Anal. Calc'd. for C₁₇H₁₈O₂S (MW 286.4-0): C, 71.30; H, 6.34; S, 11.20. Found: C, 71.15; H, 6.21; S, 11.40.

### Step 3: Preparation of 4-methylthiophenyl-3',5'-dimethylphenyl benzil

The benzil compound was synthesized according to the procedure of Example 41, Step 3 using the product of Step 2 (2.88 g, 10 mmol) and bismuth oxide (6 g, 12.5 mmol) in acetic acid (30 ml). There was obtained 2.4 g (85%) of the desired benzil as a crystalline solid: mp 100-102°C. Anal. Calc'd. for C₁₇H₁₆O₂S (MW 300.39) : C, 71.80; H, 5.67; S, 11.28. Found: C, 72.00; H, 5.49; S, 11.35.

### Step 4: Preparation of 4-methylsulfonylphenyl-3',5'-dimethylphenyl benzil

The sulfone was synthesized according to the procedure of Example 41, Step 4 using the product of Step 3 (2.4 g, 8.4 mmol) and Oxone® (13.5 g, 20 mmol) in methanol (100 ml), tetrahydrofuran (50 ml) and water (50 ml). There was isolated 2.2 g (83%) of the desired sulfone as a crystalline solid: mp 141-142°C. Anal. Calc'd. for C₁₇H₁₆O₄S (MW 316.40): C, 64.54; H, 5.10; S, 10.13. Found: C, 64.61; H, 5.03; S, 10.21.

### Step 5: Preparation of 5-(3,5-dimethylphenyl)-4-[4-(methylsulfonyl)phenyl]-2-(trifluoromethyl)-1H-imidazole

The imidazole was synthesized according to the procedure of Example 41, Step 5 using the product of Step 4 (2.2 g, 6.9 mmol), ammonium acetate (2.75 g, 35 mmol) and trifluoroacetaldehyde ethyl hemiacetal (2.1 g, 14 mmol) in acetic acid (40 ml). There was obtained 1.1g (36%) of 5-(3,5-dimethylphenyl)-4-[4-(methylsulfonyl)phenyl)-2-(trifluoromethyl)-1H-imidazole after recrystallization from ethanol: mp 255-257°C. Anal. Calc'd. for C₁₉H₁₇N₂O₂F₃S (MW 394.42): C, 57.86; H, 4.34; N, 7.10; S, 8.13. Found: C, 57.46; H, 4.18; N, 7.00; S, 8.42.

### Example 45

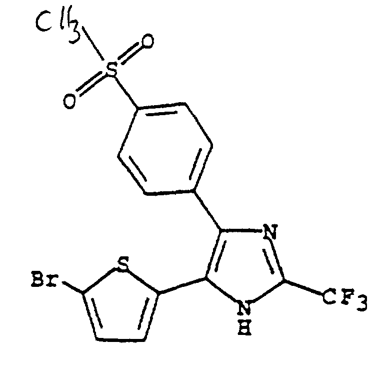

### 5-(5-Bromothien-2-yl)-4-[4-(methylsulfonyl)phenyl]-2-(trifluoromethyl)-1H-imidazole

### Step 1: Preparation of 4-methylthiophenyl-(5-bromo-2-thiophene) benzoin

The benzoin was synthesized according to the procedure of Example 41, Step 2 using the cyanohydrin of Example Step 1 (5 g, 20 mmol), 5-bromothiophene-2-carboxaldehyde (3.82 g, 20 mmol) and 1M lithium bis(trimethylsilyl)amide in tetrahydrofuran (23 ml, 23 mmol). The crude benzoin was purified by chromatography on silica gel using 20% ethyl acetate in hexane as the eluent to give 970 mg (14%) of the desired compound as a crystalline solid: mp 115-117°C. Anal. Calc'd. for C₁₃H₁₁BrO₂S₂ (MW 343.26) C, 45.49; H, 3.23; S, 18.68; Br, 23.28. Found: C, 45.21; H, 3.09; S, 18.82; Br, 23.00.

### Step 2: Preparation of 4-methylthiophenyl-(5-bromo-2-thiophene) benzil

The benzil was synthesized according to the procedure of Example 41, Step 3 using the product of Step 1 (950 mg, 2.7 mmol) and bismuth oxide (1.5 g, 3.2 mmol) in acetic acid (15 ml). There was obtained 680 mg (74%) of the desired benzil after recrystallization from methanol: mp 181-183°C. Anal. Calc'd. for C₁₃H₉BrO₂S₂ (MW 341.25) : C, 45.76; H, 2.66; S, 18.79; Br, 23.42. Found: C, 45.46; H, 2.49; S, 18.99; Br, 23.11.

### Step 3 : Preparation of 4-methylsulfonylphenyl-(5-bromo-2-thiophene)benzil

The sulfone was synthesized according co the procedure of Example 41, Step 4 using the produce of Step 2 (675 mg, 2 mmol) and Oxone® (3g, 5 mmol) in methanol (25 ml), tetrahydrofuran (15 ml) and water (15 ml). There was isolated 553 mg (75%) of the desired sulfone as a crystalline solid: mp 198-200°C. Anal. Calc'd. for C₁₃H₉BrO₄S₂ (MW 373.25): C, 41.83; H, 2.43; S, 17.18; Br, 21.41. Found: C, 41.68; H, 2.32; S, 17.40; Br, 21.20.

### Step 4: Preparation of 5-(5-bromothien-2-yl)-4-[4-(methylsulfonyl)phenyl]-2-(trifluoromethyl)-1H-imidazole

The title compound was synthesized according- to the procedure of Example 41, Step 5 using the product of Step 3 (550 mg, 1.5 mmol), ammonium acetate (570 mg, 7 mmol) and trifluoroacetaldehyde ethyl hemiacetal (500 mg, 3 mmol) in acetic acid (12 ml). The crude product was purified by chromatography on silica gel using 20% ethyl acetate in toluene as the eluent and recrystallization from ethyl acetate and hexane to give 190 mg (48%) of the desired product as a crystalline solid: mp 261-262°C. Anal. Calc'd. for C₁₅H₁₀F₃BrN₂O₂S₂ (MW 451.29): C, 39.92; H, 2.23; N, 6.21; S, 14.21. Found: C, 40.29; H, 2.25; N, 5.88; S, 14.35.

### Example 46

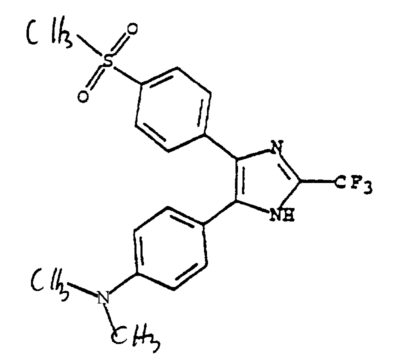

### N,N-Dimethyl-4-[4-[4-(methylsulfonyl)phenyl]-2-(trifluoromethyl)-1H-imidazol-5-yl]benzenamine

### Step 1: Preparation of 4-methylthiophenyl-4'-dimethylaminophenyl benzoin

The benzoin was synthesized according to the procedure of Example 41, Step 2 using the product of Example 41, Step 1 (5 g, 20 mmol), 4-dimethylaminobenzaldehyde (3 g, 20 mmol) and 1M lithium bis(trimethylsilyl)amide in tetrahydrofuran (23 ml, 23 mmol). The crude product was triturated with 10% ethyl acetate in hexane and filtered to give 1.4 g (21%) -of the benzoin as a crystalline solid: mp 92-94°C. Anal. Calc'd. for C₁₇H₁₉NO₂S (MW 301.41) : C, 67.74; H, 6.35; N, 4.65; S, 10.64. Found: C, 67.59; H, 6.29; N, 4.38; S, 10.75.

### Step 2: Preparation of 4-methythiophenyl-4'-dimethylaminophenyl benzil

The benzil was synthesized according to the procedure of Example 41, Step 3 using the product of Step 1 (1.4 g, 4.6 mmol) and bismuth oxide (2.25 g, 4.9 mmol) in acetic acid (25 ml). The crude product was purified by chromatography on silica gel using 5% ethyl acetate in toluene as the eluent to give 940 mg (68%) of the desired benzil as a crystalline solid: mp 109-111°C. Anal. Calc'd. for C₁₇H₁₇NO₂S (MW 299.40) : C, 68.20; H, 5.72; N, 4.68; S, 10.71. Found: C, 68.10; H, 5.70; N, 4.51; S, 10.69.

### Step 3 : Preparation of 4-methylsulfonylphenyl]-4'-dimethylaminophenyl benzil

The sulfone was synthesized according to the procedure of Example 41, Step 4 using the product of Step 2 (930 mg, 3.1 mmol) and Oxone® (4.3 g, 7 mmol) in methanol (40 ml), tetrahydrofuran (15 ml) and water (15 ml). There was isolated 340 mg (33%) of the desired sulfone as a crystalline solid: mp 126-129°C. Anal. Calc'd. for C₁₇H₁₇NO₄S (MW 331.40): C, 61.62; H, 5.17; N, 4.23; S, 9.68. Found: C, 61.40; H, 5.07; N, 4.12; S, 9.89.

### Step 4: Preparation of N,N-dimethyl-4-[4-[4-(methylsulfonyl)phenyl]-2-(trifluoromethyl)-1H-imidazol-5-yl]benzenamine

The title compound was synthesized according to the procedure of Example 41, Step 5 using the product of Step 3 (331 mg, 1 mmol), ammonium acetate (500 mg, 6 mmol) and trifluoroacetaldehyde ethyl hemiacetal (320 mg, 2 mmol) in acetic acid (6 ml). The crude product was purified by chromatography on silica gel using 20% ethyl acetate in toluene as the eluent and recrystallization from ethyl acetate and hexane to give 65 mg (16%) of the desired product as a crystalline solid: mp 248-251°C. Anal. Calc'd. for C₁₉H₁₈N₃O₂F₃S (MW 409.43): C, 55.74; H, 4.43; N, 10.26; S, 7.83. Found: C, 55.28; H, 4.33; N, 9.95; S, 7.49.

### Example 47

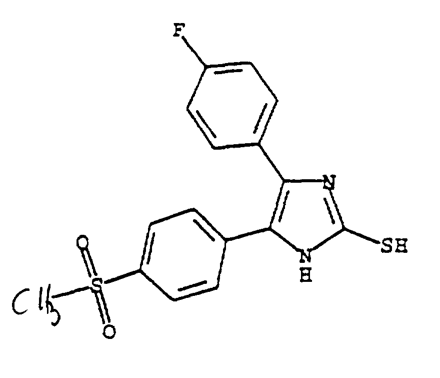

### 4-(4-Fluorophenyl)-5-[4-(methylsulfonyl) phenyl]-1H-imidazole-2-thiol

### Step 1: Preparation of 4-methylthiophenyl-4'-fluorophenyl benzoin

The benzoin was synthesized according to the procedure of Example 41, Step 2 using 4-fluorobenzaldehyde trimethylsilylcyanohydrin (5 g, 21 mmol), 4-methylthiobenzaldehyde (3.62 g, 21 mmol) and 1M lithium bis (trimethylsilyl)amide in tetrahydrofuran (24 ml, 24 mmol). The crude product was purified by chromatography on silica gel using 20% ethyl acetate in hexane to give 3.8 g (67%) of the title compound as a crystalline solid: mp 95-97°C. Anal. Calc'd. for C₁₅H₁₃O₂FS (MW 276.33): C, 65.20; H, 4.74; S, 11.60. Found: C, 65.09; H, 4.59; S, 11.65.

### Step 2: Preparation of 4-methylsulfonylphenyl-4'-fluorophenyl benzoin

The sulfone was synthesized according to the procedure of Example 41, Step 4 using the product of Step 1 (1.8 g, 6.5 mmol) and Oxone® (10 g, 15 mmol) in methanol (125 ml), tetrahydrofuran (50 ml) and water (50 ml). Because of its water solubility, methylene chloride was used to extract the product from the aqueous reaction mixture. There was isolated 2.25 g (98%) of the desired benzoin as a crystalline solid: mp 106-107°C. Anal. Calc'd. for C₁₅H₁₃FO₄S (MW 308.33): C, 58.43; H, 4.25; S, 10.40. Found: C, 58.22; H, 4.12; S, 10.70.

### Step 3 : Preparation of 4-[4-fluorophenyl)-5-[4-(methylsulfonyl)phenyl]-1H-imidazole-2-thiol

A solution of the sulfone from Step 2 (2.25g, 7.3 mmol) and thiourea (1.05 g, 14.6 mmol) in dimethylformamide (30 ml) was heated at reflux under a nitrogen atmosphere for 3.5 hours. The reaction was cooled, poured into water (150 ml) and the precipitate filtered and air dried. Recrystallization from ethyl acetate and hexane gave 1.4 g (55%) of the desired product as a crystalline solid: mp 158-161°C. Anal. Calc'd. for C₁₆H₁₃N₂O₂FS₂ (MW 348.42): C, 55.16; H, 3.76; N, 8.04; S, 18.41. Found: C, 54.95; H, 3.77; N, 7.70; S, 17.98.

### Example 48

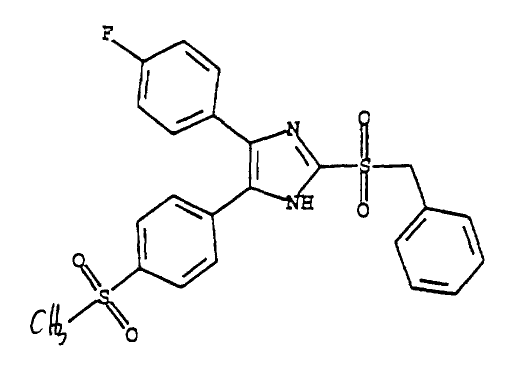

### 4-(4-Fluorophenyl)-5-[4-(methylsulfonyl)phenyl]-2-[(phenylmethyl)sulfonyl]-1H-imidazole

### Step 1: Preparation of 2-thiobenzyl-4-(4-fluorophenyl)-5-(4-methylsulfonylphenyl)-1H-imidazole

A solution of 4-(4-fluorophenyl)-5-[4-(methylsulfonyl)phenyl]-1H-imidazole-2-thiol (Example 49) (300 mg, 0.86 mmol) and benzyl bromide (150 mg, 0.87 mmol) in 2-propanol was heated at reflux for 3 hours. After cooling and removal of the solvent, the residue was stirred with 5% aqueous potassium carbonate (10 ml) and methylene chloride (15 ml). The organic layer was separated and dried over sodium sulfate. The drying agent was filtered, the filtrate concentrated in vacuo and the residue was purified by chromatography on silica gel using 25% ethyl acetate in toluene as the eluent to give 170 mg (40%) of the desired thiobenzyl compound as a glass: Anal. Calc'd. for C₂₃H₁₉N₂O₂FS₂ (MW 438.55): C, 62.99; H, 4.37; N, 6.39; S, 14.62. Found : C, 62.52; H, 4.19; N, 6.08; S, 14.41.

### Step 2: Preparation of 4-(4-fluorophenyl)-5-[4-(methylsulfonyl)phenyl]-2-[(phenylmethyl)sulfonyl]-1H-imidazole

The title compound was synthesized according to the procedure of Example 41, Step 4 using the thiobenzyl compound of Step 1 (160 mg, 0.36 mmol) and Oxone® (350 mg, 0.43 mmol) in methanol (5 ml), tetrahydrofuran (3 ml) and water (3 ml). There was isolated 545 mg (96%) of the desired product as a crystalline solid: mp 211-213°C, after recrystallization from ethyl acetate and hexane. Anal. Calc'd. for C₂₃H₁₉N₂O₄FS₂ (MW 470.54): C, 58.71; H, '4.07; N, 5.95; S, 13.63. Found: C, 58.43; H, 3.79; N, 5.66; S. 13.69.

### Example 49

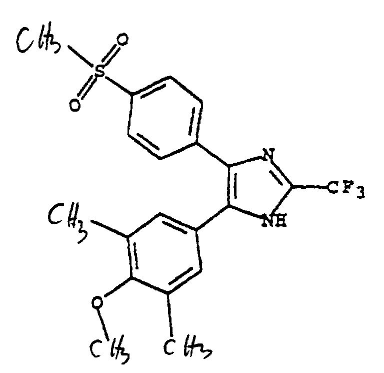

### 5-(3,5-Dimethyl-4-methoxyphenyl)-4-[4-(methylsulfonyl)phenyl]-2-(trifluoromethyl)-1H-imidazole

### Step 1 : Preparation of methyl-4-methoxy-3,5-dimethylbenzoate

A solution of 4-methoxy-3,5-dimethyl benzoic acid (10 g, 45 mmol) and sulfuric acid (5 g) in methanol (200 ml) was stirred at 25°C for 3 days. The mixture was poured into dilute aqueous sodium bicarbonate solution and extracted with ethyl acetate. The combined organic layers were dried over sodium sulfate and concentrated to give 9 g (94%) of the desired compound which was used without further purification.

### Step 2 : Preparation of 4-methoxy-3,5-dimethylbenzyl alcohol

A mixture of ester from Step 1 (9 g, 49 mmol) and 1M lithium aluminum hydride solution in tetrahydrofuran (50 ml) in tetrahydrofuran (150 ml) was heated at reflux for 1 hour under an argon atmosphere. After cooling, the reaction was quenched by adding water (2 ml), 15% aqueous sodium hydroxide solution (2 ml) and more water (6 ml). The precipitated aluminum salts were filtered and the filtrate was evaporated to give 7.5 g (95%) of the desired compound suitable for use in the next reaction without further purification.

### Step 3: Preparation of 4-methoxy-3,5-dimethylbenzaldehyde

The aldehyde was synthesized according to the procedure of Example 46, Step 1 using the alcohol from Step 2 (7.5 g, 45 mmol), oxalyl chloride (6.35 g, 50 mmol), dimethylsulfoxide (8.8 g, 100 mmol) and triethylamine (10.2 g, 110 mmol) and in methylene chloride (100 ml). There was obtained 7 g (92%) of the desired aldehyde which was used in the next step without further purification.

### Step 4: Preparation of 4-methylthiophenyl-4'-methoxy-3',5'-dimethylphenyl benzoin

The benzoin was synthesized according to the procedure of Example 41, Step 2 using the aldehyde of Step 3 (3.3 g, 20 mmol), the cyanohydrin of Example 41, Step 1 (5 g, 20 mmol) and 1M lithium bis(trimethylsilyl)amide in tetrahydrofuran (23 ml, 23 mmol). The crude compound was purified by chromatography on silica gel using 25% ethyl acetate in hexane to give 2.7 g (40%) of the benzoin as a crystalline solid: mp 121-125°C. Anal. Calc'd. for C₁₈H₂₀O₃S (MW 316.42): C, 68.33; H, 6.37; S, 10.13. Found: C, 68.00; H, 6.20; S, 10.00.

### Step 5: Preparation of 4-methylthiophenyl-4'-methoxy-3',5'-dimethylphenyl benzil

The benzil was synthesized according to the procedure of Example 41, Step 3 using the benzoin of Step 4 (1.6 g, 5 mmol) and bismuth oxide (2.9 g, 6 mmol) in acetic acid (16 ml). There was obtained 1.5 g (95%) of the desired benzil as a crystalline solid: mp L32-134°C. Anal. Calc'd. for C₁₈H₁₈O₃S (MW 314.40): C, 68.76; H, 5.77; S, 10.20. Found: C, 68.70; H, 5.81; S, 10.25.

### Step 6: Preparation of 4-methylsulfonylphenyl-4'-methoxy-3',5'-dimethylphenyl benzil

The sulfone was synthesized according to the procedure of Example 41, Step 4 using the compound of Step 5 (1.5 g, 4.8 mmol) and Oxone® (7.3 g, 10.5 mmol) in methanol (50 ml), tetrahydrofuran (25 ml) and water (25 ml). There was isolated 1.1 g (70%) of the desired sulfone as a crystalline solid: mp 158-160°C. Anal. Calc'd. for C₁₈H₁₈O₅S (MW 346.40): C, 62.41; H, 5.24; S, 9.26. Found: C, 62.30; H, 5.30; S, 9.35.

### Step 7 : Preparation of 5-(3,5-dimethyl-4-methoxyphenyl)-4-[4-(methylsulfonyl)phenyl]-2-(trifluoromethyl)-1H-imidazole

The title compound was synthesized according to the procedure of Example 41, Step 5 using the sulfone of Step 6 (1.1 g, 3.2 mmol), ammonium acetate (1.5 g, 16 mmol) and trifluoroacetaldehyde ethyl hemiacetal (1.2 g, 6.4 mmol) in acetic acid (16 ml). The crude product was purified by chromatography on silica gel using 20% ethyl acetate in toluene as the eluent give 563 mg (44%) of the desired product as a crystalline solid: mp 265-267°C, after recryscallization from ethanol. Anal. Calc'd. for C₂₀H₁₉N₂O₃F₃S (MW 424.44): C, 56.60; H, 4.51; N, 6.60; S, 7.55. Found: C, 56.75; H, 4.69; N, 6.31; S, 7.50.

### Example 50

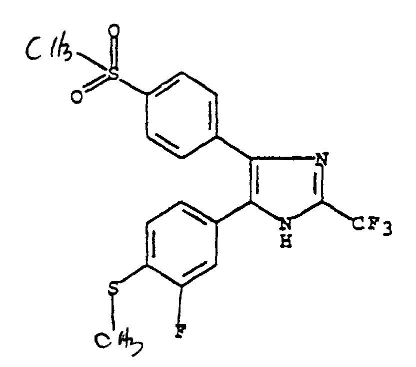

### 5-[3-Fluoro-4-(methylthio)phenyl]-4-[4-(methylsulfonyl)phenyl]-2-(trifluoromethyl) -1H-imidazole

### Step 1: Preparation of 4-methylthio-3-fluorobenzaldehyde

A solution of 3,4-difluorobenzaldehyde (2.8 g, 20 mmol) and sodium thiomethoxide (1.5 g, 20 mmol) in dimethylformamide (20 ml) was heated at 80°C under an argon atmosphere for 2 hours. The reaction was cooled, poured into water (125 ml) and extracted with methylene chloride. The combined organic layers were dried over sodium sulfate and evaporated. The residue was distilled (bp 110-111°C, 0.5 mm Hg) to give 2.5 g (75%) of the desired aldehyde: Anal. Calc'd. for C₈H₇FSO (MW 170.21): C, 56.45; H, 4.15; S, 18.84. Found: C, 55.40; H, 4.00; S, 18.90.

### Step 2: Preparation of 4-fluorophenyl-3'-fluoro-4'-methylthiophenyl benzoin

The title compound was synthesized according to the procedure of Example 41, Step 2 using the aldehyde of Step 1 (2.5 g, 14 mmol), 4-fluorobenzaldehyde trimethylsilylcyanohydrin (3.3 g, 14 mmol) and 1M lithium bis(trimethylsilyl)amide in tetrahydrofuran (16 ml, 16 mmol). The crude benzoin was purified by chromatography on silica gel using 10% ethyl acetate in toluene to give 2.1 g (49%) of the compound as an oil. Anal. Calc'd. for C₁₅H₁₂F₂O₂S (MW 294.32): C, 61.21; H, 4.11; S, 10.89. Found: C, 61.10; H, 4.15; S, 10.90.

### Step 3: Preparation of 4-fluorophenyl-3'-fluoro-4'-methylthiophenyl benzil

The benzil was synthesized according to the procedure of Example 41, Step 3 using the benzoin of Step 2 (2.1 g, 7 mmol) and bismuth oxide (3.5 g, 8,4 mmol) in acetic acid (19 ml). There was obtained 2 g (95%) of the desired benzil as a crystalline solid: mp 92-93°C. Anal. Calc'd. for C₁₅H₁₀F₂O₂S (MW 292.31): C, 61.64; H, 3.45; S, 10.97. Found: C, 61.43; H, 3.21; S, 11.05.

### Step 4: Preparation of 4-methylsulfonylphenyl-3'-fluoro-4'-methylthiophenyl benzil

A mixture of the benzil from Step 3 (2 g, 6.8 mmol) and sodium methylsulfinate (775 mg, 7.1 mmol) in dimethylformamide (10 ml) was stirred at 90°C for 16 hours. The reaction was cooled, poured into water (120 ml) and the mixture was extracted with methylene chloride. The organic layer was dried over sodium sulfate, filtered and the filtrate concentrated. The residue was recrystallized from ethyl acetate and hexane to give 760 mg (45%) of the desired sulfone: mp 130-132°C. Anal. Calc'd. for C₁₆H₁₃FO₄S₂ (MW 352.41): C, 54.53; H, 3.72; S, 18.20. Found: C, 54.20; H, 3.51; S, 18.40.

### Step 5: Preparation of 5-[3-fluoro-4-(methylthio)phenyl]-4-[4-(methylsulfonyl)phenyl]-2-(trifluoromethyl)-1H-imidazole

The imidazole was synthesized according to the procedure of Example 41, Step 5 using the sulfone of Step 4 (750 mg, 2.1 mmol), ammonium acetate (850 mg, 10.5 mmol) and trifluoroacetaldehyde ethyl hemiacetal (675 mg, 4.2 mmol) in acetic acid (10 ml). There was obtained 305 mg (35%) of the desired product as a crystalline solid: mp 272-274°C, after recrystallization from ethyl acetate. Anal. Calc'd. for C₁₈H₁₄N₂F₄O₂S₂ (MW 430.44): C, 50.23; H, 3.28; N, 6.51; S, 14.90. Found: C, 50.41; H, 3.32; N, 6.21; S, 14.80.

### Example 51

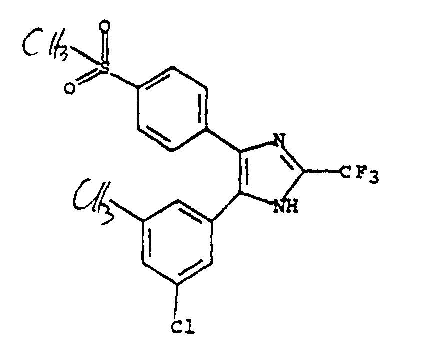

### 5-(3-Chloro-5-methylphenyl)-4-[4-(methylsulfonyl)phenyl]-2-(trifluoromethyl)-1H-imidazole

### Step 1: Preparation of 4-fluorophenyl-3'-methyl-5'-chlorophenyl benzoin

The benzoin was synthesized according to the procedure of Example 41, Step 2 using 4-fluorobenzaldehyde trimethylsilylcyanohydrin (4 g, 18 mmol), 3-methyl-5-chlorobenzaldehyde (2.78 g, 18 mmol) and 1M lithium bis(trimethylsilyl)amide in tetrahydrofuran (20 ml, 20 mmol). The crude compound was purified by chromatography on silica gel using 5% ethyl acetate in toluene as the eluent to give 1.85 g (37%) of the benzoin as a crystalline solid: mp 101-103°C. Anal. Calc'd. for C₁₅H₁₂FClO₂ (MW 278.71): C, 64.64; H, 4.34. Found: C, 64.51; H, 4.29.

### Step 2: Preparation of 4-fluorophenyl-3'-methyl-5'-chlorophenyl benzil

The benzil was synthesized according to the procedure of Example 41, Step 3 using the benzoin of Step 1 (1.85 g, 6.6 mmol) and bismuth oxide (3.3 g, 7.3 mmol) in acetic acid (15 ml). There was obtained 1.6 g (90%) of the desired benzil as a crystalline solid: mp 115-116°C. Anal. Calc'd. for C₁₅H₁₀FClO₂ (MW 276.70) : C, 65.11; H, 3.64. Found: C, 65.00; H, 3.48.

### Step 3: Preparation of 4-methylsulfonylphenyl-3'-methyl-5'-chlorophenyl benzil

Following the procedure of Example 53, Step 4 and using the product from Step 2 (1.5 g, 5.4 mmol) and sodium methylsulfinate (665 mg, 6.5 mmol) in dimethylformamide (15 ml), there was obtained 1.4 g (75%) of the desired benzil sulfone: mp 145-147 °C, after recrystallization from ethyl acetate and hexane. Anal. Calc'd. for C₁₆H₁₃ClO₄S (MW 336.80): C, 57.06; H, 3.89; S, 9.52. Found: C, 56.98; H, 3.71; S, 9.62.

### Step 4: Preparation of 5-(3-chloro-5-methylphenyl)-4-(4-(methylsulfonyl)phenyl]-2-(trifluoromethyl)-1H-imidazole

The title compound was synthesized according to the procedure of Example 41, Step 5 using the benzil sulfone of Step 3 (1 g, 3 mmol), ammonium acetate (1.25g, 15 mmol) and trifluoroacetaldehyde ethyl hemiacetal (875 mg, 6 mmol) in acetic acid (15 ml). There was obtained 480 mg (45%) of the desired product as a crystalline solid: mp 260-262°C, after recrystallization from ethyl acetate and hexane. Anal. Calc'd. for C₁₈H₁₄N₂O₂F₃ClS (MW 414.84.): C, 52.12; H, 3.40; N, 6.75; S, 7.73. Found: C, 52.47, H, 3.3,1; N, 6.52; S, 7.57.

### Example 52

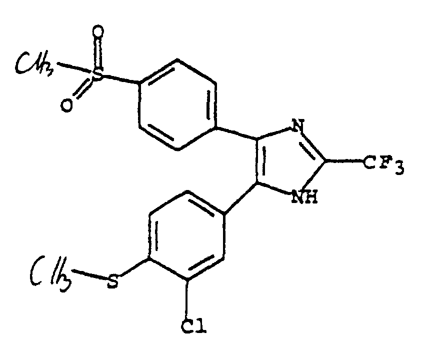

### 5- [3-Chloro-4-(methylthio)phenyl]-4-[4-(methylsulfonyl)phenyl]-2-(trifluoromethyl)-1H-imidazole

### Step 1: Preparation of 3-chloro-4-methylthio benzaldehyde

The aldehyde was synthesized according to the procedure of Example 53, step 1 using 4-fluoro-3-chlorobenzaldehyde (2.3 g, 14 mmol) and sodium methyl mercaptide (1.08 g, 15 mmol) in dimethylformamide (10 ml). There was obtained 1.58 g (60%) of the desired aldehyde as a crystalline solid: mp 59-61°C, after chromatography on silica gel using 5% ethyl acetate in hexane as the eluent. Anal. Calc'd. for C₈H₇ClOS (MW 186.66): C, 51.48; H, 3.78; S, 17.18. Found: C, 51.29; H, 3.52; S, 17.10.

### Step 2: Preparation of 4-fluorophenyl-3'-chloro-4'-methylthiophenyl benzoin

The benzoin was synthesized according to the procedure of Example 41, Step 2 using the compound of Step 1 (1.5 g, 8 mmol), 4-fluorobenzaldehyde trimethylsilylcyanohydrin (1.78 g, 8 mmol) and 1M lithium bis(trimethylsilyl)amide in tetrahydrofuran (9 ml, 9 mmol). The crude compound was purified by chromatography on silica gel using 10% ethyl acetate in toluene to give 1.5 g (61%) of the benzoin as a crystalline solid: mp 96-98°C. Anal. Calc'd. for C₁₅H₁₂O₂FClS (MW 310.78) : C, 57.97; H, 3.89; S, 10.32. Found: C, 58.04; H, 3.80; S, 10.47.

### Step 3: Preparation of 4-fluorophenyl-3'-chloro-4'-methylthiophenyl benzil

The benzil was synthesized according to the procedure of Example 41, Step 3 using the benzoin of Step 2 (1.5 g, 4.8 mmol) and bismuth oxide (2.65 g, 5.3 mmol) in acetic acid (15 ml). There was obtained 1.45 g (98%) of the desired benzil as a crystalline solid: mp 124-126°C. Anal. Calc'd. for C₁₅H₁₀O₂FClS (MW 308.77): C, 58.35; H, 3.26; S, 10.38. Found: C, 58.18; H, 3.09; S, 10.55.

### Step 4: Preparation of 4-methylsulfonylphenyl-3'-chloro-4'-thiomethylphenyl benzil

Following the procedure of Example 53, Step 4 using the benzil of Step 3 (1.5 g, 4.9 mmoles) and sodium methylsulfinate (620 mg, 5.5 mmol) in dimethylformamide (15 ml), there was obtained 450 mg, (25%) of the desired sulfone: mp 138-139°C, after recrystallization from ethyl acetate. Anal. Calc'd. for C₁₆H₁₃O₄ClS₂ (MW 368.86) : C, 52.10; H, 3.55; S, 17.39. Found: C, 51.93; H, 3.31; S, 17.52.

### Step 5: Preparation of 5-[3-chloro-4-(methylthio)phenyl]-4-[4-(methylsulfonyl)phenyl]-2-(trifluoromethyl)-1H-imidazole

The title compound was synthesized according to the procedure of Example 41, Step 5 using the sulfone of Step 4 (440 mg, 1.2 mmol), ammonium acetate (560 mg, 7.2 mmol) and trifluoroacetaldehyde ethyl hemiacetal (390 mg, 2.4 mmol) in acetic acid (7 ml). The crude product was purified by recrystallization from ethyl acetate and hexane to give 283 mg (53%) of the title compound as a crystalline solid: mp 255-257C. Anal. Calc'd. for C₁₈H₁₄N₂O₂F₃ClS₂ (MW 446.90): C,48.38; H, 3.16; N, 6.27; S, 14.35. Found: C, 48.54; H, -3.15; N, 5.89; S, 14.57.

### Example 53

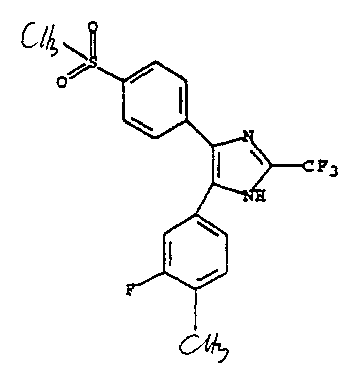

### 5-(3-Fluoro-4-methylphenyl)-4-[4-(methylsulfonyl)phenyl]-2-(trifluoromethyl)-1H-imidazole

### Step 1: Preparation of 3-fluoro-4-methylbenzaldehyde

*tert*-Butyllithium (31 ml of a 1.7 M solution in toluene, 52 mmol) was added slowly over 30 minutes to a cold (-70°C), stirred solution of 4-bromo-2-fluorotoluene (5 g, 26 mmol) in tetrahydrofuran (25 ml). After addition was complete, the mixture was stirred for 15 minutes and a solution of dimethylformamide (2 g, 28 mmol) in tetrahydrofuran (10 ml) was then added over 10 minutes. After addition was complete, the reaction solution was warmed to room temperature over 30 minutes. The reaction was poured into 0.5N aqueous potassium bisulfate (125 ml) and extracted with ethyl ether. The combined organic extracts were dried over sodium sulfate and evaporated to give 3.3 g (90%) of the desired product as an oil. This material was suitable for use without further purification.

### Step 2: Preparation of 4-fluorophenyl-3'-fluoro-4'-methylphenyl benzoin

The benzoin was synthesized according to the procedure of Example 41, Step 2 using the product of Step 1 (3.3 g, 24 mmol), 4-fluorobenzaldehyde trimethylsilylcyanohydrin (5.3 g 24 mmol) and 1M lithium bis(trimethylsilyl)amide in tetrahydrofuran (25 ml, 25 mmol). The crude compound was purified by chromatography on silica gel using 10% ethyl acetate in toluene as the eluent to give 2.4 g (48%) of the title benzoin as a crystalline solid: mp 69-72°C. Anal. Calc'd. for C₁₅H₁₂F₂O₂ (MW 262.25) : C, 68.70; H, 4.61. Found: C, 68.53; H, 4.49.

### Step 3: Preparation of 4-fluorophenyl-3'-fluoro-4'-methylphenyl benzil

The benzil compound was synthesized according to the procedure of Example 41, Step 3 using the product of Step 2 (2.4 g, 9 mmol) and bismuth oxide (5 g, 12 mmol) in acetic acid (25 ml). There was obtained 2.3 g (95%) of the desired benzil as a crystalline solid: mp 81-82°C. Anal. Calc'd. for C₁₅H₁₀F₂O₂ (MW 260.24): C, 69.23; H, 3.87. Found: C, 69.04; H, 3.80.

### Step 4: Preparation of 4-methylsulfonylphenyl-3'-fluoro-4'-methylphenyl benzil

Following the procedure of Example 53, Step 4 using the benzil from Step 3 (2.15 g 8.2 mmol) and sodium methylsulfinate (1.05 g, 10 mmol) in dimethyl formamide (20 ml), there was obtained 1 g (50%) of the desired sulfone: mp 146-147°C, after recrystallization from ethyl acetate and hexane. Anal. Calc'd. for C₁₆H₁₃FO₄S (MW 320.34): C, 59.99; H, 4.09; S, 10.01. Found: C, 59.68; H, 4.17; S, 10.13.

### Step 5: Preparation of 5-(3-fluoro-4-methylphenyl)-4-[4-(methylsulfonyl)phenyl]-2-(trifluoromethyl)-1H-imidazole

The title compound was synthesized according to the procedure of Example 41, Step 5 using the product of Step 4 (355 mg, 1.1 mmol), ammonium acetate (510 mg, 6.6 mmol) and trifluoroacetaldehyde ethyl hemiacetal (360 mg, 2.2 mmol) in acetic acid (5 ml). The crude product was purified by recrystallization from ethyl acetate and hexane to give 140 mg (40%) of the title compound: mp 251-252°C. Anal. Calc'd. for C₁₈H₁₄N₂F₄O₂S (MW 398.38): C, 54.27; H, 3.54; N, 7.03; S, 8.05. Found: C, 53.98; H, 3.40; N, 6.72; S, 7.84.

### Example 54

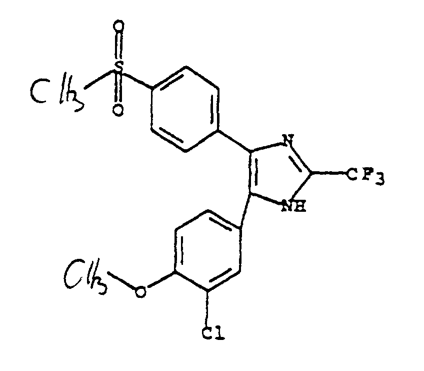

### 5-(3-Chloro-4-methoxyphenyl)-4-[4-(methylsulfonyl)phenyl]-2-(trifluoromethyl)-1H-imidazole

### Step 1 : Preparation of 4-fluorophenyl-3'-chloro-4'-methoxyphenyl benzoin

The benzoin compound was synthesized according to the procedure of Example 41, Step 2 using 4-fluorobenzaldehyde trimethylsilylcyanohydrin (5.3 g, 24 mmol), 3-chloro-4-methoxybenzaldehyde (4.1 g, 24 mmol) and 1M lithium bis(trimethylsilyl)amide in tetrahydrofuran (25 ml, 25 mmol). The crude compound was purified by chromatography on silica gel using 10% ethyl acetate in toluene as the eluent to give 3.5 g (51%) of the title benzoin as a crystalline solid: mp 119-121°C. Anal. Calc'd. for C₁₅H₁₂ClFO₃ (MW 294.71): C, 61.13; H, 4.10. Found: C, 61.00; H, 4.21.

### Step 2: Preparation of 4-fluorophenyl-3'-chloro-4'-methoxyphenyl benzil

The benzil compound was synthesized according to the procedure of Example 41, Step 3 using the product of Step 1 (3.5 g, 12 mmol) and bismuth oxide (6.6 g, 14 mmol) in acetic acid (35 ml). There was obtained 2.8 g (85%) of the desired benzil as a crystalline solid: mp 152-154°C. Anal. Calc'd. for C₁₅H₁₀FClO₃ (MW 292.70) : C, 61.55; H, 3.44. Found: C, 61.40; H, 3.31.

### Step 3: Preparation of 4-methylsulfonylphenyl-3'-chloro-4'-methoxyphenyl benzil

Following the procedure of Example 53, Step 4 using the benzil from Step 2 (2.8 g, 9.6 mmol) and sodium methylsulfinate (1.25 g, 12 mmol) in dimethylformamide (25 ml), there was obtained 2.3 g (70%) of the desired product: mp 163-166°C. Anal. Calc'd. for C₁₆H₁₃ClO₅S (MW 352.79): C, 54.47; H, 3.71; S, 9.09. Found: C, 54.11; H, 3.52; S, 9.30.

### Step 4: Preparation of 5-(3-chloro-4-methoxyphenyl)-4-[4-(methylsulfonyl)phenyl]-2-(trifluoromethyl)-1H-imidazole

The title compound was synthesized according to the procedure of Example'41, Step 5 using the sulfone of Step 3 (2.2 g, 6.5 mmol), ammonium acetate (3 g, 20 mmol) and trifluoroacetaldehyde ethyl hemiacetal (2.1g, 13 mmol) in -acetic acid (30 ml). The crude product was purified by chromatography on silica gel using 25% ethyl acetate in toluene as the eluent give 760 mg (35%) of the desired product as a crystalline solid: mp 288-291°C, after recrystallization from ethyl acetate and hexane. Anal. Calc'd. for C₁₈H₁₄F₃ClN₂O₃S (MW 430.83): C, 50.16; H, 3.28; N, 6.50; S, 7.44. Found: C, 50.17; H, 3.17; N, 6.31; S, 7.58.

### Example 55

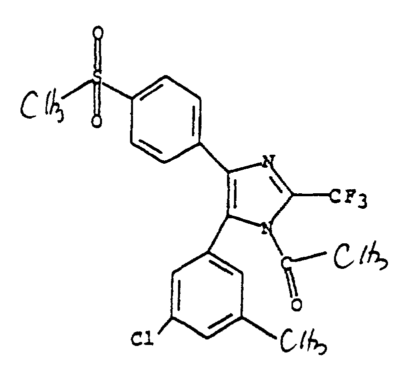

### 1-[5-(3-Chloro-5-methylphenyl)-4-[4-(methylsulfonyl)phenyl]-2-(trifluoromethyl)-1H-imidazol-1-yl]ethanone

Acetyl chloride (50 mg, 0.6 mmol) was added to a cold (5°C), stirred solution of Example 54 (150 mg, 0.3 mmol) and triethylamine (80 mg, 0.8 mmol) in acetonitrile (3 ml). The reaction was warmed to room temperature and stirred for 4 hours. The solvent was removed and the residue was purified by chromatography on silica gel using 20% ethyl acetate in toluene as the eluent to give 15 mg of the desired product as a crystalline solid: mp 257°C. Anal. Calc'd. for C₂₀H₁₆N₂O₃ClF₃S (MW 456.87): C, 52.58; H, 3.53; N, 6.13; S, 7.02. Found: C, 52.21; H, 3.61; N, 5.95; S, 7.30.

### Example 56

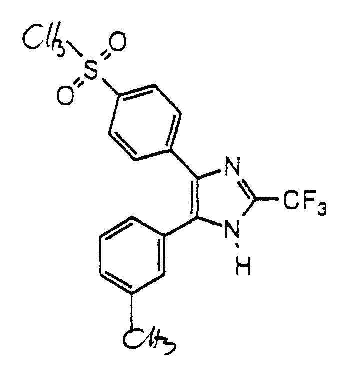

### 5-(3-Methylphenyl)-4-[4-(methylsulfonyl)phenyl]-2-trifluoromethyl-1H-imidazole

### Step 1: Preparation of 3-methyl-α-[(trimethylsilyl)oxy]benzeneacetonitrile

The silyl compound was prepared by allowing trimethylsilylcyanide (52 mmol, 7-ml) and m-tolualdehyde (50 mmol, 5.88 ml) to stir at room temperature in the presence of zinc(II) iodide (50 mg) for 3 hours. The compound (10.6 g, 96%) was obtained as an oil by distilling the reaction mixture *in vacuo*: bp 89 °C, ca. 1 torr).

### Step 2: Preparation of 2-hydroxy-1-(3-methylphenyl)-2-[4-(methylthio)phenyl]-ethanone

Dry tetrahydrofuran (100 ml) was introduced to a dry round-bottom flask, and cooled to -78%C. Lithium hexamethyldisilazide (1M in THF, 50 ml) was added, followed by the silyl compound from step 1 (48 mmol, 10.6 g), rinsing with ca. 10 ml additional THF to ensure complete transfer. After 30 minutes, (4-methylthio)benzaldehyde (50 mmol, 6.67 ml), was added. After 1 hour the reaction was quenched with 10% HCl (200 ml) and KHF₂ (60 mmol, 4.68 g). The resulting mixture was stirred at ambient temperature for 1 hour, then extracted using ethyl acetate (400 ml). The organic phase was separated and washed with sat. NaCl (60 ml). The organic phase was dried over magnesium sulfate, filtered, concentrated, and subjected to silica gel chromatography (2:1 hexanes: ethyl acetate), affording the title material (8.31 g, 62%) as a waxy semisolid, which was used in the next step.

### Step 3: Preparation of 1-(3-methylphenyl)-2-[4-(methylthio)phenyl]ethane-1,2-dione

The ketone from step 2 (30.5 mmol, 8.31 g) was diluted with acetic acid (90 ml) and heated to ∼90°C. Bi₂O₃ (30.5 mmol, 14.2 g) was added. After stirring 15 minutes, additional bismuth oxide (15 mmol, 7 g) was added. After 30 additional minutes, the mixture was filtered through silica and Celite®, then lyophilized. The residue was subjected to chromatography (3:1 hexane: ethyl acetate), affording the title compound as a yellow solid (3.56 g, 43%), which was used in the next step.

### Step 4 : Preparation of 5-[3-methylphenyl)-4-[4-(methylthio)phenyl]-2-(trifluoromethyl)-1H-imidazole

The dione from step 3 (13 mmol, 3.51 g) was combined with acetic acid (40 ml), ammonium acetate (78 mmol, 6.01 g) and ethyl trifluoroacetaldehyde hemiacetal (39 mmol, 4.53 ml). The mixture was stirred at reflux for 1 hour. Additional hemiacetal (ca 13 mmol, 1.5 ml) was added and heating was continued for another hour. The mixture was cooled, lyophilized, and subjected to chromatography (5% methanol in toluene), affording the product as a thick semi-solid (2.35 g, 54%) which was carried through the next step.

### Step 5: Preparation of 5-(3-methylphenyl)-4-[4-(methylsulfonyl)phenyl]-2-(trifluoromethyl)-1H-imidazole

Acetic acid (30 ml) was added to the imidazole from step 4 (6.95 mmol, 2.35 g), followed by 30% hydrogen peroxide (∼24 mmol, 2.4 ml). The mixture was heated for thirty minutes over a steam bath, cooled, lyophilized, and then purified by chromatography (ethyl acetate). Final purification was accomplished by recrystallization from acetonitrile , affording the title compound (0.60 g, 24%): Anal. Calc'd. for C₁₈H₁₅F₃N₂O₂S: C, 56.84; H, 3.97; N, 7.36. Found: C, 56.80; H, 4.09; N, 7.29.

### Example 57

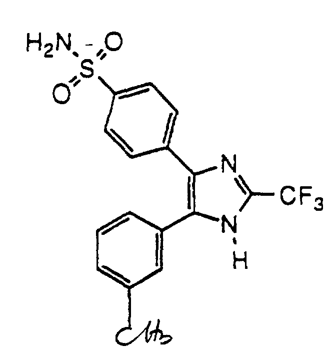

### 5-(3-Methylphenyl)-4-[4-(sulfonamido)phenyl]-2-trifluoromethyl-1H-imidazole

### Step 1: Preparation of 5-(3-methylphenyl)-4-[4-(methylsulfonyl)phenyl]-2-(trifluoromethyl)-1-[(2-trimethylsilylethoxy)methyl]-1H-imidazole

Sodium hydride (60% in oil, 1.06 mmol, 42 mg) was washed with hexane (*ca* 2 X 1 ml), then covered with N,N-dimethyformamide (2.1 ml). 5-(3-Methylphenyl)-4-[4-(methylsulfonyl)phenyl]-2-trifluoromethyl-1H-imidazole (Example 59) (0.70 mmol, 268 mg) was added, with stirring. After 15 minutes, 2-(trimethylsilyl)ethoxymethyl chloride (SEM-Cl) (1.06 mmol, 0.19 ml) was added and the mixture was stirred overnight. The reaction was quenched with saturated ammonium chloride (ca. 4 drops), and concentrated *in vacuo*. The residue was subjected to chromatography (2:1 hexane: ethyl acetate) and concentrated to afford the title compound (284 mg, 79%) as an oil as a mixture of the two possible N alkylated isomers (by NMR).

### Step 2: Preparation of 5-(3-methylphenyl)-4-[4-(sulfonamido)phenyl]-2-trifluoromethyl-1H-imidazole

The compound of step 1 (1.0 mmol, 510 mg) was diluted with tetrahydrofuran (3 ml) and cooled to 0°C. n.-Butylmagnesium chloride (2.0 M in THF, 2.5 ml) was added over 1 minute. After 5 minutes, the cold bath was removed and the reaction mixture was warmed the reflux. The solution was cooled to 0°C after 40 hours at reflux, and a solution of hydroxylamine O-sulphonic acid (0.9 g) and sodium hydroxide (0.9 g) in water (4 ml) was added. The reaction mixture was stirred for 7 hours at ambient temperature, and extracted into ethyl acetate (50 ml). The organic phase was dried over magnesium sulphate, filtered through a silica plug, and concentrated. The residue was diluted with ethanol (8 ml) and 6N HCl (2 ml). After 90 minutes, a solution of ammonium hydroxide (0.5 ml) in water (5 ml) was added, and the mixture was taken up with ethyl acetate (100 ml). The organic phase was separated, dried using magnesium sulphate, concentrated, and subjected to chromatography (2:1 hexane: ethyl acetate). The product was re-concentrated from acetone to afford the title compound as a white powder (173 mg, 45%) : Anal. Calc'd. for C₁₇H₁₄F₃N₃O₂S•(1/2 acetone) : C, 54.14; H, 4.18; N, 10.24. Found: C, 54.06; H, 4.23; N, 9.86.

### Example 58

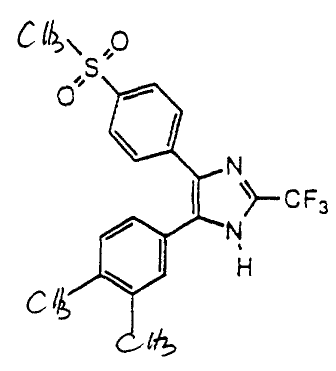

### 5-(3,4-Dimethylphenyl)-4-[4-(methylthio)phenyl]-2-(trifluoromethyl)-1H-imidazole

### Step 1: Preparation of 3,4-dimethyl-α-[(trimethylsilyl)oxy]benzeneacetonitrile

The title compound was prepared by the method of Example 59, step 1, using (41 mmol, 5.5 g) of 3,4-dimethylbenzaldehyde and affording the title compound as an oil (8.51 g, 98%) (bp_{∼1torr}=105°C).

### Step 2: Preparation of 1-(3,4-dimethylphenyl)-2-hydroxy-2-[4-(methylthio)phenyl]-ethanone

The title compound was prepared by the method of Example 59, step 2, using (40.9 mmol, 8.50 g) of 3,4-dimethyl-α-[(trimethylsilyl)oxy]benzeneacetonitrile from Step 1, and affording (9.7 g, 83%) of the title compound as an oil.

### Step 3 : Preparation of 1-(3,4-dimethylphenyl)-2-[4-(methylthio)phenyl]ethane-1,2-dione

The title compound was prepared from 1-(3,4-dimethylphenyl)-2-hydroxy-2-[4-(methylthio)phenyl]-ethanone from step 2 (34 mmol, 9.7 g)) by the method of Example 59, step 3. The title compound (4.65 g, 50%), a yellow solid, was used directly in the next step.

### Step 4 : Preparation of 5-(3,4-dimethylphenyl)-4-[4-(methylthio)phenyl]-2-(trifluoromethyl)-1H-imidazole

The title compound was obtained by the method of Example 59, step 4, using 1-(3,4-dimethylphenyl)-2-[4-(methylthio)phenyl]ethane-1,2-dione from step 3 (16 mmol, 4.5 g). The product was a viscous oil (2.3 g, 41%).

### Step 5: Preparation of 5-(3,4-dimethylphenyl)-4-[4-(methylsulfonyl)phenyl]-2-(trifluoromethyl)-1H-imidazole

The title compound was obtained from 5-(3,4-dimethylphenyl)-4-[4-(methylthio)phenyl]-2-(trifluoromethyl)-1H-imidazole (Step 4) (6.6 mmol, 2.3 g) by the method of Example 59, step 5. This analog was recrystallized first from 2-butanone, and then acetone, and finally dried by concentration from acetonitrile, affording the desired product (150 mg, 6%). mp (DSC) 279-284°C. Anarl. Calc'd. for C₁₉H₁₇F₃N₂O₂S•1/3 H₂O: C, 56.99; H, 4.45; N, 7.00.

Found: C, 56.95; H, 4.08; N, 7.00.

### Example 59

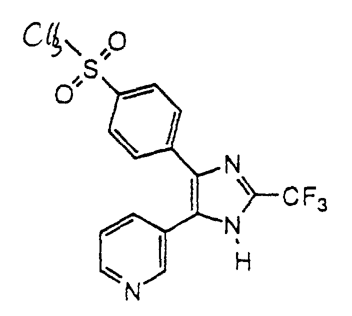

### 4-[4-(Methylsulfonyl)phenyl]-5-(3-pyridyl)-2-(trifluoromethyl)-1H-imidazole

### Step 1: Preparation of 4-methylthio-α-((trimethylsilyl)oxy]benzeneacetonitrile

To a mixture of KCN (50 mg) and trimethysilylcyanide (55 mmol, 7.31 ml) was added 4-methylthiobenzaldehyde (50 mmol, 6.66 ml). The reaction was stirred at ambient temperature 4 hours, then subjected to distillation (bp_{~1torr}=131°C), affording the product as an oil (9.7 g, 77%).

### Step 2: Preparation of 2-hydroxy-1-[4-(methylthio)phenyl]-2-(3-pyridyl)-ethanone

4-Methylthio-α-[(trimethylsilyl)oxy]benzeneacetonitrile (39 mmol, 9.70 g) was added to a solution of lithium hexamethydisilazide (1M in THF, 42 ml) in dry tetrahydrofuran (42 ml) at -78°C. After 15 minutes, 3-pyridinecarboxaldehyde (39 mmol, 4.13 ml) was added. The reaction was quenched after an additional 30 minutes using 10% HCl (150 ml), followed by KHF₂ (4.0 - g). The mixture was warmed to ambient temperature and basified to ca. pH 14, using 50% NaOH. The product was extracted into ethyl acetate (200 ml, then- 2 X 100 ml). The combined organic layers were dried using magnesium sulfate, filtered, concentrated, and subjected to chromatography (2:1 hexane:ethyl acetate), affording the title compound as a solid (6.3 g, 61%), which was used in the next step.

### Step 3: Preparation of 1-[4-(methylthio)phenyl]-2-(3-pyridyl)-ethane-1,2-dione

A solution of dimethylsulphoxide (30 mmol, 2.13 ml) in methylene chloride (90 ml) was cooled to -65°C. Trifluoroacetic acid anhydride (20 mmol, 2.82 ml) was added over 3 minutes, then stirred 10 minutes. 2-Hydroxy-1-[4-(methylthio)phenyl]-2-(3-pyridyl)-ethanone (step 2) (10 mmol, 2.59 g) was suspended in ca 10 ml of methylene chloride and rinsed into the reaction vessel using ca 5 ml additional solvent. After 40 minutes, triethylamine (58 mmol, 8.07 ml) was added. The mixture was warmed to 0°C over 30 minutes, with continued stirring, then diluted with water (100 ml) and extracted with ethyl acetate (200, then 2 X 50 ml). The combined organic layers were dried over magnesium sulfate, filtered, concentrated, and subjected to chromatography (2:1 hexane:ethyl acetate), affording the title compound as a yellow solid (2.43 g, 94%), which was used in the next step.

### Step 4: Preparation of 4-[4-(methylthio)phenyl]-5-(3-pyridyl)-2-(trifluoromethyl)-1H-imidazole

The title compound was synthesized from 1-[4-(methylthio)phenyl]-2-(3-pyridyl)-ethane-1,2-dione (Step 3) by the method of Example 59, step 4, except that after lyophilization, the residue was diluted with ethyl acetate (200 ml) and washed with 10% ammonium hydroxide (100 ml). The aqueous layer was extracted with ethyl acetate (2X 50 ml), and the combined organic layers were dried over magnesium sulfate, filtered, concentrated, and subjected to chromatography, affording the product as a viscous oil (3.13 g, ∼quant.), which was used in the next step.

### Step 5: Preparation of 4-[4-(methylsulfonyl)phenyl]-5-(3-pyridyl)-2-(trifluoromethyl)-1H-imidazole

4-[4-(Methylthio)phenyl]-5-(3-pyridyl)-2-(trifluoromethyl)-1H-imidazole (Step 4) (9.3 mmol, 3.13 g) was diluted with acetic acid (20 ml), treated with hydrogen peroxide (30%, ∼19 mmol, 1.9 ml), and heated over a steam bath for 30 minutes. After subsequent lyophilization, the residue was treated with amberlyst (OH-) resin and washed onto a chromatography column, where the product was eluted using 90:10:1 ethyl acetate:methanol:ammomium hydroxide. The concentrated product was recrystallized twice from ethyl acetate/hexane, and concentrated from acetone solution to remove final traces of water and solvent. The structure of the product (640 mg, 22%) was verified spectroscopically. Anal. Calc'd. for C₁₆H₁₂F₃N₃O₂S: C, 52.31; H, 3.29; N, 11.44. Found: C, 52.74; H, 3.48; N, 10.22.

### Example 60

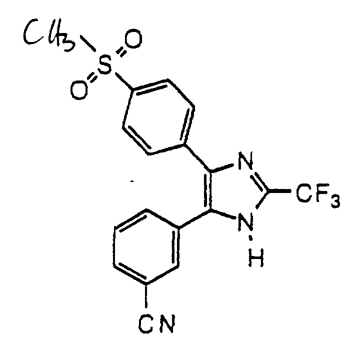

### 5-(3-Cyanophenyl)-4-[4-(methylthio)phenyl]-2-(trifluoromethyl)-1H-imidazole

### Step 1: Preparation of 1-(3-cyanophenyl)-2-hydroxy-2-[4-(methylthio)phenyl]-ethanone

Sodium cyanide (3 mmol, 0.147 g) was added to a mixture of ethanol (30 ml), water (30 ml), 3-cyanobenzaldehyde (30 mmol, 3.93 g) and 4-(methylthio)benzaldehyde (30 mmol, 4.0 ml). The reaction was stirred at reflux 30 minutes, diluted with water (20 ml) and extracted with ethyl acetate (250 ml). The organic layer was separated, dried over magnesium sulfate, concentrated, and the residue purified by silica gel chromatography (2:1 hexane:ethyl acetate), affording the title compound as a solid (5.2, 61%).

### Step 2: Preparation of 1-(3-cyanophenyl)-2-[4-(methylthio)phenyl]ethane-1,2-dione

The ketone from step 1 (7.07 mmol, 2.00 g) was transformed into the title dione using a Swern oxidation similar to Example 62, step 3. The title compound (2.0 g, ∼quant), a yellow solid, was used in the next step.

### Step 4: Preparation of 5-(3-cyanophenyl)-4-[4-(methylthio)phenyl]-2-(trifluoromethyl)-1H-imidazole

1-(3-Cyanophenyl)-2-[4-(methylthio)phenyl]ethane-1,2-dione (Step 3) (7.00 mmol, 1.97 g) was converted into the title compound using the method of Example 59, step 4, affording a foam (1.79 g, 71%).

### Step 5: Preparation of 5-(3-cyanophenyl)-4-[4-(methylsulfonyl)phenyl]-2-(trifluoromethyl)-1H-imidazole

5-(3-cyanophenyl)-4-[4-(methylthio)phenyl]-2-(trifluoromethyl)-1H-imidazole (Step 4) (1.4 mmol, 502 mg) was oxidized using the method of Example 59, step 5. The chromatographed product was recrystallized from ethyl acetate/hexane, then dried by concentration from acetonitrile, affording 132 mg (25%). Anal. Calc'd. for C₁₈H₁₂F₃N₃O₂S: C, 55.24; H, 3.09; N, 10.74. Found: C, 55.25; H, 3.11; N, 10.74.

### Example 61

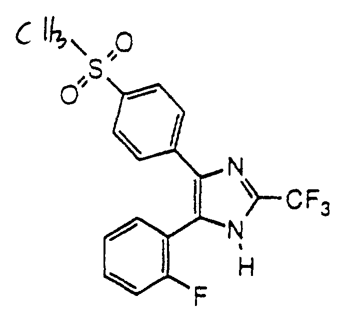

### 5-(2-Fluorophenyl)-4-[4-(methylsulfonyl)phenyl]-2-(trifluoromethyl)-1H-imidazole

### Step 1: Preparation (2-fluorophenyl)methyltriphenylphosphonium bromide

2-Fluorobenzyl bromide (80 mmol, 12.4 ml) was added to a solution of triphenylphosphine (80 mmol, 21.0 g) and tetrabutylammonium iodide (100 mg) in toluene (60 ml). The mixture was stirred at ambient temperature 24 hours, then heated at reflux for 15 minutes. On cooling, the-title compound was isolated by filtration, dried *in vacuo* to constant weight (34 g, 94%) and used without characterization.

### Step 2: Preparation of 1-fluoro-2-[2-[4-(methylsulfonyl)phenyl]ethylenyl]benzene

The phosphonium salt from step 1 (20 mmol, 9.02 g) was suspended in ethanol (60 ml) and cooled to 0°C. n-Butyllithium (1.6 M in hexane, 13.1 ml) was added slowly (over ∼15 minutes). Five minutes after complete addition, {4-methylthio)benzaldehyde (20 mmol, 2.67 ml) was added, and the mixture was stirred 2.5 hours at ambient temperature. Water (40 ml) was added, and the product was extracted into methylene chloride (200 ml). The organic phase was dried over magnesium sulfate, passed through a large silica plug, and concentrated, affording a solid material, which was diluted with methanol (80 ml) and cooled to 0°C. Oxone® (50 mmol, 30.8 g) in aqueous (150 ml) suspension was added, the reaction was stirred at 0°C for 15 minutes, then 1.5 hours at ambient temperature. The product was extracted into methylene chloride (200 ml, then 2 X 50 ml). The combined organic phases were dried (MgSO₄), filtered through a silica plug, and concentrated to afford the title compound (3.5 g, 63%) as a semi-solid mixture (∼2:1) of olefin isomers. The material was used, as is, in the next transformation.

### Step 3: Preparation of 1-(2-fluorophenyl)-2-[4-(methylsulfonyl)phenyl]ethane-1,2-dione

Based on a procedure by: K. B. Sharpless,et al. [J. Am. Chem. Soc., **93,** 3303 (1971)] the stilbene from step 2 (12.5 mmol, 3.45 g) was dissolved in acetic anhydride (60 ml) and cooled to 0°C. Solid KMnO₄ (50 mmol, 7.90 g) was added in 5 portions over a 20 minute period. After an additional hour, the reaction was quenched as follows: toluene (200 ml, chilled to 0°C) was added; then sodium bisulfite (14 g/ 60 ml, chilled to 0°C) in a portionwise manner. Slow quenching is desirable because the reduction of excess permanganate is exothermic. The mixture was stirred at 0°C until no purple color remained, then washed with a cooled (0°C) 1N NaOH solution (200 ml). The organic layer was separated, dried over MgSO₄, concentrated, and subjected to chromatography, yielding the title compound as a yellow solid (3.58 g, 95%).

### Step 4: Preparation of 5-(2-fluorophenyl)-4-[4-(methylsulfonyl)phenyl]-2-(trifluoromethyl)-1H-imidazole

The title compound was prepared from the dione in the previous step (11 mmol, 3.43 g) using the method of Example 59, step 5, except that the reaction was at reflux 8 hours. Purification consisted of chromatography [hexane:ethyl acetate (2:1), then methylene chloride/ethyl acetate (9:1)], recrystallization from ethyl acetate/hexane, and drying by concentration from acetone solution, affording a white solid (508 mg, 12%). Mass spectrum (EI, M/e): 384. Anal. Calc'd. for C₁₇H₁₂F₄N₂O₂S: C, 53.13; H, 3.15; N, 7.29. Found: C, 53.01; H, 3.04; N, 6.76.

### Example 62

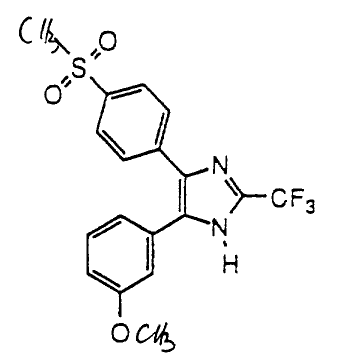

### 5-(3-Methoxyphenyl)-4- [4-(methylsulfonyl)phenyl]-2-(trifluoromethyl)-1H-imidazole

### Step 1: Preparation (3-methoxyphenyl)methyltriphenylphosphonium chloride

The title compound was prepared from (80 mmol, 11.6 ml) of 3-methoxybenzyl chloride by the method of Example 64, step 1, except that the reaction was heated 3 days at reflux, affording the title compound (21.6 g, 65%).

### Step 2: Preparation of 1-methoxy-3-[2-[4-(methylsulfonyl)phenyl]ethylenyl]benzene

The title compound was prepared by method of Example 64, step 2. From 20 mmol phosphonium salt (8.36 g) was obtained 5.47 g (95%) of the title compound.

### Step 3: Preparation of 1-(3-methoxyphenyl)-2-[4-(methylsulfonyl)phenyl]ethane-1,2-dione

The title compound was obtained from 1-methoxy-3-[2-(4-(methylsulfonyl)phenyl]ethylenyl]benzene (Step 2) (18.4 mmol, 5.29 g), using the method of Example 64, step 3, and affording the desired dione as a yellow solid (1.55 g, 27%).

### Step 4: Preparation of 5-(3-methoxyphenyl)-4-[4-(methylsulfonyl)phenyl]-2-(trifluoromethyl)-1H-imidazole

The title compound was prepared from the dione in the previous step (4.9 mmol, 1.55 g) using the method of Example 59, step 5, except that the reaction was at reflux for 5 hours. Purification consisted of chromatography (9:1 methylene chloride:ethyl acetate), followed by recrystallization from ethyl acetate/hexane, affording a white solid (676 mg, 35%) : mp (DSC) = 202-208 °C. Anal. Calc'd. for C₁₈H₁₅F₃N₂O₃S•1/3 H₂O: C, 53.73; H, 3.92; N, 6.96. Found: C, 53.78; H, 3.63; N, 6.45.

### Example 63

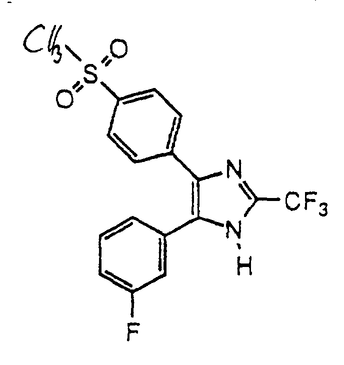

### 5-(3-Fluorophenyl)-4-[4-(methylsulfonyl)phenyl]-2-(trifluoromethyl)-1H-imidazole

### Step 1: Preparation (3-fluorophenyl)methyltriphenylphosphonium bromide

The title compound was prepared from (80 mmol, 11.6 ml) of 3-fluorobenzyl bromide by the method of Example 64, step 1, except that the reaction was stirred 3 days at ambient, affording the title compound (27.1 g, 76%).

### Step 2: Preparation of 1-fluoro-3-[2-[4-(methylsulfonyl)phenyl]ethylenyl]benzene

The title compound was prepared by method of Example 64, step 2. From 20 mmol phosphonium salt (8.80 g) was obtained 2.68 g (48%) of the title compound.

### Step 3: Preparation of 1-(3-fluorophenyl)-2-[4-(methylsulfonyl)phenyl]ethane-1,2-dione

The title compound was obtained from 1-fluoro-3-[2-[4-(methylsulfonyl)phenyl]ethylenyl]benzene (Step 2) (9.71 mmol, 2.68 g), using the method of Example 64, step 3, and affording the desired dione as a yellow solid (770 mg, 26%).

### Step 4: Preparation of 5-(3-fluorophenyl)-4-[4-(methylsulfonyl)phenyl]-2-(trifluoromethyl)-1H-imidazole

The title compound was prepared from the dione in the previous step (2.5 mmol, 770 mg) using the method of Example 59, step 5, except that the reaction was at reflux 5 hours. Purification consisted of chromatography (20:1 methylene chloride:ethyl acetate), followed by recrystallization from ethyl acetate/hexane, affording a white solid (377 mg, 39%): Mass spectrum (EI, M/e): 384. mp (DSC)= 225-229°C. Anal. Calc'd. for C₁₇H₁₂F₄N₂O₂S: C, 53.13; H, 3.15; N, 7.29. Found: C, 52.98; H, 3.01; N, 7.04.

### Example 64

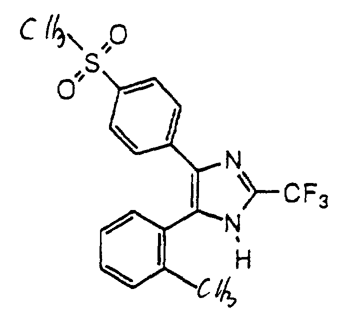

### 5- (2-Methylphenyl)-4-[4-(methylsulfonyl)phenyl]-2-(trifluoromethyl)-1H-imidazole

### Step 1: Preparation (2-methylphenyl)methyltriphenylphosphonium bromide

The title compound was prepared from (105 mmol, 14.1 ml) of 2-fluorobenzyl bromide by the method of Example 64, step 1, except that the reaction was stirred 3 days at ambient, affording the title compound (39.8 g, 85%).

### Step 2: Preparation of 1-methyl-2-[2-[4-(methylsulfonyl)phenyl]ethylenyl]benzene

The title compound was prepared by method of Example 64, step 2. From 40 mmol phosphonium salt (17.9 g) was obtained 10.5 g (96%) of the title compound.

### Step 3: Preparation of 1-(2-methylphenyl)-2-[4-(methylsulfonyl)phenyl]ethane-1,2-dione

The title compound was obtained from 1-methyl-2-[2-[4-(methylsulfonyl)phenyl]ethylenyl]benzene (Step 2) (38.8 mmol, 10.5 g), using the method of Example 64, step 3, and affording the desired dione as a yellow solid (3.50 mg, 28%).

### Step 4: Preparation of 5-(2-methylphenyl)-4-[4-(methylsulfonyl)phenyl]-2-(trifluoromethyl)-1H-imidazole

The title compound was prepared from the dione in the previous step (11 mmol, 10.5 g) using the method of Example 59, step 5, except that the reaction was at reflux 6 hours. Purification consisted of chromatography (20:1 methylene chloride:ethyl acetate), followed by recrystallization from ethyl acetate/hexane, affording a white solid (299 mg, 39%). mp (DSC) 180-190°C. Anal. Calc'd. for C₁₈H₁₅F₃N₂O₂S•(1/2 H₂O): C, 55.52; H, 4.14; N, 7.19. Found: C, 55.67; H, 3.89; N, 6.60.

### Example 65

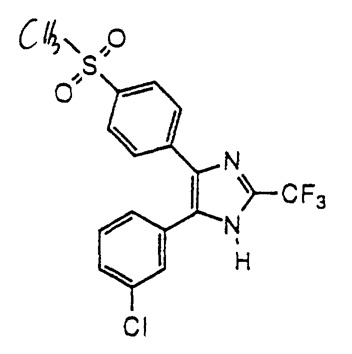

### 5-(3-Chlorophenyl)-4-[4-(methylsulfonyl)phenyl]-2-(trifluoromethyl)-1H-imidazole

### Step 1: Preparation (3-chlorophenyl)methy]triphenylphosphonium bromide

The title compound was prepared from 3-chlorobenzyl chloride (100 mnol, 12.7 ml) by the method of Example 64, step 1, affording after three days of reflux the desired white solid (9.03 g, 20%).

### Step 2: Preparation of 1-chloro-3-[2-[4-(methylsulfonyl)phenyl]ethylenyl]benzene

The title compound was prepared by method of Example 64, step 2. From 21 mmol phosphonium salt (8.92 g) was obtained 4.72 g (76%) of the title compound.

### Step 3: Preparation of 1-(3-chlorophenyl)-2-[4-(methylsulfonyl)phenyl]ethane-1,2-dione

The title compound was obtained from the product of step .2 (15.2 mmol, 4.44 g), using the method of Example 64, step 3, and affording the desired dione as a yellow solid (2.90 g, 60%).

### Step 4: Preparation of 5-(3-chlorophenyl)-4-[4-(methylsulfonyl)phenyl]-2-(trifluoromethyl)-1H-imidazole

The title compound was prepared from the dione in the previous step (9.0 mmol, 2.9 g) using the method of Example 59, step 5, except that the reaction was at reflux 6 hours. Purification consisted of chromatography (20:1 methylene chloride:ethyl acetate), followed by recrystallization twice from ethyl acetate/hexane, affording a white solid (730 mg, 20%). mp (DSC) 224-226°C. Mass spectrum (EI, M/e): 400. Anal. Calc'd. for C₁₇H₁₂ClF₃N₂O₂S: C, 50.94; H, 3.02; N, 6.99. Found: C, 50.81; H, 3.03; N, 6.84.

### Example 66

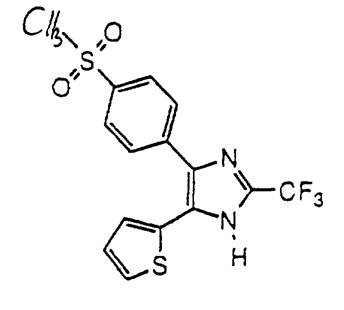

### 4-[4-(Methylsulfonyl)phenyl]-5- (2-thiophenyl)-2-(trifluoromethyl)-1H-imidazole

### Step 1: Preparation of [4-(methylsulfonyl)phenyl]ethylenyl]-2-thiophene

The title compound was prepared by the method of Example 69, step 3. From 5.2 mmol (0.48 ml) of thiophene-2-carboxaldehyde, 0.67 g (49%) of the desired olefin was obtained as a yellow solid.

### Step 2: Preparation of 1-[4-(methylsulfonyl)phenyl]-2-(2-thiophenyl)ethane-1,2-dione

The olefin from the previous step (2.5 mmol, 0.67 g) was transformed by the method of Example 64, step 3 into the title compound, a yellow semisolid (357 mg, 44%).

### Step 3: Preparation of 4-[4-(methylsulfonyl)phenyl]-5-(2-thiophenyl)-2-(trifluoromethyl)-1H-imidazole

The dione (1.1 mmol, 325 mg) was convened to final product by the method of Example 59, step 5, affording, after ethyl acetate/hexane recrystallization, a tan solid (78 mg): mp (DSC) = 95-97°C. Mass spectrum (EI, M/e): 372. Anal. Calc'd. for C₁₅H₁₁F₃N₂O₂S₂: C, 48.38; H, 2.98; N, 7.52. Found: C, 48.30; H, 2.99; N, 7.02.

### Example 67

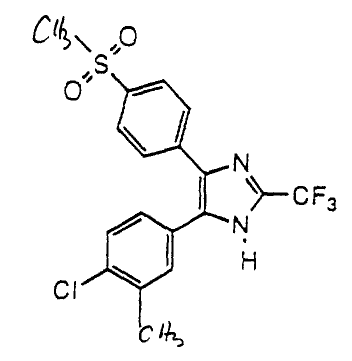

### 5-(4-Chloro-3-methylphenyl)-4-[4-(methylsulfonyl)phenyl]-2-(trifluoromethyl)-1H-imidazole

### Step 1: Preparation of 4-(methylsulfonyl)phenylmethyl triphenylphosphonium chloride

4-(Methylsulfonyl)benzyl chloride (Transworld Chemical) (121 mmol, 24.7 g) and triphenylphosphine (130 mmol, 34.1 g) were heated at reflux in toluene (125 ml) with stirring for 56 hours. The reaction was cooled, and the title compound was collect by filtration (48.0 g, 85%).

### Step 2 : Preparation of 4-chloro-3-methylbenzaldehyde

4-Chloro-3-methylbenzoic acid, methyl ester (Lancaster) (29 mmol, 5.40 g) was dissolved in methylene chloride (30 ml) and cooled to 0°C. Diisobutylaluminum hydride (1 M in toluene, 64 ml) was added slowly (over ca. 10 minutes). One hour after complete addition, the reaction was quenched with methanol (64 ml), then poured into ether (500 ml). This mixture was stirred at room temperature 1 hour, filtered through silica gel and Celite®, eluting with addition ether. Concentration afforded 4-chloro-3-methylbenzyl alcohol (23 mmol, 3.53 g) as a solid, which was diluted with methylene chloride (46 ml), combined with Celite® (3.5 g) and MgSO₄ (1.5 g), and cooled to 0°C. Pyridinium chlorochromate (45 mmol, 9.67 g) was added over 2-3 minutes. In 1 hour's time, the reaction mixture was filtered through silica and concentrated to afford 4-chloro-3-methylbenzaldehyde as a waxy solid (2.10 g, 47% overall), which was used in the next step.

### Step 3: Preparation of 1-chloro-2-methyl-4-[2-[4-(methylsulfonyl)phenyl]ethylenyl]benzene

The aldehyde from step 2 (11 mmol, 1.69 g) was reacted with the phosphonium chloride from step 1, under the reaction conditions established in Example 69, step 3, affording the title compound as a solid (3.09 g, ∼quant.), which was used in the next step.

### Step 4: Preparation of 1-(4-chloro-3-methylphenyl)-2-[4-(methylsulfonyl)phenyl]ethane-1,2-dione

The title compound was obtained from the product of step 3 (10 mmol, 3.1 g), using the method of Example 64, step 3, and affording the desired dione as a yellow solid (0.63 g, 19%).

### Step 5: Preparation of 5-(4-chloro-3-methylphenyl)-4-[4-(methylsulfonyl)phenyl]-2-(trifluoromethyl)-1H-imidazole

The title compound was prepared from the dione in the previous step (1.86 mmol, 625 mg) using the method of Example 59, step 5, except that the reaction was at reflux 6 hours. Purification consisted of chromatography (20:1 methylene chloride:ethyl acetate), followed by recrystallization twice from ethyl acetate/hexane, affording a white solid (344 mg, 45%): mp (DSC) = 261-264°C. Anal. Calc'd. for C₁₈H₁₄ClF₃N₂O₂S: C, 52.12; H, 3.40; N, 6.75. Found: C, 52.50; H, 3.46; N, 6.33.

### Example 68

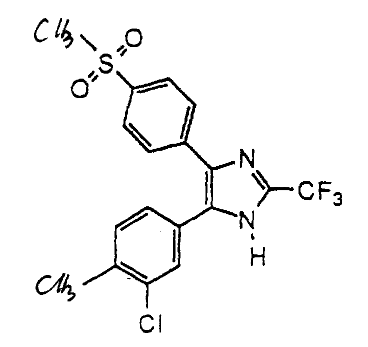

### 5-(3-Chloro-4-methylphenyl)-4-[4-(methylsulfonyl)phenyl]-2-(trifluoromethyl)-1H- imidazole

### Step 1 : Preparation of 3-chloro-4-methylbenzaldehyde

3-Chloro-4-methylbenzonitrile (12 mmol, 1.82 g) was dissolved in methylene chloride (12 ml) and cooled to 0°C. Diisobutylaluminum hydride (1 M in toluene, 13 ml) was added over several minutes, and the ice bath was removed. After 2.5 hours, the reaction was quenched at 0°C with acetaldehyde (4 ml), then after 5 minutes, poured into ice-cold 5% HCl (100 ml). The product was extracted using methylene chloride (150 ml) and the organic layer was dried using magnesium sulfate. Filtration through silica, followed by concentration afforded the title compound, an oil (1.61, 88%), which was used in the next step.

### Step 2: Preparation of 1-chloro-2-methyl-5-[2-[4-(methylsulfonyl)phenyl]ethylenyl]benzene

The aldehyde from step 1 (10 mmol, 1.61 g) was reacted with the phosphonium chloride from Example 71, step 1, under the reaction conditions established in Example 69, step 3, affording the title compound as a solid (2.70 g, 88%.), which was used in the next step.

### Step 3: Preparation of 1-(3-chloro-4-methylphenyl)-2-[4-(methylsulfonyl)phenyl]ethane-1,2-dione

The title compound was obtained from the compound of step 2 (8.8 mmol, 2.7 g), using the method of Example 64, step 3, and affording the desired dione as a yellow solid (1.90 g, 64%).

### Step 4: Preparation of 5-(3-chloro-4-methylphenyl)-4-[4-(methylsulfonyl)phenyl]-2-(trifluoromethyl)-1H-imidazole

The title compound was prepared from the dione in the previous step (5.6 mmol, 1.90 g) using the method of Example 59, step 5, except that the reaction was at reflux 5 hours. Purification consisted of chromatography (20:1 methylene chloride:ethyl acetate), followed by recrystallization twice from acetone/hexane, affording a white solid (359 mg, 15%) : mp (DSC) = 273-275°C. Mass spectrum (EI, M/e) : 414. Anal. Calc'd. for C₁₈H₁₄ClF₃N₂O₂S: C, 52.12; H, 3.40; N, 6.75. Found: C, 52.32; H, 3.36; N, 6.65.

### Example 69

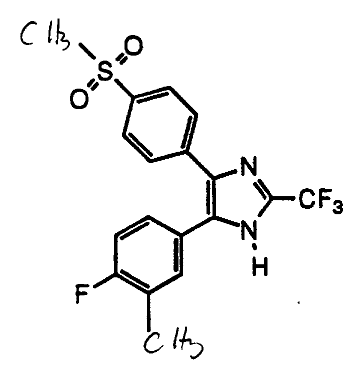

### 5-(4-Fluoro-3-methylphenyl)-4-[4-(methylsulfonyl)phenyl]-2-(trifluoromethyl)-1H-imidazole

### Step 1: Preparation of 4-fluoro-3-methylbenzaldehyde

5-Bromo-2-fluoro-toluene (26 mmol, 5.0 g) was dissolved in tetrahydrofuran (25 ml) and cooled to -78°C. t-Butyllithium (1.7 M in pentane, 31 ml) was added over 10 minutes. After 30 minutes, N,N-dimethylformamide (2.1 ml) was added. The reaction mixture was warmed to ambient over 30 minutes and 6% HCl (100 ml) was added with vigorous swirling. The mixture was extracted with ethyl acetate (200 ml). The organic layer was dried over MgSO₄, then concentrated affording the title compound as an oil (3.6 g, ~quant.).

### Step 2: Preparation of 1-fluoro-2-methvl-4-[2-[4-(methylsulfonyl)phenyl]ethylenyl]benzene

The aldehyde from step 1 (10 mmol, 1.5 g) was reacted with the phosphonium chloride from Example 71, step 1, under the reaction conditions established in Example 69, step 3, affording the title compound as a solid (2.98 g, ∼quant.), which was used in the next step.

### Step 3: Preparation of 1-(4-fluoro-2-methylphenyl)-2-[4-(methylsulfonyl)phenyl]ethane-1,2-dione

The title compound was obtained from the compound of step 2 (10 mmol, 2.98 g), using the method of Example 64, step 3, and affording the desired dione as a yellow solid (2.2 g, 69%).

### Step 4: Preparation of 5-(3-fluoro-2-methylphenyl)-4-[4-(methylsulfonyl)phenyl]-2-(trifluoromethyl)-1H-imidazole

The title compound was prepared from the dione in the previous step (6.9 mmol, 2.2 g) using the method of Example 59, step 5, except that the reaction was at reflux 6 hours. Purification consisted of chromatography (20:1 methylene chloride:ethyl acetate), followed by recrystallization twice from acetone/hexane, affording a white solid (204 mg, 7%) : Anal. Calc'd. for C₁₈H₁₄F₄N₂O₂S: C, 54.27; H, 3.54; N, 7.03. Found: C, 54.33; H, 3.49; N, 6.39.

### Example 70

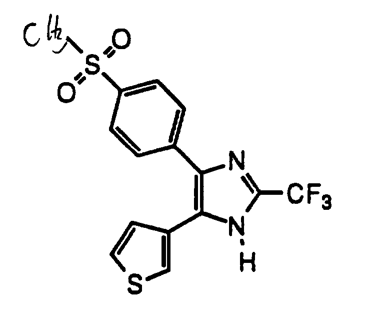

### 4-[4-(Methylsulfonyl)phenyl]-5-(3-thiophenyl)-2-(trifluoromethyl)-1H-imidazole

### Step 1: Preparation of [4-(methylsulfonyl)phenyl]ethylenyl]-3-thiophene

The title compound was prepared by the method of Example 69, step 3 using the phosphonium chloride from Example 71, step 1. From 10.5 mmol (0.92 ml) of thiophene-3-carboxaldehyde, 2.64 g (~quant) of the desired olefin was obtained as a solid.

### Step 2: Preparation of 1-[4-(methylsylfonyl)phenyl]-2-(3-thiophenyl)ethane-1,2-dione

The olefin from the previous step (10 mmol, 2.64 g) was transformed by the method of Example 64, step 3 into the title compound, a yellow semisolid (1.25g, 42%).

### Step 3: Preparation of 4-[4-(methylsulfonyl)phenyl]-5-(3-thiophenyl)-2-(trifluoromethyl)-1H-imidazole

The dione (4.25 mmol, 1.25 g) was converted to final product by the method of Example 59, -step 5, affording, after recrystallization twice from acetone/hexane, 408 mg (26%). mp (DSC)= 230-232°C. Mass spectrum (EI, M/e): 372. Anal. Calc'd. for C₁₅H₁₁F₃N₂O₂S₂: C, 48.38; H, 2.98; N, 7.52. Found: C, 48.49; H, 2.93; N, 7.34.

### Example 71

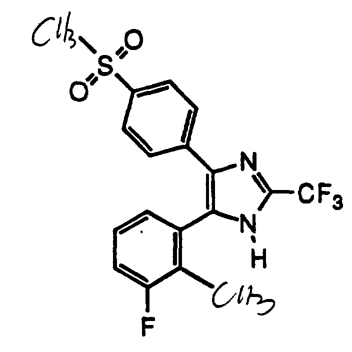

### 5-(3-Fluoro-2-methylphenyl)-4-[4-(methylsulfonyl)phenyl]-2-(trifluoromethyl)-1H-imidazole

### Step 1: Preparation of 3-fluoro-2-methyl-3-[2-[4-(methylsulfonyl)phenyl]ethylenyl]benzene

The 3-fluoro-2-methylbenzaldehyde (10 mmol, 1.2 g) was reacted with the phosphonium chloride from Example 71, step 1, under the reaction conditions established in Example 69, step 3, affording the title compound as a solid (2.9 g, ∼quant.), which was used in the next step.

### Step 2: Preparation of 1-(3-fluoro-2-methylphenyl)-2-[4-(methylsulfonyl)phenyl]ethane-1,2-dione

The title compound was obtained from the compound of step 2 (10 mmol, 2.9 g), using the method of Example 64, step 3, and affording the desired dione as a yellow solid (1.9 g, 59%).

### Step 3: Preparation of 5-(3-fluoro-2-methylphenyl)-4-[4-(methylsulfonyl)phenyl]-2-(trifluoromethyl)-1H-imidazole

The title compound was prepared from the dione in the previous step (6.9 mmol, 2.2 g) using the method of Example 59, step 5, except that the reaction was at reflux 6 hours. Purification consisted of chromatography (20:1 methylene chloride:ethyl acetate), followed by recrystallization twice from acetone/hexane, which gave a white solid (184 mg, 6%). mp (DSC) 192-196°C. Anal. Calc'd. for C₁₈H₁₄F₄N₂O₂S: C, 54.27; H, 3.54; N, 7.03. Found: C, 54.23; H, 3.18; N, 6.76.

### Example 72

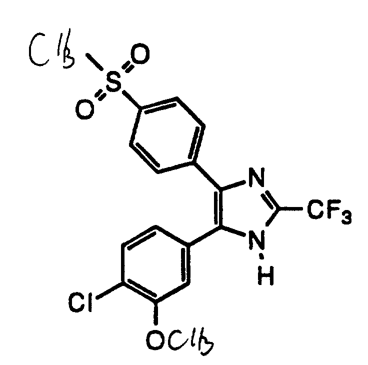

### 5-(4-Chloro-3-methoxyphenyl)-4-[4-(methylsulfonyl)phenyl]-2- (trifluoromethyl)-1H-imidazole

### Step 1: Preparation of 2-chloro-5-methylanisole

2-Chloro-5-methylphenol (50 mmol, 7.13 g), NaOH pellets (60 mmol, 2.4 g), and methanol (50 ml) were stirred together until a homogeneous solution was obtained. The flask was cooled to 0°C, and methyl iodide (60 mmol, 3.77 ml) was added. The reaction was warmed over 2 hours to ambient and stirred for 16 hours. Reaction was still incomplete by thin layer chromatography; additional NaOH (∼300 mg) and CH₃I (∼5 ml) was introduced, and stirring continued for another 24 hours. TLC again indicated incomplete conversion, so additional time and reagents were again applied. The mixture was at length concentrated, diluted with water (50 ml), and extracted with methylene chloride (2 X 25 ml). The combined organic layer was dried (MgSO₄), filtered through silica, and concentrated to afford the title compound as an oil (5.09 g, 65%).

### Step 2: Preparation (4-chloro-3-methoxyphenyl)methyltriphenylphosphonium bromide

The compound from step 1 (33 mmol, 5.1 g) was combined with N-bromosuccinimide (33 mmol, 5.1 g) and benzoyl peroxide (1 mmol, 240 mg) in CCl₄ (40 ml) and heated for 3 hours at reflux. The mixture was diluted with additional CCl₄ (∼60 ml), filtered trough Celite®, and washed with sodium bisulfite solution (1 g/10 ml water). The organic layer was dried (MgSO₄), concentrated, and azeotroped with toluene (∼20 ml). The residue was dissolved in toluene (50 ml) and triphenylphosphine (32 mmol, 8.54 g) was added. After 3 days, the product was collected by filtration (1.95 g, 12%).

### Step 3: Preparation of 1-chloro-2-methoxy-4-[2-[4-(methylsulfonyl)phenyl]ethylenyl]benzene

The title compound was prepared by method of Example 64, step 2. From 5.5 mmol phosphonium salt (2.7 g) was obtained 1.40 g (80%) of the title compound.

### Step 4: Preparation of 1-(4-chloro-3-methoxyphenyl)-2-[4-(methylsulfonyl)phenyl]ethane-1,2-dione

The title compound was obtained from the compound of step 3 (4.33 mmol, 1.39 g), using the method of Example 64, step 3, and affording the desired dione as a yellow solid (580 mg, 38%).

### Step 5: Preparation of 5-(4-chloro-3-methoxyphenyl)-4-[4-(methylsulfonyl)phenyl]-2-(trifluoromethyl)-1H-imidazole

The title compound was prepared from the dione in the previous step (1.65 mmol, 580 mg) using the method of Example 59, step 5, except that the reaction was at reflux for 5 hours. Purification, which consisted of chromatography (20:1 methylene chloride:ethyl acetate), followed by recrystallization twice from ethyl acetate/hexane, afforded a white solid (84 mg, 12%). Mass spectrum (EI, M/e): 430. mp (DSC)= 233-234°C. Anal. Calc'd. for C₁₈H₁₄ClF₃N₂O₃S: C, 50.18; H, 3.28; N, 6.50. Found: C, 50.33; H, 3.63; N, 5.76.

### Example 73

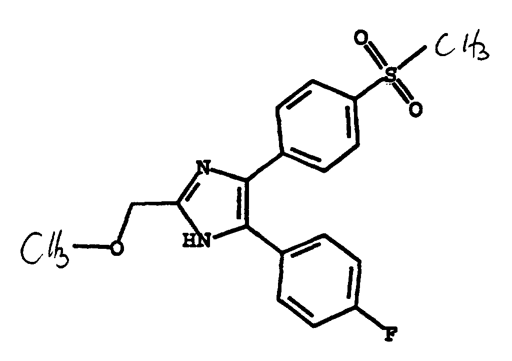

### 5-(4-Fluorophenyl)-2-(methoxymethyl)-4-[4-(methylsulfonyl)-phenyl]-1H-imidazole

### Step 1: Preparation of 1-(1-ethoxyethyl)-4-(4-fluorophenyl)-5-[4-(methylthio)phenyl]-1H-imidazol-2-yl]-1H-imidazole-2-methanol

A solution of 5-(4-fluorophenyl)-4-(4-methylthiophenyl)-1-(1-methyl-1-ethoxymethyl)-1H-imidazole (Example 9, step 2) (4.19 g; 11.75 mmol) and tetramethylethylene diamine (TMEDA) (4.1 g mg; 13.39 mmol) in tetrahydrofuran (30 mL) was cooled to -70°C under an argon atmosphere. n-Butyllithium (5.1 mL, 1.6 M in hexane, 12.9 mmol) was added and the solution was stirred at -78°C for 15 minutes. Dimethylformamide (859 mg; 0.90 mL) was added and the solution was warmed to 0°C. The reaction was quenched by the addition of a saturated NaHCO₃ solution and extracted with diethyl ether. The organic extracts were dried (MgSO₄), filtered and concentrated *in vacuo*. The crude compound was redissolved in 23.5 ml of methanol and to this solution was added sodium borohydride (789.6 mg; 23.4 mmol). After stirring for 1 hour at 25°C, the reaction was quenched with a 1% aqueous hydrochloric acid solution till evidence of gas evolution ceased. The solution was extracted with methylene chloride, dried (MgSO₄), filtered and concentrated *in vacuo.* The residue was purified by chromatography (SiO₂; ethyl acetate/hexane) to yield ca. 4 g of the desired compound.

### Step 2: Preparation of 5-(4-fluorophenyl)-2-(methoxymethyl)-4-[4-(methylsulfonyl)-phenyl]-1H-imidazole

To a solution of 1-(1-ethoxyethyl)-4-(4-fluorophenyl)-5-[4-(methylthio)phenyl]-1H-imidazol-2-yl]-1H-imidazole-2-methanol (Step 1) (549.3 mg; 1.42 mmol) in 5.6 ml of THF, at 25°C, was added 34.0 mg (1.42 mmol) of dry sodium hydride. After all the solids were dissolved. 88 µl of methyl iodide (201.55 g; 1.42 mmol) was added. After stirring at 25°C for two hours, the reaction was diluted with 25 ml of ether and this solution was washed with 25 ml of water. The organic extracts were dried (MgSO₄), evaporated to dryness under reduced pressure, and purified with flash chromatography (SiO₂; 90/10 toluene/methanol) to give 334 mg of material containing the desired product. This material was dissolved in 16 ml of methanol/THF (1.6:1). Oxone® (900 mg) was dissolved in 4 ml of water and added to this solution all at once. After stirring the reaction at 25°C for 2 hours, the reaction was poured into water and extracted with methylene chloride. The organic extracts were dried (MgSO₄) and evaporated under reduced pressure to give a white solid. Purification by preparative thin layer chromatography (SiO₂; 90/10 toluene/ethyl acetate) gave 33 mg of the desired sulfone: mp 169 °C. Anal. Calc'd. for C₁₈H₁₇FN₂O₃S: C, 59.99; H, 4.75; N 7.77. Found: C, 59.47; H, 4.66; N, 7.32.

### Example 74

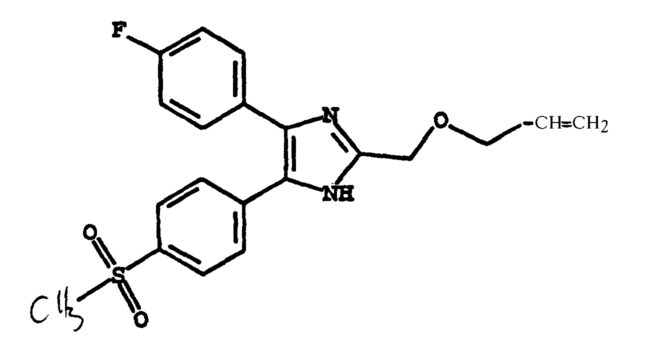

### 4-(4-Fluorophenyl) -5- [4-(methylsulfonyl)phenyl]-2-[(2-propenyloxy)methyl]-1H-imidazole

To a solution of 1-(1-ethoxyethyl)-4-(4-fluorophenyl)-5- [4- (methylthio)phenyl]-1H-imidazol-2-yl]-1H-imidazole-2-methanol (Example 79, Step 1) (200 mg; 0.52 mmol) in 2 ml of DMF, at 25°C, was added 20.0 mg (0.52 mmol) of sodium hydride (60% mineral oil dispersion). After stirring at 25°C for 15-20 minutes, 44 µl of allyl bromide (62.9 mg; 0.52 mmol) was added. After stirring at 25°C for 2 hours, the reaction was diluted with 40 ml of ethyl acetate and washed with 3 x 30 ml of brine. The organic extracts were dried (MgSO₄), and evaporated to dryness under reduced pressure to provide 184 mg of a oil, which was purified by flash chromatography (SiO₂; 25% ethyl acetate/toluene) to give 96 mg of the desired intermediate. This material was dissolved in 8 ml of methanol/THF (1.6:1). Oxone® (450 mg) was dissolved in 2 ml of water and added to this solution all at once. After stirring the reaction at 25°C for 1.5 hours, the reaction was poured into 50 ml of methylene chloride and extracted with 2 x 50 ml of water. The organic extracts were dried (MgSO₄) and evaporated under reduced pressure to give 82.5 mg of a white solid which was further purified by preparative thin layer chromatography (SiO₂; 75/25 ethyl acetate/toluene) to give 56.4 mg of the desired sulfone: Anal. Calc'd. for C₂₀H₁₉FN₂O₃S+0.5 mol H₂O: C, 60.75; H, 5.10; N 7.08; S, 8.11. Found: C, 60.53; H, 4.83; N, 6.93; S, 8.11.

### Example 75

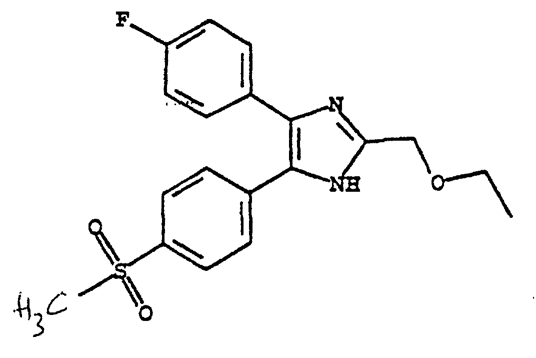

### 2-(Ethoxymethyl)-4-(4-fluorophenyl)-5-[4-(methylsulfonyl)phenyl]-1H-imadazole

To a solution of 1-(1-ethoxyethyl)-4-(4-fluorophenyl)-5-[4-(methylthio)phenyl]-1H-imidazol-2-yl]-1H-imidazole-2-methanol (Example 79, Step 1) 200 mg; 0.52 mmol) in 2 ml of DMF, at 25°C, was added 20.0 mg (0.52 mmol) of sodium hydride (60% mineral oil dispersion). After stirring at 25°C for 15-20 minutes, 41 µl of ethyl iodide (81.0 mg; 0.52 mmol) was added. After stirring at 25°C for 22 hours, the reaction was diluted with 40 ml of ethyl acetate and washed with 3 x 30 ml of brine. The organic extracts were dried (MgSO₄), and evaporated to dryness under reduced pressure to provide 151 mg of a orange oil, which was purified by flash chromatography (SiO₂; 25% ethyl acetate/toluene) to give 40.6 mg of the desired intermediate. This material was dissolved in 8 ml of methanol/THF (1.6:1). Oxone® (450 mg) was dissolved in 2 ml of water and added to this solution all at once. After stirring the reaction at 25°C for 1.5 hours, the reaction was poured into water and extracted with methylene chloride. The organic extracts were dried (MgSO₄) and evaporated under reduced pressure to give 38 mg of a white solid which was purified by preparative thin layer chromatography (SiO₂; 75/25 ethyl acetate/toluene) to give 21.0 mg of the desired sulfone: HRMS, m/e Calc'd. for C₁₉H₁₉N₂O₃SF: 374.1100; measured: 374.1125.

### Example 76

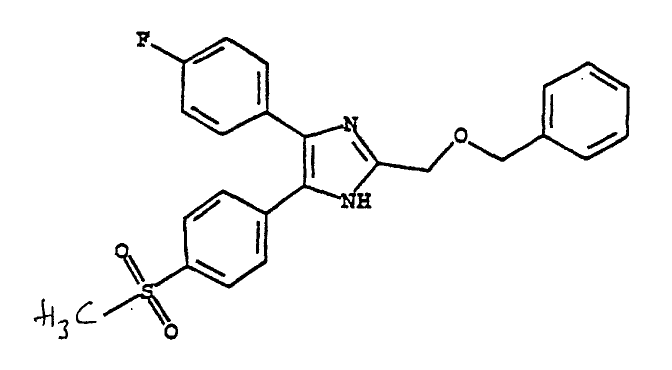

### 4-(4-Fluorophenyl)-5-[4-(methylsulfonyl)phenyl]-2-(phenyl-methoxymethyl)-1H-imidazole

To a solution of 1-(1-ethoxyethyl)-4-(4-fluorophenyl)-5-[4-(methylthio)phenyl]-1H-imidazol-2-yl]-1H-imidazole-2-methanol (Example 79, Step 1) (220 mg; 0.56 mmol) in 2.2 ml of THF, at 25°C, was added 13.4 mg (0.56 mmol) of dry sodium hydride. After stirring at 25°C for 15 minutes, 66 µl of benzylbromide (95.7 mg; 0.56 mmol) was added. After stirring at 25°C for 24 hours, the reaction was diluted with ether and washed with water. The organic extracts were dried (MgSO₄), and evaporated to dryness under reduced pressure to provide 234 mg of material, which was purified by flash chromatography (SiO₂; 25% ethyl acetate/toluene) to give 90 mg of material containing the desired intermediate. This material was dissolved in 8 ml of methanol/THF (1.6:1). Oxone® (450 mg) was dissolved in 2 ml of water and added to this solution all at once. After stirring the reaction at 25°C for 2 hours, the reaction was poured into water and extracted with methylene chloride. The organic extracts were dried (MgSO₄) and evaporated under reduced pressure to give 67 mg of a white solid which was further purified by preparative thin layer chromatography (SiO₂; 50% ethyl acetate/toluene) to give 48 mg of the desired sulfone: Anal. Calc'd. for C₂₄H₂₁FN₂O₃S+0.5 H₂O: C, 64.70; H, 4.98; N 6.29. Found: C, 64.75; H, 4.36; N, 6.00.

### Example 77

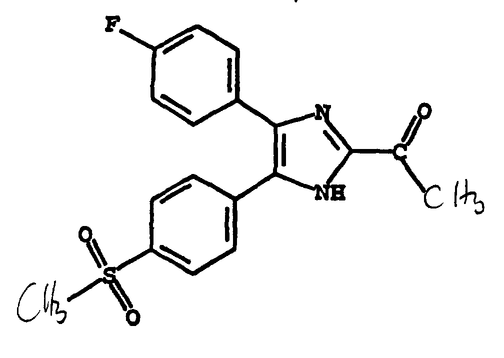

### 1-[4-(4-Fluorophenyl)-5-[4-(methylsulfonyl)phenyl]-1H-imidazol-2-yl] ethanone

To a solution of 1-(1-ethoxyethyl)-4-(4-fluorophenyl)-5-[4-(methyl-thio)phenyl]-1H-imidazole (Example 9, Step 2) (206 mg; 0.53 mmol) and tetramethylethylenediamine (TMEDA) (324 mg; 1.65 mmol) in 3.0 ml of THF at -78°C was added 0.28 ml of a 2.5 M solution of n-butyllithium in hexanes. After stirring for 15 minutes, 66 µl of N,N-dimethylacetamide (62.7 mg; 0.72 mmol) was added. After 30 minutes, the solution was slowly warmed to 25°C, 2 ml of an aqueous solution of sodium bicarbonate was added, followed by dilution with 50 ml of diethyl ether. The solution was washed with 50 ml of a saturated solution of sodium bicarbonate and the organic extracts were dried (MgSO₄) and filtered. The solvent was removed under reduced pressure to give 230 mg of a yellow oil, which was purified by flash chromatography (SiO₂; 25% ethyl acetate/hexane) to yield 86 mg of slightly yellow oil (37%). This material was dissolved in 8 ml of methanol/THF (1.6:1). Oxone® (450 mg) was dissolved in 2 ml of water and this solution was added all at once. After stirring the reaction at 25°C for 2 hours, the reaction was poured into water and extracted with methylene chloride. The organic extracts were dried (MgSO₄) and evaporated under reduced pressure to give a white solid which was purified by preparative thin- layer chromatography (SiO₂; 75% ethyl acetate/toluene) to give 56 mg of the desired sulfone: mp 215-217°C. Anal. Calc'd. for C₁₈H₁₅FN₂O₃S: C, 60.32; H, 4.22; N 7.82. Found: C, 60.09; H, 4.00; N, 7.76.

### Example 78

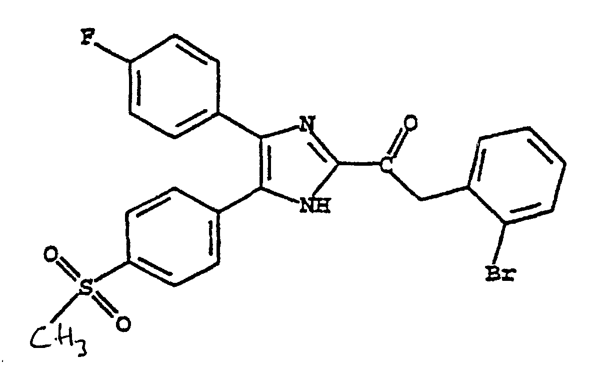

### 2-(2-Bromophenyl)-1-[4-(4-fluorophenyl)-5-[4-(methylsulfonyl)phenyl]-1H-imidazol-2-yl]ethanone

### Step 1: Preparation of 2-bromo-N-methoxy-N-methylbenzene acetamide

To 2 g of 2-bromophenylacetic acid (9.3 mmol), in 33 ml of methylene chloride at 0°C, was added N,O-dimethylhydroxylamine hydrochloride (906.7 mg; 10.23 mmol), 4.26 ml of triethylamine (3.09 g; 30.6 mmol) and 2-chloromethylpyridinium iodide (2.6 g; 10.23 mmol). After stirring at 25°C for 12 hours, the reaction mixture was poured into 250 ml of methylene chloride, then this solution was extracted with 150 ml of 1N hydrochloric acid, 150 ml saturated, aqueous solution of sodium bicarbonate, and 150 ml of brine. The organic extracts were dried (MgSO₄), filtered and evaporated under reduced pressure to give 1.98 g of a light pink oil, which was purified by flash chromatography (SiO₂; 25% ethyl acetate/ hexane) to give 849 mg of 2-bromo-N-methoxy-N-methylbenzene acetamide as an oil: Anal. Calc'd. for C₁₀H₁₂BrNO₂ : C, 46.53; H, 4.69; N 5.43; Br 30.96. Found: C, 46.45; H, 4.71; N, 5.34; Br, 30.66.

### Step 2: Preparation of 2-(2-bromophenyl)-1-[4-(4-fluorophenyl)-5-[4-(methylsulfonyl)phenyl]-1H-imidazol-2-yl]ethanone

To a solution of 1-(1-ethoxyethyl)-4-(4-fluorophenyl)-5-[4-(methyl-thio)phenyl]-1H-imidazole (Example 83 Step 1) (658.9 mg; 1.70 mmol) and TMEDA (639 mg; 5.4 mmol) in 10 ml of THF at -78°C was added 0.91 ml of a 2.5 M n-butyllithium in hexanes. After stirring for 15 minutes, a solution of 2-bromo-N-methoxy-N-methylbenzene acetamide (step 1) (562.2 g; 2.29 mmol) in 1 ml of THF was added. The solution was warmed to 25°C over 1 hour. The reaction was diluted with diethyl ether and washed with a saturated, aqueous, solution of sodium bicarbonate. The organic extracts were dried (MgSO₄), filtered and evaporated under reduced pressure to give a yellow oil, which was purified by flash chromatography (SiO₂; 50% ethyl acetate/hexane) to give 56 mg of a white foam. The foam (56 mg) was dissolved in 8 ml of methanol/THF (1.6:1). Oxone® (450 mg) was dissolved in 2 ml of water and this solution was added all at once. After stirring the reaction at 25°C for 1.5 hours, the reaction was poured into water and extracted with methylene chloride. The organic extracts were dried (MgSO₄) and evaporated under reduced pressure to give a white solid which was purified by preparative thin layer chromatography (SiO2; 75% ethyl acetate/toluene) to give 38 mg of the desired sulfone: Anal. Calc'd. for C₂₄H₁₈FN₂O₃SBr: C, 56.15; H, 3.53; N 5.46, Br, 15.56. Found: C, 56.23; H, 3.17; N, 5.26; Br, 15.74.

### Example 79

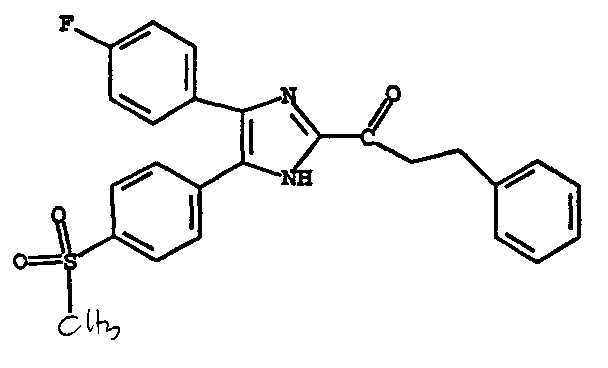

### 1-(4-(4-Fluorophenyl)-5-[4-methylsulfonyl)phenyl]-1H-imidazol-2-yl]-3-phenyl-1-propanone

### Step 1: Preparation of N-methoxy-N-methylbenzenepropaneamide

To 1.39 g of dihydrocinnamic acid (9.3 mmol), in 33 ml of methylene chloride at 0°C, was added N,O-dimethylhydroxylamine hydrochloride (906.7 mg; 10.23 mmol), 4.26 ml of triethylamine (3.09 g; 30.6 mmol) and 2-chloromethylpyridinium iodide (2.6 g; 10.23 mmol). After stirring at 25°C for 12 hours, the reaction mixture was poured into 250 ml of methylene chloride, then this solution was extracted with 150 ml of 1N hydrochloric acid, ml saturated, aqueous, solution of sodium bicarbonate with added sodium thiosulfate and 150 ml of brine. The organic extracts were dried (MgSO₄), filtered and evaporated under reduced pressure to give 1.3208 g of a light yellow oil, which was purified by flash chromatography (SiO₂; 50% ethyl acetate/hexane) to give 849 mg of N-methoxy-N-methylbenzenepropaneamide as an oil: Anal. Calc'd. for C₁₁H₁₅NO₂: C, 68.37; H, 7.82; N 7.25. Found: C, 68.60; H, 7.84; N,7.24.

### Step 2: Preparation of 1-(4-(4-fluorophenyl)-5-[4-methylsulfonyl)phenyl]-1H-imidazol-2-yl]-3-phenyl-1-propanone

To a solution of 1-(1-ethoxyethyl)-4-(4-fluorophenyl)-5-[4-(methyl-thio)phenyl]-1H-imidazole (Example 83 Step 1) (503 mg; 1.30 mmol) and TMEDA (485 mg; 4.1 mmol) in 7.42 ml of THF at -78°C was added 0.91 ml of a 2.5 M n-butyllithium in hexane. After stirring for 15 minutes, a solution of N-methoxy-N-methylbenzenepropaneamide (Step 1) (338.1; 1.75 mmol) in 1 ml of THF was added. The solution was warmed to 25°C over a period of one hour. The reaction was diluted with diethyl ether and washed with a saturated, aqueous, solution of sodium bicarbonate. The organic extracts were dried (MgSO₄), filtered and evaporated under reduced pressure to give a yellow oil, which was purified by flash chromatography (SiO₂; 25% ethyl acetate/hexane) to give 515 mg of a colorless oil. The oil (100 mg) was dissolved in 8 ml of methanol/THF (1.6:1). Oxone® (450 mg) was dissolved in 2 ml of water and this solution was added all at once. After stirring the reaction at 25°C for 1.5 hours, the reaction was poured into water and extracted with methylene chloride. The organic extracts were dried (MgSO₄) and evaporated under reduced pressure to give a white solid which was purified by preparative thin layer chromatography (SiO₂; 75% ethyl acetate/ toluene) to give 51.5 mg of the desired sulfone: Mp 227-231°C; Anal. Calc'd. for C₂₅H₂₁FN₂O₃S: C, 66.95; H, 4.72; N 6.25; S, 7.15. Found: C, 67.04; H, 4.58; N, 6.13; S, 7.24.

### Example 80

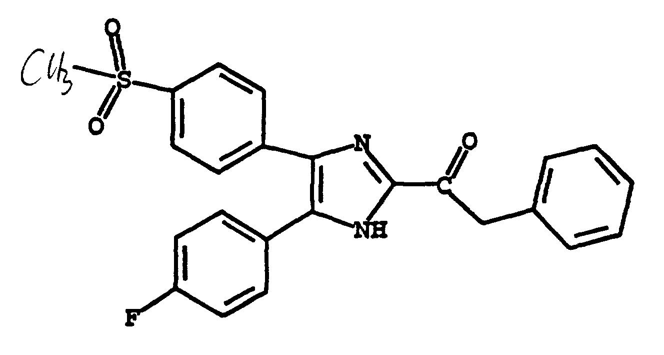

### 1-[5-(4-Fluorophenyl)-4-[4-(methylsulfonyl)phenyl]-1H-imidazol-2-yl]-2-phenylethanone

### Step 1: Preparation of N-methoxy-N-methylbenzeneacetamide

To 1.26 g of phenylacetic acid, in 33 ml of methylene chloride at 0°C, was added N,O-dimethylhydroxylamine hydrochloride (906.7 mg; 10.23 mmol), 4.26 ml of triethylamine (3.09 g; 30.6 mmol) and 2-chloromethylpyridinium iodide (2.6 g; 10.23 mmol). After stirring at 25°C for 12 hours, the reaction mixture was poured into 250 ml of methylene chloride, then this solution was extracted with 150 ml of 1N hydrochloric acid, 150 ml saturated, aqueous, solution of sodium bicarbonate, and 150 ml of brine. The organic extracts were dried (MgSO₄), filtered and evaporated under reduced pressure to give 1.98 g of a oil, which was purified by flash chromatography (SiO₂; 50% ethyl acetate/hexane) to give 1.1334 g of N-methoxy-N-methylbenzeneacetamide as an oil: Anal. Calc'd. for C₁₀H₁₃NO₂ +0.25 H₂O: C, 65.38; H, 6.93; N 7.49. Found: C, 65.67; H, 6.93; N, 7.49.

### Step 2: Preparation of 1-(5-(4-fluorophenyl)-4-[4-(methylsulfonyl)phenyl]-1H-imidazol-2-yl]-2-phenylethanone

To a solution of 1-(1-ethoxyethyl)-4-(4-fluorophenyl)-5-[4-(methyl-thio)phenyl]-1H-imidazole (Example 83 Step 1) (476 mg; 1.23 mmol) and TMEDA (462 mg; 3.97 mmol) in 7.0 ml of THF at -78°C was added 0.66 ml of a 2.5 M n-butyllithium in hexanes. After stirring for 15 minutes, a solution of N-methoxy-N-methylbenzeneacetamide (338.1 mg; 1.75 mmol) in 1 ml of THF was added. The solution was warmed to 25°C over 1 hour. The reaction was diluted with diethyl ether and washed with a saturated, aqueous, solution of sodium bicarbonate. The organic extracts were dried (MgSO₄), filtered and evaporated under reduced pressure to give a yellow oil, which was purified by flash chromatography (SiO₂; 25% ethyl acetate/hexane) to give 355 mg of a light yellow solid. This solid (100 mg) was dissolved in 8 ml of methanol/THF (1.6:1). Oxone® (450 mg) was dissolved in 2 ml of water and this solution was added all at once. After stirring the reaction at 25°C for 1.5 hours, the reaction was poured into water and extracted with methylene chloride. The organic extracts were dried (MgSO₄) and evaporated under reduced pressure to give a white solid which was purified by flash chromatography (SiO₂; 25% ethyl acetate/toluene) to give 63 mg of the desired sulfone: mp 263.2°C; Anal. Calc'd. for C₂₄H₁₉FN₂O₃S: C, 66.35; H, 4.41; N 6.45; S, 7.38. Found: C, 66.43; H, 4.26; N, 6.21; S, 7.12.

### Example 81

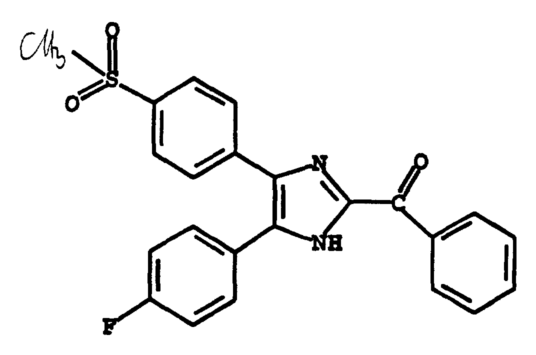

### [5-(4-Fluorophenyl)-4-[4-(methylsulfonyl)phenyl]-1H-imidazol-2-yl]phenylmethanone

### Step 1: Preparation of N-methoxy-N-methylbenzenecarboxamide

To 1.13 g of benzoic acid (9.3 mmol), in 33 ml of methylene chloride at 0°C, was added N,O-dimethylhydroxylamine hydrochloride (906.7 mg; 10.23 mmol), 4.26 ml of triethylamine (3.09 g; 30.6 mmol) and 2-chloromethylpyridinium iodide (2.6 g; 10.23 mmol). After stirring at 25°C for 12 hours, the reaction mixture was poured into 250 ml of methylene chloride, then this solution was extracted with 150 ml of 1N hydrochloric acid, 150 ml saturated, aqueous solution of sodium bicarbonate, and 150 ml of brine. The organic extracts were dried (MgSO₄), filtered and evaporated under reduced pressure to give a oil, which was purified by flash chromatography (SiO₂; 50% ethyl acetate/ hexane) to give 0.872 of N-methoxy-N-methylbenzenecarboxamide as an oil: Anal. Calc'd. for C₉H₁₁NO₂ + 0.25 H₂O: C, 63.70; H, 6.29; N 8.14. Found: C, 63.73; H, 6.83; N, 8.25

### Step 2: Preparation of [5-(4-fluorophenyl)-4-[4-(methylsulfonyl)phenyl]-1H-imidazol-2-yl]phenylmethanone

To a solution of 1-(1-ethoxyethyl)-4-(4-fluorophenyl)-5-[4- (methyl-thio)phenyl] -1H-imidazole (Example 83 Step 1) (374 mg; 0.96 mmol) and TMEDA (362 mg; 3.1 mmol) in 5.48 ml of THF at -78°C was added 0.52 ml of a 2.5 M n-butyllithium in hexanes. After stirring for 15 minutes, a solution of N-methoxy-N-methyl-benzenecarboxamide (Step 1) (214 mg; 1.30 mmol) in 1 ml of THF was added. The solution was warmed to 25°C over a period of one hour. The reaction was diluted with diethyl ether and washed with a saturated, aqueous solution of sodium bicarbonate. The organic extracts were dried (MgSO₄), filtered and evaporated under reduced pressure to give the crude product, which was purified by flash chromatography (SiO₂; 25% ethyl acetate/hexane) to give 294 mg of product. 133 mg of this material was dissolved in 8 ml of methanol/THF (1.6:1). Oxone® (450 mg) was dissolved in 2 ml of water and then this solution was added all at once. After stirring the reaction at 25°C for 1.5 hours, the reaction was poured into water and extracted with methylene chloride. The organic extracts were dried (MgSO₄) and evaporated under reduced pressure to give a white solid which was purified by flash chromatography (SiO₂; 50% ethyl acetate/toluene) to give 49.5 mg of the desired sulfone: mp 264° C (dec.). Anal. Calc'd. for C₂₃H₁₇FN₂O₃S: C, 65.70; H, 4.08; N 6.66; S, 7.63. Found: C, 65.51; H, 4.34; N, 6.40; S, 7.68.

### Example 82

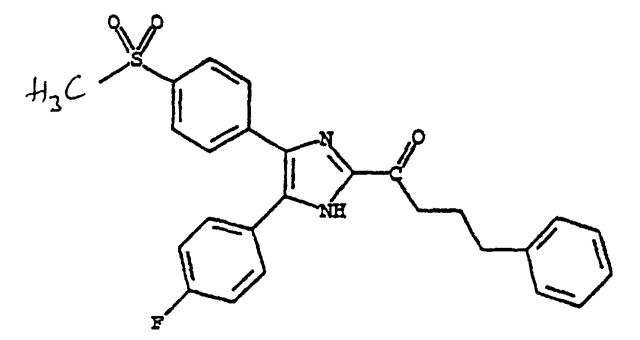

### 1- [5-(4-Fluorophenyl)-4-[4-(methylsulfonyl)phenyl]-1H-imidazol-2-yl]-4-phenyl-1-butanone

### Step 1: Preparation of N-methoxy-N-methylbenzenebutanamide

To 1.52 g of phenylbutyric acid (9.3 mmol), in 33 ml of methylene chloride at 0°C, was added N,O-dimethylhydroxylamine hydrochloride (906.7 mg; 10.23 mmol), 4.26 ml of triethylamine (3.09 g; 30.6 mmol) and 2-chloromethylpyridinium iodide (2.6 g; 10.23 mmol). After stirring at 25°C for 12 hours, the reaction mixture was poured into 250 ml of methylene chloride, then this solution was extracted with 150 ml of 1N hydrochloric acid, 150 ml saturated, aqueous solution of sodium bicarbonate, and 150 ml of brine. The organic extracts were dried (MgSO₄), filtered and evaporated under reduced pressure to give an oil, which was purified by flash chromatography (SiO₂; 50% ethyl acetate/hexane) to give 0.525 g of N-methoxy-N-methylbenzenebutanamide: Anal. Calc'd. for C₁₂H₁₇NO₂ + 0.15 H₂O: C, 68.64; H, 8.30; N 6.67. Found: C, 68.70; H, 7.96; N, 6.52.

### Step 2: Preparation of 1-[5-(4-fluorophenyl)-4-[4-(methylsulfonyl)phenyl]-1H-imidazol-2-yl]-4-phenyl-1-bubanone

To a solution of 1-(1-ethoxyethyl)-4-(4-fluorophenyl)-5-[4-(methyl-thio)phenyl]-1H-imidazole (Example 83 Step 1) (533 mg; 1.3 mmol) and TMEDA (491.56 mg; 4.21 mmol) in 7 ml of THF at -78°C was added 0.70 ml of a 2.5 M n-butyllithium in hexanes. After stirring for 15 minutes, a solution of N-methoxy-N-methylbenzenebutanamide (Step 1)(363 mg; 1.75 mmol) in 1 ml of THF was added. The solution was warmed to 25°C over 1 hour. The reaction was diluted with diethyl ether and washed with a saturated, aqueous solution of sodium bicarbonate. The organic extracts were dried (MgSO₄), filtered and evaporated under reduced pressure to give the crude product, which was purified by flash chromatography (SiO₂; 25% ethyl acetate/hexane) to give 229 mg of product. This material (74 mg) was dissolved in 8 ml of methanol/THF (1.6:1). Oxone® (450 mg) was dissolved in 2 ml of water and this solution was added all at once. After stirring the reaction at 25°C for 1.5 hours, the reaction was poured into water and extracted with methylene chloride. The organic extracts were dried (MgSO₄) and evaporated under reduced pressure to give a white solid which was purified by flash chromatography (SiO₂; 50% ethyl acetate/toluene) to give 43 mg of the desired sulfone: Anal. Calc'd. for C₂₆H₂₃FN₂O₃S + 0.4 H₂O: C, 66.48; H, 5.11; N 5.96. Found: C, 66.65; H, 4.94; N, 5.81.

### Example 83

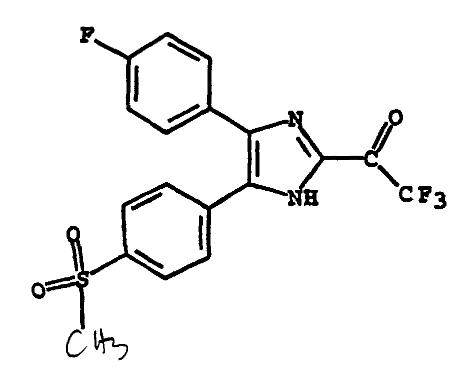

### 2,2,2-Trifluoro-1-[4-(4-fluorophenyl)-5-[4-methylsulfonyl)phenyl]-1H-imidazol-2-yl]ethanone

### Step 1: Preparation of 1-[1-(1-ethoxyethyl)-4-(4-fluorophenyl)-5-[4-(methylthio)phenyl]-1H-imidazol-2-yl]-2,2,2-trifluoroethanone

To a solution of 1-(1-ethoxyethyl)-4-(4-fluorophenyl)-5-[4-(methyl-thio)phenyl]-1H-imidazole (Example 83 Step 1) (403.8 mg; 1.04 mmol) and TMEDA (582.7 mg; 5.01 mmol) in 6 ml of THF at -78°C was added 1.23 ml of 2.5 M n-butyllithium in hexanes. After stirring for 15 minutes, a solution of N-methoxy-N-methyltrifluoracetamide, (167 mg; 1.41 mmol) in 1 ml of THF was added. The solution was warmed to 25°C over one hour. The reaction was diluted with diethyl ether and washed with a saturated, aqueous, solution of sodium bicarbonate. The organic extracts were dried (MgSO₄), filtered and evaporated under reduced pressure to give the crude product, which was purified by flash chromatography (SiO₂; 25% ethyl acetate/hexane) to give 81 mg of 1-[1-(1-ethoxyethyl)-4-(4-fluorophenyl)-5-[4-(methylthio)phenyl]-1H-imidazol-2-yl]-2,2,2-trifluoroethanone which was used directly in the next step.

### Step 2: Preparation of 2,2,2-trifluoro-1-[4-(4-fluorophenyl)-5-[4-methylsulfonyl)phenyl]-1H-imidazol-2-yl]ethanone

1-[1-(1-Ethoxyethyl)-4-(4-fluorophenyl)-5-[4-(methylthio)phenyl]-1H-imidazol-2-yl]-2,2,2-trifluoroethanone (Step 1) was dissolved in 8 ml of methanol/THF (1.6:1). Oxone® (450 mg) was dissolved in 2 ml of water and this solution was added all at once to methanol/THF solution. After stirring the reaction at 25°C for 1.5 hours, the reaction was poured into water and extracted with methylene chloride. The organic extracts were dried (MgSO₄) and evaporated under reduced pressure to give a white solid which was purified by preparative thin layer chromatography (SiO₂; 50% ethyl acetate/toluene) to give 23 mg of the desired sulfone: mp 291.7°C. Anal. Calc'd. for C₁₈H₁₂F₄N₂O₃S: C, 52.43; H, 2.93; N 6.79. Found: C, 52.38; H, 2.96; N, 6.26.

### Example 84

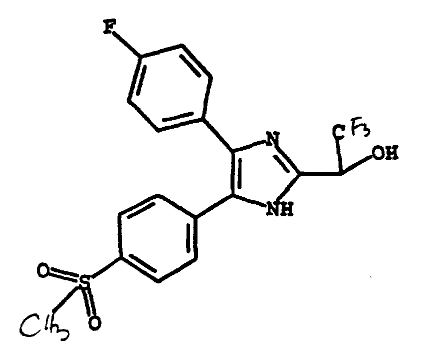

### 4-(4-Fluorophenyl)-5-(4-(methylsulfonyl)phenyl]-α-(trifluoromethyl)-1H-imidazole-2-methanol

To a solution of 85 mg of 1-[1-(1-ethoxyethyl)-4-(4-fluorophenyl)-5-[4-(methylthio)phenyl]-1H-imidazol-2-yl]-2,2,2-trifluoroethanone (Example 89, Step 1) (0.18 mmol) in 1 ml of methanol at 0°C was added 10 mg of sodium borohydride (0.26 mmol). The solution turned immediately from yellow to colorless. The reaction was quenched with 1% solution of aqueous hydrochloric acid and diluted with methylene chloride then washed with brine. The organic extracts were dried (MgSO₄), filtered and the solvent was removed under reduced pressure to provide 77.1 mg of a white film. All of this material was dissolved in 8 ml of methanol/THF (1.6:1). Oxone® (450 mg) was dissolved in 2 ml of water and the solution was added all at once. After stirring the reaction at 25°C for 1.5 hours, the reaction was poured into water and extracted with methylene chloride. The organic extracts were dried (MgSO₄) and evaporated under reduced pressure to give a white foam which was purified by preparative thin layer chromatography (SiO₂; 100% ethyl acetate) to give 50.5 mg of the desired sulfone: mp 126-130° C. Anal. Calc'd. for C₁₈H₁₄F₄N₂O₃S: C, 51.49; H, 3.54; N 6.33. Found: C, 51.53; H, 3.26; N, 6.31.

### Example 85

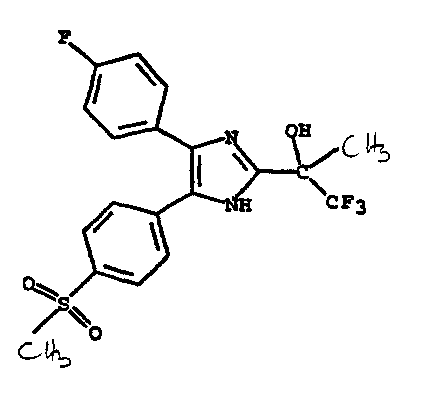

### 4-(4-Fluorophenyl)-α-methyl-5-[4-(methylsulfonyl)phenyl]-α-(trifluoromethyl)-1H-imidazole-2-methanol

To a solution of 1-(1-ethoxyethyl)-4-(4-fluorophenyl)-5-[4-(methyl-thio)phenyl]-1H-imidazole (Example 83 Step 1) (346.6 mg; 0.89 mmol) and TMEDA (495.9 mg; 4.26 mmol) in 5.08 ml of THF, at -78°C, was added 0.48 ml of a 2.5 M n-butyllithium in hexanes. After stirring for 15 minutes, a solution of 1,1,1-trifluoroacetone (135.5 mg; 1.21 mmol) in 1 ml of THF was added. The solution was warmed to 25°C over a period of one hour. The reaction was diluted with diethyl ether and washed with a saturated, aqueous, solution of sodium bicarbonate. The organic extracts were dried (MgSO₄), filtered and evaporated under reduced pressure to give the crude product, which was purified by flash chromatography (SiO₂; 50% ethyl acetate/hexane) to give 220 mg of a yellow oil. This material (100 mg) was dissolved in 8 ml of methanol/THF (1.6:1). Oxone® (450 mg) was dissolved in 2 ml of water and the solution was added all at once to the methanol/THF solution. After stirring the reaction at 25°C for 1.5 hours, the reaction was poured into water and extracted with methylene chloride. The organic extracts were dried (MgSO₄) and evaporated under reduced pressure to give a white solid which was purified by preparative thin layer chromatography (SiO₂; 50% ethyl acetate/toluene) to give 40 mg of the desired sulfone: Anal. Calc'd. for C₁₉H₁₆F₄N₂O₃S: C, 53.27; H, 3.76; N 6.54. Found: C, 53.42; H, 3.73; N, 6.29.

### Example 86

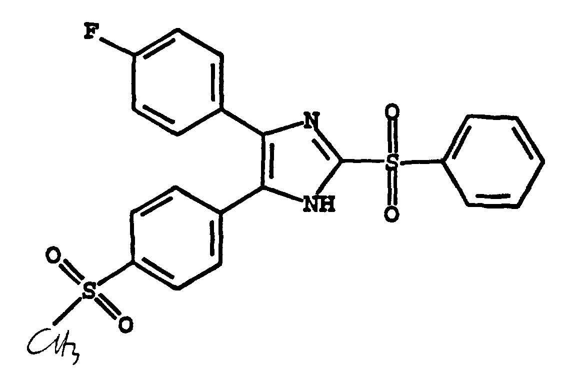

### 4-(4-Fluorophenyl)-5- [4-(methylsulfonyl)phenyl]-2-(phenyl-sulfonyl)-1H-imidazole

To a solution of 1-(1-ethoxyethyl)-4-(4-fluorophenyl)-5-[4-(methyl-thio)phenyl]-1H-imidazole (Example 83 Step 1) (208 mg; 0.53 mmol) and TMEDA (76.3 mg; 0.23 mmol) in 3.02 ml of THF, under an argon atmosphere at -78°C, was added 0.21 ml of a 2.5 M n-butyllithium in hexanes. After stirring for 15 minutes, N-fluorodibenzene sulfonamide (167 mg; 0.53 mmol) was added and the reaction was slowly warmed to 25°C. The reaction was quenched with 1-2 ml of an aqueous, saturated solution of sodium bicarbonate, diluted with ether and washed with additional saturated, sodium bicarbonate solution. The organic extracts were dried (MgSO₄), filtered and evaporated under reduced pressure to give a yellow foam, which was purified by flash chromatography (SiO₂; 25% ethyl acetate/hexane) to give 179.9 mg of a yellow foam. The foam (90 mg) was dissolved in 8 ml of methanol/THF (1.6:1). Oxone® (450 mg) was dissolved in 2 ml of water and the solution was added all at once. After stirring the reaction at 25°C for 1.5 hours, the reaction was poured into water and extracted with methylene chloride. The organic extracts were dried (MgSO₄) and evaporated under reduced pressure to give product which was purified by flash chromatography (SiO₂; 5% ethanol/ methylene chloride) to give 86 mg of the unexpected sulfone: Anal. Calc'd. for C₂₂H₁₇FN₂O₄S₂: C, 45.71; H, 3.20; N 4.44; S, 20.34. Found: C, 45.55; H, 2.95; N, 4.33; S, 20.49.

### Example 87

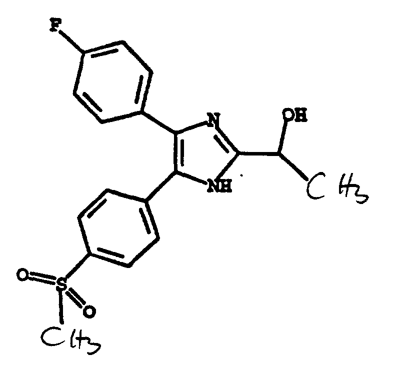

### 4-(4-Fluorophenyl)-α-methyl-5-[4-(methylsulfonyl)phenyl]-1H-imidazole-2-methanol

### Step 1: Preparation of 1-[(1-ethoxyethyl)-4-(4-fluorophenyl)-5-[4-(methylthio)phenyl]-1H-imidazol-2-yl]ethanone

To a solution of 1-(1-ethoxyethyl)-4-(4-fluorophenyl)-5-[4-(methylthio)phenyl]-1H-imidazole (Example 9, Step 2) (206 mg; 0.53 mmol) and TMEDA (324 mg; 1.65 mmol) in 3.0 mL of THF at -78°C was added 0.28 ml of a 2.5 M solution of n-butyllithium in hexanes. After stirring for 15 minutes, 66 ml of N,N-dimethylacetamide (62.7 mg; 0.72 mmol) was added. After 30 minutes, the solution was slowly warmed to 25°C, then 2 ml of an aqueous solution of sodium bicarbonate was added, followed by dilution with 50 mL of diethyl ether. The solution was washed with 50 ml of a saturated solution of sodium bicarbonate and the organic extracts were dried (MgSO₄) and filtered. The solvent was removed under reduced pressure to give 230 mg of a yellow oil, which was purified by flash chromatography (SiO₂; 25% ethyl acetate/hexane) to yield 86 mg of the protected ketone as a slightly yellow oil (37%).

### Step 2: Preparation of 1-[(1-ethoxyethyl)-4-(4-fluorophenyl)-5-[4-(methylthio)phenyl]-1H-imidazol-2-yl]ethanol

To a solution of 1-[(1-ethoxyethyl)-4-(4-fluorophenyl)-5-[4-(methylthio)phenyl]-1H-imidazol-2-yl]ethanone (Step 1) (1.08 g.; 2.9 mmol) at 25°C was added sodium borohydride (109.7 mg; 2.9 mmol). After stirring for 20 hours, the reaction mixture was cooled to 0°C and 50 ml of a 1% aqueous solution of hydrochloric acid was added. This solution was saturated with solid sodium chloride and extracted with 100 ml, then 2 x 50 ml of methylene chloride. The organics were combined then washed with 50 ml of a saturated, aqueous solution of sodium bicarbonate. The organic extracts were dried (MgSO₄), filtered and evaporated under reduced pressure to provide 1.06 g of a light yellow foam, which was purified by flash chromatography (SiO₂, 75% ethyl acetate/hexane) to provide 907 mg of the desired protected alcohol.

### Step 3: Preparation of 4-(4-fluorophenyl)-α-methyl-5-[4-(methylsulfonyl)phenyl]-1H-imidazole-2-methanol

The protected alcohol (Step 2) (400 mg) was dissolved in 8 ml of methanol/THF (1.6:1). Oxone® (450 mg) was dissolved in 2 ml of water and the solution was added all at once. After stirring the reaction at 25°C for 1.5 hours, the reaction was poured into 150 ml of water and extracted with 30 ml of methylene chloride several times. The organic extracts were dried (MgSO₄) and evaporated under reduced pressure to give 40 mg of crude product which was purified by preparative thin layer chromatography (SiO₂; 100% ethyl acetate) to give 14 mg of the sulfone: HRMS, m/e Calc'd. for C₁₈H₁₇N₂O₃SF: 360.0940; measured: 360.0977.

### Example 88

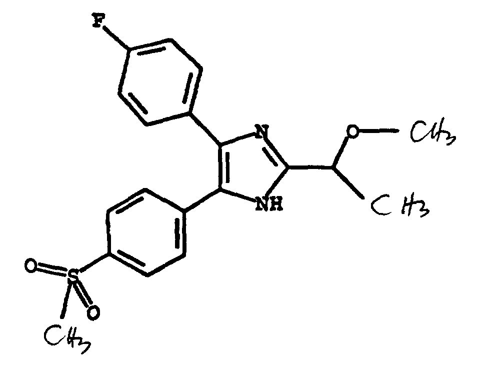

### 4-(4-Fluorophenyl)-2-(1-methoxyethyl)-5-[4-(methylsulfonyl)phenyl]-1H-imidazole

To a solution of 1-[(1-ethoxyethyl)-4-(4-fluorophenyl)-5-[4-(methylthio)phenyl]-1H-imidazol-2-yl]ethanol (Example 93, Step 2) (410 mg; 1.05 mmol) in 4.2 ml of DMF was added 36 mg of sodium hydride (60 wt% oil dispersion). After stirring at 25°C for 15 minutes, 65.4 µl of methyl iodide was added. Stirring continued at 25°C for 12 hours, then the reaction was heated to 36°C for 8 hours, then poured into ethyl acetate and extracted with brine. The organic extracts were dried (MgSO₄), filtered and the solvent was removed under reduced pressure to provide an oil which was purified by medium pressure chromatography (SiO₂; 50% ethyl acetate/hexane) to provide 133 mg of an oil. The alcohol was dissolved in 8 ml of methanol/THF (1.6:1). Oxone® (450 mg) was dissolved in 2 ml of water and the solution was added all at once to the methanol/THF solution. After stirring the reaction at 25°C for 1.5 hours, the reaction was poured into water and extracted with methylene chloride. The organic extracts were dried (MgSO₄) and evaporated under reduced pressure to give the crude product which was purified by preparative thin layer chromatography (SiO₂; 100% ethyl acetate) to give 66 mg of the desired sulfone: mp 212° C Anal. Calc'd. for C₁₉H₁₉N₂O₃SF: C, 60.95; H, 5.11; N 7.48; S, 8.56. Found: C, 61.24; H, 5.00; N, 7.45; S, 8.51.

### Example 89

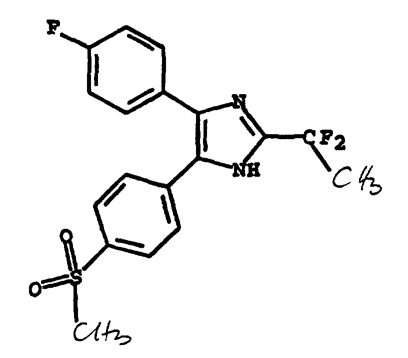

### 2-(1,1-Difluoroethyl)-4-(4-fluorophenyl)-5-[4-(methylsulfonyl)phenyl]-1H-imidazole

### Step 1: Preparation of 5-(4-fluorophenyl)-2-(2-methyl-1,3-dithian-2-yl)-4-[4-methylthio)phenyl]-1H-imidazole

To a suspension of 1-[(1-ethoxyethyl)-4-(4-fluorophenyl)-5-[4-(methylthio)phenyl]-1H-imidazol-2-yl]ethanone (Example 93, Step 1) (797 mg; 2.12 mmol) in 0.42 ml of 1,3-propanedithiol in a 1 ml flask, at 25°C, under argon was added 0.88 ml of boron trifluoride acetic acid-complex. After stirring for 12-18 hours the reaction was slowly quenched with 8 ml of a saturated, solution of sodium bicarbonate then diluted with 8 ml of ethyl acetate. The organic extracts were diluted with additional ethyl acetate and then washed with a 1N solution of aqueous sodium hydroxide and brine. The organic extracts were dried (MgSO₄), filtered and the solvent was removed under reduced pressure to provide 542 mg of a solid which was washed with 25% ethyl acetate/hexane to provide 536.8 mg of 5-(4-fluorophenyl)-2-(2-methyl-1,3-dithian-2-yl)-4-[4-methylthio)phenyl]-1H-imidazole which was used in the following step without further purification: mp 164-168°C.

### Step 2: Preparation of 2-(1,1-difluoroethyl)-4-(4-fluorophenyl)-5-[4-(methyl-sulfonyl)phenyl]-1H-imidazole

A solution of 5-(4-fluorophenyl)-2-(2-methyl-1,3-dithian-2-yl)-4-[4-methylthio)phenyl]-1H-imidazole (Step 1) (268.40 mg; 0.64 mmol) was prepared by sonicating a suspension of this material in 2 ml of methylene chloride then heated in a hot water bath till the compound dissolved. N-iodosuccinimide (863.9 mg; 3.84 ml) was suspended in 7.8 ml of anhydrous methylene chloride in a separate flask, under an atmosphere of argon, cooled to -30°C and 0.43 ml of HF-pyridine (70%) was added to the cooled suspension. Immediately the solution of 5-(4-fluorophenyl)-2-(2-methyl-1,3-dithian-2-yl)-4-[4-methylthio)phenyl]-1H-imidazole was added and the resulting solution turned purple instantaneously. After maintaining the temperature between -27°C and -40°C for 2.5 hours, the reaction mixture was diluted with a saturated, aqueous, solution of sodium bicarbonate and additional methylene chloride. A spatula tip of solid sodium thiosulfate was added. After carefully shaking the two phase mixture the color disappeared. The organic extracts were dried (MgSO₄), filtered and the solvent was removed under reduced pressure to provide 309 mg of a yellow oil which solidified upon storage in the refrigerator overnight. The yellow brown solid was suspended in heptane and the solvent was removed under reduced pressure. This step was repeated twice which resulted in 242 mg of a yellow powder. This material (214 mg) was dissolved in 8 ml of methanol/THF (1.6:1). Oxone® (450 mg) was dissolved in 2.5 ml of water and then this solution was added all at once. After stirring the reaction at 25°C for 1.25 hours, the reaction was poured into water and extracted with methylene chloride. The organic extracts were dried (MgSO₄) and evaporated under reduced pressure to give the crude product which was purified by flash chromatography (SiO₂; 50% ethyl acetate/ hexane) to give 57.4 mg of the desired sulfone: mp 211° C; Anal. Calc'd. for C₁₈H₁₅F₃N₂O₂S: C, 45.71; H, 3.20; N 4.44; S, 20.34. Found: C, 45.55; H, 2.95; N, 4.33; S, 20.49.

### BIOLOGICAL EVALUATION

### Rat Carrageenan Foot Pad Edema Test

The carrageenan foot edema test was performed with materials, reagents and procedures essentially as described by Winter, et al., (*Proc. Soc. Exp. Biol. Med.*, **111**, 544 (1962)). Male Sprague-Dawley rats were selected in each group so that the average body weight was as close as possible. Rats were fasted with free access to water for over sixteen hours prior to the test. The rats were dosed orally (1 ml) with compounds suspended in vehicle containing 0.5% methylcellulose and 0.025% surfactant, or with vehicle alone. One hour later a subplantar injection of 0.1 ml of 1% solution of carrageenan/sterile 0.9% saline was administered and the volume of the injected foot was measured with a displacement plethysmometer connected to a pressure transducer with a digital indicator. Three hours after the injection of the carrageenan, the volume of the foot was again measured. The average foot swelling in a group of drug-treated animals was compared with that of a group of placebo-treated animals and the percentage inhibition of edema was determined (Otterness and Bliven, *Laboratory Models* for Testing *NSAIDs*, in **Non-steroidal Anti-Inflammatory Drugs**, (J. Lombardino, ed. 1985)). The % inhibition shows the % decrease from control paw volume determined in this procedure and the data for selected compounds in this invention are summarized in Table I.

### Rat Carrageenan-induced Analgesia Test

The analgesia test using rat carrageenan was performed with materials, reagents and procedures essentially as described by Hargreaves, et al., (*Pain*, **32,** 77 (1988)). Male Sprague-Dawley rats were treated as previously described for the Carrageenan Foot Pad Edema test. Three hours after the injection of the carrageenan, the rats were placed in a special plexiglass container with a transparent floor having a high intensity lamp as a radiant heat source, positionable under the floor. After an initial twenty minute period, thermal stimulation was begun on either the injected foot or on the contralateral uninjected foot. A photoelectric cell turned off the lamp and timer when light was interrupted by paw withdrawal. The time until the rat withdraws its foot was then measured. The withdrawal latency in seconds was determined for the control and drug-treated groups, and percent inhibition of the hyperalgesic foot withdrawal determined. Results are shown in Table I.

**TABLE I.**

| | **RAT PAW EDEMA** % Inhibition¹ | **ANALGESIA** % Inhibition¹ |
|---|---|---|
| Example | | |
| 1 | 22 | 25 |
| 55 | 14 | |
| 66 | 27 | 29* |

| | | |
|---|---|---|
| * - 50 mg/kg body weight | | |
| 1- @ 30 mg/kg body weight | | |

### Evaluation of COX I and COX II activity in vitro

The compounds of this invention exhibited inhibition *in vitro* of COX II. The COX II inhibition activity of the compounds of this invention illustrated in the Examples was determined by the following methods.

### a. Preparation of recombinant COX baculoviruses

A 2.0 kb fragment containing the coding region of either human or murine COX-I or human or murine COX-II was cloned into a BamH1 site of the baculovirus transfer vector pVL1393 (Invitrogen) to generate the baculovirus transfer vectors for COX-I and COX-II in a manner similar to the method of D.R. O'Reilly et al (*Baculovirus Expression Vectors: A Laboratory Manual* (1992)). Recombinant baculoviruses were isolated by transfecting 4 µg of baculovirus transfer vector DNA into SF9 insect cells (2x10e8) along with 200 ng of linearized baculovirus plasmid DNA by the calcium phosphate method. See M.D. Summers and G.E. Smith, *A Manual of Methods for Baculovirus Vectors and Insect Cell Culture Procedures,* Texas Agric. Exp. Station Bull. 1555 (1987). Recombinant viruses were purified by three rounds of plaque purification and high titer (10E7 - 10E8 pfu/ml) stocks of virus were prepared. For large scale production, SF9 insect cells were infected in 10 liter fermentors (0.5 x 10⁶/ml) with the recombinant baculovirus stock such that the multiplicity of infection was 0.1. After 72 hours the cells were centrifuged and the cell pellet homogenized in Tris/Sucrose (50 mM: 25%, pH 8.0) containing 1% 3-[(3-cholamidopropyl)dimethylammonio] -1-propanesulfonate (CHAPS). The homogenate was centrifuged at 10,000xG for 30 minutes, and the resultant supernatant was stored at -80°C before being assayed for COX activity.

### b. Assay for COX I and COX II activity:

COX activity was assayed as PGE₂ formed/µg protein/time using an ELISA to detect the prostaglandin released. CHAPS-solubilized insect cell membranes containing the appropriate COX enzyme were incubated in a potassium phosphate buffer (50 mM, pH 8.0) containing epinephrine, phenol, and heme with the addition of arachidonic acid (10 µM). Compounds were pre-incubated with the enzyme for 10-20 minutes prior to the addition of arachidonic acid. Any reaction between the arachidonic acid and the enzyme was stopped after ten minutes at 37°C/room temperature by transferring 40 µl of reaction mix into 160 µl ELISA buffer and 25 µM indomethacin. The PGE₂ formed was measured by standard ELISA technology (Cayman Chemical). Results are shown in Table II.

**TABLE II.**

| **Example** | **Human COX II** **ID**_{**50**} **µM** | **Human COX I** **ID**_{**50**} **µM** |
|---|---|---|
| 1 | 0.2 | >100 |
| 2 | 0.2 | >100 |
| 3 | 0.2 | 2.2 |
| 4 | 0.5 | >100 |
| 5 | 1.2 | >100 |
| 6 | 4.6 | >100 |
| 7 | 4.8 | >100 |
| 8 | <0.1 | 2.1 |
| 9 | 5.1 | >100 |
| 10 | 1.2 | 63.5 |
| 11 | 0.4 | 10.4 |
| 12 | 1.3 | >100 |
| 13 | <0.1 | 9.6 |
| 14 | 0.6 | 2.0 |
| 15 | 2.9 | 5.6 |
| 16 | 0.6 | 20 |
| 17 | 0.3 | >100 |
| 18 | 0.2 | >100 |
| 19 | 0.5 | >100 |
| 20 | 40.7 | >100 |
| 21 | <0.1 | 10.8 |
| 45 | 0.1 | 12.9 |
| 46 | 8.7 | >100 |
| 47 | 55 | 10.8 |
| 48 | 1.7 | >100 |
| 52 | 0.3 | >100 |
| 55 | 0.2 | >100 |
| 57 | 0.1 | 6.2 |
| 59 | 2.4 | >100 |
| 60 | 1.3 | >100 |
| 70 | 1.4 | >100 |
| 73 | 8.3 | >100 |
| 74 | 2.9 | >100 |
| 75 | 3.3 | 77 |
| 76 | 0.7 | >100 |
| 77 | 4.3 | >100 |
| 79 | 0.4 | >100 |
| 83 | 5.3 3 | >100 |
| 84 | 3.9 | >100 |
| 85 | 5.9 | >100 |
| 86 | 3.0 | >100 |
| 88 | 6.4 | >100 |
| 89 | 2.4 | >100 |

Biological paradigms for testing the cytokine-inhibiting activity of these compounds are found in WO-A-9513067, published 18 May 1995.

Also embraced within this invention is a class of pharmaceutical compositions comprising the active compounds of this combination therapy in association with one or more non-toxic, pharmaceutically-acceptable carriers and/or diluents and/or adjuvants (collectively referred to herein as "carrier" materials) and, if desired, other active ingredients. The active compounds of the present invention may be administered by any suitable route, preferably in the form of a pharmaceutical composition adapted to such a route, and in a dose effective for the treatment intended. The active compounds and composition may, for example, be administered orally, intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The active ingredient may also be administered by injection as a composition wherein, for example, saline, dextrose or water may be used as a suitable carrier.

The amount of therapeutically active compounds that are administered and the dosage regimen for treating a disease condition with the compounds and/or compositions of this invention depends on a variety of factors, including the age, weight, sex and medical condition of the subject, the severity of the disease, the route and frequency of administration, and the particular compound employed, and thus may vary widely. The pharmaceutical compositions may contain active ingredients in the range of 0.1 to 2000 mg, preferably in the range of 0.5 to 500 mg and most preferably between 1 and 100 mg. A daily dose of 0.01 to 100 mg/kg body weight, preferably between 0.5 and about 20 mg/kg body weight and most preferably between 0.1 to 10 mg/kg body weight, may be appropriate. The daily dose can be administered in one to four doses per day.

In the case of psoriasis and other skin conditions, it may be preferable to apply a topical preparation of compounds of this invention to the affected area two to four times a day.

For inflammations of the eye or other external tissues, e.g., mouth and skin, the formulations are preferably applied as a topical ointment or cream, or as a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base. Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs. The compounds of this invention can also be administered by a transdermal device. Preferably topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredients are dissolved or suspended in suitable carrier, especially an aqueous solvent for the active ingredients. The antiinflammatory active ingredients are preferably present in such formulations in a concentration of 0.5 to 20%, advantageously 0.5 to 10% and particularly 1.5% w/w.

For therapeutic purposes, the active compounds of this combination invention are ordinarily combined with one or more adjuvants appropriate to the indicated route of administration. If administered *per os*, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or nonaqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

## Claims

1. A compound having the Formula I 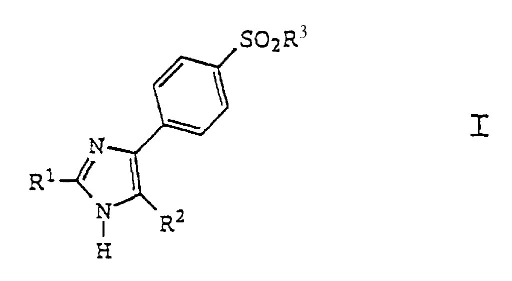 wherein R¹ is selected from C₁-C₁₀-alkyl, C₁-C₆-haloalkyl, aryl-C₁-C₆-alkyl, heterocyclic-C₁-C₆-alkyl, heteroaryl-C₁-C₆-alkyl, formyl, cyano, mercapto, C₁-C₆-alkoxy, C₁-C₆-alkylthio, C₁-C₆-alkylthio-C₁-C₆-alkyl, C₁-C₆-alkylsulfonyl, C₁-C₆-haloalkylsulfonyl, halo, C₁-C₆-hydroxyalkyl, C₁-C₆-alkoxy-C₁-C₆-alkyl, C₂-C₆-alkenyloxy-C₁-C₆-alkyl, C₁-C₆-alkylcarbonyl, arylcarbonyl, aryl-C₁-C₆-alkylcarbonyl, cyano-C₁-C₆-alkyl, aryl-C₂-C₆-alkenyl, C₁-C₆-aminoalkyl, C₁-C₆-alkylamino-C₁-C₆-alkyl, N-arylamino-C₁-C₆-alkyl, N-C₁-C₆-alkyl-N-arylamino-C₁-C₆-alkyl, carboxy-C₁-C₆-alkyl, C₁-C₆-alkoxycarbonyl-C₁-C₆-alkyl, C₁-C₆-alkoxycarbonyl, C₁-C₆-haloalkylcarbonyl, carboxyl, aminocarbonyl, C₁-C₆-alkylaminocarbonyl, mono or di C₁-C₆-alkylaminocarbonyl-C₁-C₆-alkyl, aryl-C₁-C₆-alkoxy, aryl-C₁-C₆-alkylthio, phenylsulfonyl, heteroaryl-C₁-C₆-alkoxy, heteroaryl-C₁-C₆-alkylthio, heteroaryl-C₁-C₆-alkoxy-C₁-C₆-alkyl, heteroaryloxy-C₁-C₆-alkyl, heteroarylthio-C₁-C₆-alkyl, heteroaryl-C₁-C₆-alkylthio-C₁-C₆-alkyl, heteroaryloxy, heteroarylthio, aryl-C₁-C₆-thioalkyl, aryloxy-C₁-C₆-alkyl, arylthio, aryloxy, aryl-C₁-C₆-alkylthio-C₁-C₆-alkyl, aryl-C₁-C₆-alkoxy-C₁-C₆-alkyl, C₁-C₆-alkoxyaryl-C₁-C₆-alkoxy-C₁-C₆-alkyl, aryl and heteroaryl, wherein the aryl and heteroaryl radicals are optionally substituted at a substitutable position with one or more radicals selected from halo, C₁-C₆-alkylthio, C₁-C₆-alkylsulfinyl, C₁-C₁₀-alkyl, cyano, C₁-C₆-haloalkyl, hydroxyl, C₁-C₆-alkoxy, C₁-C₆-hydroxyalkyl and C₁-C₆-haloalkoxy; wherein R² is selected from heteroaryl, and aryl, wherein the heteroaryl, and aryl radicals are substituted with one or more radicals selected from hydrido, halo, C₁-C₆-alkylthio, C₁-C₆-alkylsulfinyl, C₁-C₆-alkyl, cyano, carboxyl, C₁-C₆-alkoxycarbonyl, C₁-C₆-haloalkyl, hydroxyl, C₁-C₆-alkoxy, C₁-C₆-hydroxyalkyl, C₁-C₆-alkoxy-C₁-C₆-alkyl, C₁-C₆-haloalkoxy, amino, mono and di C₁-C₆-alkylamino, mono and di arylamino and nitro; and wherein R³ is selected from C₁-C₁₀-alkyl and amino;
wherein aryl means phenyl or naphthyl
wherein heterocyclic/heterocyclo means saturated, partially saturated or unsaturated heteroatom containing 5 or 6 membered radicals where the heteroatom is selected from nitrogen, sulfur and oxygen;
wherein heteroaryl is a fully unsaturated heterocyclic radical; provided R¹ is not C₁-C₁₀-alkyl when R³ is methyl and when R² is phenyl or phenyl substituted with methyl, methoxy or chloro; further provided R¹ is not propylsulfonyl or trifluoroacetyl when R³ is methyl; or a pharmaceutically-acceptable salt thereof.

2. Compound of Claim 1 wherein R¹ is selected from heterocyclic-C₁-C₆-alkyl, heteroaryl-C₁-C₆-alkyl, formyl, cyano, mercapto, C₁-C₆-alkoxy, C₁-C₆-alkylthio, C₁-C₆-alkylthio-C₁-C₆-alkyl, C₁-C₆-alkylsulfonyl, C₁-C₆-haloalkylsulfonyl, arylsulfonyl, halo, C₂-C₆-alkenyloxy-C₁-C₆-alkyl, C₁-C₆-alkylcarbonyl, benzoyl, aryl-C₁-C₆-alkylcarbonyl, C₁-C₆-cyanoalkyl, aryl-C₂-C₆-alkenyl, C₁-C₆-aminoalkyl, C₁-C₆-alkylamino-C₁-C₆-alkyl, N-arylamino-C₁-C₆-alkyl, N-C₁-C₆-alkyl-N-arylaminoalkyl, C₁-C₆-alkoxycarbonyl, C₁-C₆-haloalkylcarbonyl, aminocarbonyl, C₁-C₆-alkylaminocarbonyl, C₁-C₆-alkylaminocarbonyl-C₁-C₆-alkyl, aryl-C₁-C₆-alkoxy, aryl-C₁-C₆-alkylthio, phenylsulfonyl, heteroaryl-C₁-C₆-alkoxy, heteroaryloxy, heteroarylthio, arylthio-C₁-C₆-alkyl, aryloxy-C₁-C₆-alkyl, arylthio, aryloxy, aryl-C₁-C₆-alkylthio-C₁-C₆-alkyl, aryl-C₁-C₆-alkoxy-C₁-C₆-alkyl, C₁-C₆-alkoxyaryl-C₁-C₆-alkoxy-C₁-C₆-alkyl, aryl and heteroaryl, wherein the aryl and heteroaryl radicals are optionally substituted at a substitutable position with one or more radicals selected from halo, C₁-C₆-alkylthio, C₁-C₆-alkylsulfinyl, C₁-C₆-alkyl, cyano, C₁-C₆-haloalkyl, hydroxyl, C₁-C₆-alkoxy, C₁-C₆-hydroxyalkyl and C₁-C₆-haloalkoxy; wherein R² and R³ are defined as in claim 1; or a pharmaceutically-acceptable salt thereof.

3. Compound of Claim 1 wherein R¹ is selected from C₁-C₆-alkyl, C₁-C₆-haloalkyl, C₁-C₆-hydroxyalkyl, C₁-C₆-alkoxy-C₁-C₆-alkyl, aryl-C₁-C₆-alkylsulfonyl, arylthio-C₁-C₆-alkyl, heteroaryl-C₁-C₁₀-alkoxy-C₁-C₆-alkyl, heteroaryloxy-C₁-C₆-alkyl, heteroarylthio-C₁-C₆-alkyl, heteroaryl-C₁-C₆-alkylthio-C₁-C₆-alkyl, aryl-C₁-C₆-alkylthio, and aryl-C₁-C₆-alkoxy; wherein R² is selected from heteroaryl, and aryl, wherein the heteroaryl, and aryl radicals are substituted at a substitutable position with one or more radicals selected from hydrido, halo, C₁-C₆-alkylthio, C₁-C₆-alkylsulfinyl, C₁-C₆-alkyl, cyano, carboxyl, C₁-C₆-alkoxycarbonyl, C₁-C₆-haloalkyl, hyrdoxyl, C₁-C₆-alkoxy, C₁-C₆-hydroxyalkyl, C₁-C₆-alkoxy-C₁-C₆-alkyl, C₁-C₆-haloalkoxy, amino, C₁-C₆-alkylamino, phenylamino and nitro; and wherein R³ is defined as in claim 2;
or a pharmaceutically-acceptable salt thereof.

4. Compound of Claim 1 selected from compounds, and their pharmaceutically acceptable salts, of the group consisting of
5-(3-chlorophenyl)-4-(4-methylsulfonylphenyl)-2-trifluoromethyl-1H-imidazole;
5-(3-chloro-4-methylphenyl)-4-(4-methylsulfonylphenyl)-2-trifluoromethyl-1H-imidazole;
5-(3-fluorophenyl)-4-(4-methylsulfonylphenyl)-2-trifluoromethyl-1H-imidazole;
5-(3-chloro-4-methoxyphenyl)-4-(4-methylsulfonylphenyl)-2-trifluoromethyl-1H-imidazole;
5-(4-fluorophenyl)-4-(4-methylsulfonylphenyl)-2-trifluoromethyl-1H-imidazole;
5-(4-fluorophenyl)-4-(4-methylsulfonylphenyl)-2-phenoxymethyl-1H-imidazole;
5-(4-fluorophenyl)-4-(4-methylsulfonylphenyl)-2-(2-phenyl-trans-eth-1-ene)-1H-imidazole;
2-(2-benzofuryl)-5-(4-fluorophenyl)-4-(4-methylsulfonylphenyl)-1H-imidazole;
5-(4-fluorophenyl)-2-(2-furyl)-4-(4-methylsulfonylphenyl)-1H-imidazole;
5-(4-fluorophenyl)-2-methyl-4-(4-methylsulfonylphenyl)-1H-imidazole;
5-(4-fluorophenyl)-2-isopropyl-4-(4-methylsulfonylphenyl)-1H-imidazole;
4-[5-(4-fluorophenyl)-2-hydroxymethyl-4-(4-methylsulfonylphenyl)-1H-imidazole;
2-benzylthio-5-(4-fluorophenyl)-4-[4-(methylsulfonyl)phenyl]imidazole;
5-(3-fluoro-4-methoxyphenyl)-4-[4-(methylsulfonyl)phenyl]-2-(trifluoromethyl)-1H-imidazole;
5-(4-chlorophenyl)-4-[4-(methylsulfonyl)phenyl]-2-(trifluoromethyl)-1H-imidazole;
5-(2-chlorophenyl)-4-[4-(methylsulfonyl)phenyl]-2-(trifluoromethyl)-1H-imidazole;
5-(3,4-dichlorophenyl)-4-[4-(methylsulfonyl)phenyl]-2-(trifluoromethyl)-1H-imidazole;
4-[4-(methylsulfonyl)phenyl]-5-(2-naphthyl)-2-(trifluoromethyl)-1H-imidazole;
5-(4-methoxyphenyl)-4-[4-(methylsulfonyl)phenyl]-2-(trifluoromethyl)-1H-imidazole;
5-(4-methylphenyl)-4-[4-(methylsulfonyl)phenyl]-2-(trifluoromethyl)-1H-imidazole;
5-(2,4-difluorophenyl)-4-[4-(methylsulfonyl)phenyl]-2-(trifluoromethyl)-1H-imidazole;
5-(3,4-difluorophenyl)-4-[4-(methylsulfonyl)phenyl]-2-(trifluoromethyl)-1H-imidazole;
4-[4-(methylsulfonyl)phenyl]-5-phenyl-2-(trifluoromethyl)-1H-imidazole;
5-(4-fluorophenyl)-2-(3-furyl)-4-(4-methylsulfonylphenyl)-1H-imidazole;
5-(3,5-dimethylphenyl)-4-[4-(methylsulfonyl)phenyl]-2-(trifluoromethyl)-1H-imidazole;
5-(5-bromothien-2-yl)-4-[4-(methylsulfonyl)phenyl]-2-(trifluoromethyl)-1H-imidazole;
N,N-dimethyl-4-[4-[4-(methylsulfonyl)phenyl]-2-(trifluoromethyl)-1H-imidazol-5-yl]benzenamine;
5-(3,5-dimethyl-4-methoxyphenyl)-4-[4-(methylsulfonyl)phenyl]-2-(trifluoromethyl)-1H-imidazole;
5-[3-fluoro-4-(methylthio)phenyl]-4-[4-(methylsulfonyl)phenyl]-2-(trifluoromethyl)-1H-imidazole;
5-(3-chloro-5-methylphenyl)-4-[4-(methylsulfonyl)phenyl]-2-(trifluoromethyl)-1H-imidazole;
5-[3-chloro-4-(methylthio)phenyl]-4-[4-(methylsulfonyl)phenyl]-2-(trifluoromethyl)-1H-imidazole;
5-(3-fluoro-4-methylphenyl)-4-[4-(methylsulfonyl)phenyl]-2-(trifluoromethyl)-1H-imidazole;
5-(3-methylphenyl)-4-[4-(methylsulfonyl)phenyl]-2-trifluoromethyl-1H-imidazole;
4-[4-(methylsulfonyl)phenyl]-5-(3-pyridyl)-2-(trifluoromethyl)-1H-imidazole;
5-(2-fluorophenyl)-4-[4-(methylsulfonyl)phenyl]-2-(trifluoromethyl)-1H-imidazole;
5-(3-methoxyphenyl)-4-[4-(methylsulfonyl)phenyl]-2-(trifluoromethyl)-1H-imidazole;
5-(2-methylphenyl)-4-[4-(methylsulfonyl)phenyl]-2-(trifluoromethyl)-1H-imidazole;
5-(cyclohexyl)-4-[4-(methylsulfonyl)phenyl]-2-(trifluoromethyl)-1H-imidazole;
4-[4-(methylsulfonyl)phenyl]-5-(2-thiophenyl)-2-(trifluoromethyl)-1H-imidazole;
5-(4-chloro-3-methylphenyl)-4-[4-(methylsulfonyl)phenyl]-2-(trifluoromethyl)-1H-imidazole;
5-(4-cycloheptyl)-4-[4-(methylsulfonyl)phenyl]-2-(trifluoromethyl)-1H-imidazole;
5-(4-fluoro-3-methylphenyl)-4-[4-(methylsulfonyl)phenyl]-2-(trifluoromethyl)-1H-imidazole;
5-(cyclopentyl)-4-[4-(methylsulfonyl)phenyl]-2-(trifluoromethyl)-1H-imidazole;
4-[4-(methylsulfonyl)phenyl]-5-(3-thiophenyl)-2-(trifluoromethyl)-1H-imidazole;
5-(3-flouro-2-methylphenyl)-4-[4-(methylsulfonyl)phenyl]-2-(trifluoromethyl)-1H-imidazole;
5-(4-chloro-3-methoxyphenyl)-4-[4-(methylsulfonyl)phenyl]-2-(trifluoromethyl)-1H-imidazole;
5-(4-fluorophenyl)-2-(methoxymethyl)-4-(4-(methylsulfonyl)-phenyl]-1H-imidazole.

5. Compound of Claim 1 selected from compounds, and their pharmaceutically acceptable salts, of the group consisting of
4-[5-(4-fluorophenyl)-2-(trifluoromethyl)-1H-imidazol-4-yl]benzenesulfonamide;
4-(5-(4-fluorophenyl)-2-phenoxymethyl-1H-imidazol-4-yl)benzenesulfonamide;
4-[5-(4-fluorophenyl)-2-(2-phenyl-*trans*-eth-1-ene)-1H-imidazol-4-yl]benzenesulfonamide;
4-[2-(2-benzofuryl)-5-(4-fluorophenyl)-1H-imidazol-4-yl]benzenesulfonamide;
4-[5-(4-fluorophenyl)-2-isopropyl-1H-imidazol-4-yl]benzenesulfonamide;
4-[5-(4-fluorophenyl)-2-methyl-1H-imidazol-4-yl]benzenesulfonamide;
4-[5-(4-fluorophenyl)-2-(2-furyl)-1H-imidazol-4-yl]benzenesulfonamide;
4-[2-benzylthio-5-(4-fluorophenyl)-1H-imidazol-4-yl]benzenesulfonamide;
4-[5-(4-fluorophenyl)-2-hydroxymethyl-1H-imidazol-4-yl]benzenesulfonamide;
4-[5-(3-fluoro-4-methoxyphenyl)-2-(trifluoromethyl)-1H-imidazol-4-yl]benzenesulfonamide;
4-[5-(4-chlorophenyl)-2-(trifluoromethyl)-1H-imidazol-4-yl]benzenesulfonamide;
4-[5-(2-chlorophenyl)-2-(trifluoromethyl)-1H-imidazol-4-yl]benzenesulfonamide;
4-[5-(3,4-dichlorophenyl)-2-(trifluoromethyl)-1H-imidazol-4-yl]benzenesulfonamide;
4-[5-(2-naphthyl)-2-(trifluoromethyl)-1H-imidazol-4-yl]benzenesulfonamide;
4-[5-(4-methoxyphenyl)-2-(trifluoromethyl)-1H-imidazol-4-yl]benzenesulfonamide;
4-[5-(4-methylphenyl)-2-(trifluoromethyl)-1H-imidazol-4-yl]benzenesulfonamide;
4-[5-(2,4-difluorophenyl)-2-(trifluoromethyl)-1H-imidazol-4-yl]benzenesulfonamide;
4-[5-(3,4-difluorophenyl)-2-(trifluoromethyl)-1H-imidazol-4-yl]benzenesulfonamide;
4-[5-phenyl-2-(trifluoromethyl)-1H-imidazol-4-yl]benzenesulfonamide; and
4-[5-(4-fluorophenyl)-2-(3-furyl)-1H-imidazol-4-yl]benzenesulfonamide.

6. A pharmaceutical composition comprising a therapeutically-effective amount of a compound, said compound selected from a family of compounds according to any of Claims 1 - 5; or a pharmaceutically-acceptable salt thereof.

7. Use of a compound of any of Claims 1, 2, 3, 4, or 5; or a pharmaceutically-acceptable salt thereof for preparing a medicament for treating inflammation or an inflammation-associated disorder in a subject.

8. Use according to Claim 7 wherein the inflammation-associated disorder is arthritis.

9. Use according to Claim 7 wherein the inflammation-associated disorder is pain.

10. Use according to Claim 7 wherein the inflammation-associated disorder is fever.

## Patentansprüche

1. Eine Verbindung der Formel I 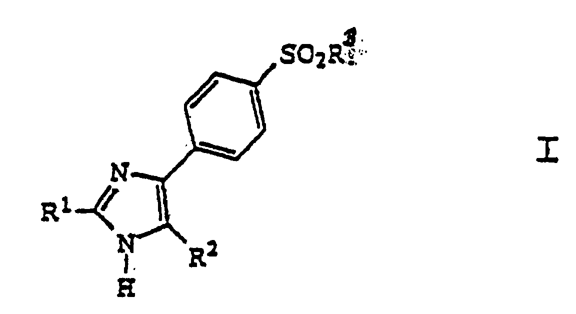 worin R1 ausgewählt ist aus C₁-C₁₀-Alkyl, C₁-C₆-Haloalkyl, Aryl-C₁-C₆-alkyl, heterocyclisches-C₁-C₆-Alkyl, Heteroaryl-C₁-C₆-alkyl, Formyl, Cyano, Mercapto, C₁-C₆-Alkoxy, C₁-C₆-Alkylthio, C₁-C₆-Alkylthio-C₁-C₆-alkyl, C₁-C₆-Alkylsulfonyl, C₁-C₆-Haloalkylsulfonyl, Halo, C₁-C₆-Hydroxyalkyl, C₁-C₆-Alkoxy-C₁-C₆-alkyl, C₂-C₆-Alkenyloxy-C₁-C₆-alkyl, C₁-C₆-Alkylcarbonyl, Arylcarbonyl, Aryl-C₁-C₆-alkylcarbonyl, Cyano-C₁-C₆-alkyl, Aryl-C₂-C₆-alkenyl, C₁-C₆-Aminoalkyl, C₁-C₆-Alkylamino-C₁-C₆-alkyl, N-Arylamino-C₁-C₆-alkyl, N-C₁-C₆-Alkyl-N-arylamino-C₁-C₆-alkyl, Carboxy-C₁-C₆-alkyl, C₁-C₆-Alkoxycarbonyl-C₁-C₆-alkyl, C₁-C₆-Alkoxycarbonyl, C₁-C₆-Haloalkylcarbonyl, Carboxyl, Aminocarbonyl, C₁-C₆-Alkylaminocarbonyl, mono- oder di-C₁-C₆-Alkylaminocarbonyl-C₁-C₆-alkyl, Aryl-C₁-C₆-alkoxy, Aryl-C₁-C₆-alkylthio, Phenylsulfonyl, Heteroaryl-C₁-C₆-alkoxy, Heteroaryl-C₁-C₆-alkylthio, Heteroaryl-C₁-C₆-alkoxy-C₁-C₆-alkyl, Heteroaryloxy-C₁-C₆-alkyl, Heteroarylthio-C₁-C₆-alkyl, Heteroaryl-C₁-C₆-alkylthio-C₁-C₆-alkyl, Heteroaryloxy, Heteroarylthio, Aryl-C₁-C₆-thioalkyl, Aryloxy-C₁-C₆-alkyl, Arylthio, Aryloxy, Aryl-C₁-C₆-alkylthio-C₁-C₆-alkyl, Aryl-C₁-C₆-alkoxy-C₁-C₆-alkyl, C₁-C₆-Alkoxyaryl-C₁-C₆-alkoxy-C₁-C₆-alkyl, Aryl und Heteroaryl, worin die Aryl- und Heteroarylreste gegebenenfalls substituiert sind an einer substituierbaren Position mit einem oder mehreren Resten ausgewählt aus Halo, C₁-C₆-Alkylthio, C₁-C₆-Alkylsulfinyl, C₁-C₁₀-Alkyl, Cyano, C₁-C₆-Haloalkyl, Hydroxyl, C₁-C₆-Alkoxy, C₁-C₆-Hydroxyalkyl and C₁-C₆-Haloalkoxy; worin R² ausgewählt ist aus Heteroaryl und Aryl, worin die Heteroaryl- und Arylreste substituiert sind mit einem oder mehreren Resten ausgewählt aus Hydrido, Halo, C₁-C₆-Alkylthio, C₁-C₆-Alkylsulfinyl, C₁-C₆-Alkyl, Cyano, Carboxyl, C₁-C₆-Alkoxycarbonyl, C₁-C₆-Haloalkyl, Hydroxyl, C₁-C₆-Alkoxy, C₁-C₆-Hydroxyalkyl, C₁-C₆-Alkoxy-C₁-C₆-alkyl, C₁-C₆-Haloalkoxy, Amino, mono- und di-C₁-C₆-Alkylamino, mono- und di-Arylamino und Nitro; und worin R³ ausgewählt ist aus C₁-C₁₀-Alkyl und Amino;
worin Aryl Phenyl oder Naphthyl bedeutet,
worin heterocyclisch/Heterocyclo gesättigte, teilweise gesättigte und ungesättigte, ein Heteroatom enthaltende 5- oder 6-gliedrige Reste bedeutet, worin das Heteroatom ausgewählt ist aus Stickstoff, Schwefel und Sauerstoff;
worin Heteroaryl ein vollständig ungesättigter heterocyclischer Rest ist; mit der Maßgabe, dass R¹ nicht C₁-C₁₀-Alkyl ist, wenn R³ Methyl ist und R² Phenyl ist oder Phenyl substituiert mit Methyl, Methoxy oder Chlor; mit der weiteren Maßgabe, dass R¹ nicht Propylsulfonyl oder Trifluoracetyl ist, wenn R³ Methyl ist; oder ein pharmazeutisch verträgliches Salz davon.

2. Verbindung des Anspruchs 1, worin R¹ ausgewählt ist aus heterocyclischem-C₁-C₆-Alkyl, Heteroaryl-C₁-C₆-alkyl, Formyl, Cyano, Mercapto, C₁-C₆-Alkoxy, C₁-C₆-Alkylthio, C₁-C₆-Alkylthio-C₁-C₆-alkyl, C₁-C₆-Alkylsulfonyl, C₁-C₆-Haloalkylsulfonyl, Arylsulfonyl, Halo, C₂-C₆-Alkenyloxy-C₁-C₆-alkyl, C₁-C₆-Alkylcarbonyl, Benzoyl, Aryl-C₁-C₆-alkylcarbonyl, C₁-C₆-Cyanoalkyl, Aryl-C₂-C₆-alkenyl, C₁-C₆-Aminoalkyl, C₁-C₆-Alkylamino-C₁-C₆-alkyl, N-Arylamino-C₁-C₆-alkyl, N-C₁-C₆-Alkyl-N-arylaminoalkyl, C₁-C₆-Alkoxycarbonyl, C₁-C₆-Haloalkylcarbonyl, Aminocarbonyl, C₁-C₆-Alkylaminocarbonyl, C₁-C₆-Alkylaminocarbonyl-C₁-C₆-alkyl, Aryl-C₁-C₆-alkoxy, Aryl-C₁-C₆-alkylthio, Phenylsulfonyl, Heteroaryl-C₁-C₆-alkoxy, Heteroaryloxy, Heteroarylthio, Arylthio-C₁-C₆-alkyl, Aryloxy-C₁-C₆-alkyl, Arylthio, Aryloxy, Aryl-C₁-C₆-alkylthio-C₁-C₆-alkyl, Aryl-C₁-C₆-alkoxy-C₁-C₆-alkyl, C₁-C₆-Alkoxyaryl-C₁-C₆-alkoxy-C₁-C₆-alkyl, Aryl und Heteroaryl, worin die Aryl- und Heteroarylreste gegebenfalls substituiert sind an einer substituierbaren Position mit einem oder mehreren Resten ausgewählt aus Halo, C₁-C₆-Alkylthio, C₁-C₆-Alkylsulfinyl, C₁-C₆-Alkyl, Cyano, C₁-C₆-Haloalkyl, Hydroxyl, C₁-C₆-Alkoxy, C₁-C₆-Hydroxyalkyl und C₁-C₆-Haloalkoxy, worin R² and R³ die in Anspruch 1 definierte Bedeutung haben; oder ein pharmazeutisch verträgliches Salz davon.

3. Verbindung des Anspruchs 1 worin R¹ ausgewählt ist aus C₁-C₆-Alkyl, C₁-C₆-Haloalkyl, C₁-C₆-Hydroxyalkyl, C₁-C₆-Alkoxy-C₁-C₆-alkyl, Aryl-C₁-C₆-alkylsulfonyl, Arylthio-C₁-C₆-alkyl, Heteroaryl-C₁-C₁₀-alkoxy-C₁-C₆-alkyl, Heteroaryloxy-C₁-C₆-alkyl, Heteroarylthio-C₁-C₆-alkyl, Heteroaryl-C₁-C₆-alkylthio-C₁-C₆-alkyl, Aryl-C₁-C₆-alkylthio und Aryl-C₁-C₆-alkoxy; worin R² ausgewählt ist aus Heteroaryl und Aryl, worin die Heteroaryl- und Arylreste substituiert sind an einer substituierbaren Position mit einem oder mehreren Resten ausgewählt aus Hydrido, Halo, C₁-C₆alkylthio, C₁-C₆-Alkylsulfinyl, C₁-C₆-Alkyl, Cyano, Carboxyl, C₁-C₆-Alkoxycarbonyl, C₁-C₆-Haloalkyl, Hydroxyl, C₁-C₆-Alkoxy, C₁-C₆-Hydroxyalkyl, C₁-C₆-Alkoxy-C₁-C₆-alkyl, C₁-C₆-Haloalkoxy, Amino, C₁-C₆-Alkylamino, Phenylamino und Nitro und worin R³ die in Anspruch 2 definierte Bedeutung hat; oder ein pharmazeutisch verträgliches Salz davon.

4. Verbindung des Anspruchs 1, ausgewählt aus Verbindungen und deren pharmazeutisch verträglichen Salzen der Gruppe bestehend aus
5-(3-Chlorphenyl)-4-(4-methylsulfonylphenyl)-2-trifluormethyl-1H-imidazol;
5-(3-Chlor-4-methylphenyl)-4-(4-methylsulfonylphenyl)-2-trifluormethyl-1H-imidazol;
5-(3-Fluorphenyl)-4-(4-methylsulfonylphenyl)-2-trifluormethyl-1H-imidazol;
5-(3-Chlor-4-methoxyphenyl)-4-(4-methylsulfonylphenyl)-2-trifluormethyl-1H-imidazol;
5-(4-Fluorphenyl)-4-(4-methylsulfonylphenyl)-2-trifluormethyl-1H-imidazol;
5-(4-Fluorphenyl)-4-(4-methylsulfonylphenyl)-3-phenoxymethyl-1H-imidazol;
5-(4-Fluorphenyl)-4-(4-methylsulfonylphenyl)-2-(2-phenyl-*trans*-eth-1-en)-1H-imidazol;
2-(2-Benzofuryl)-5-(4-fluorphenyl)-4-(4-methylsulfonylphenyl)-1H-imidazol;
5-(4-Fluorphenyl)-2-(2-furyl)-4-(4-methylsulfonylphenyl)-1H-imidazol;
5-(4-Fluorphenyl)-2-methyl-4-(4-methylsulfonylphenyl)-1H-imidazol;
5-(4-Fluorphenyl)-2-isopropyl-4-(4-methylsulfonylphenyl)-1H-imidazol;
4-[5-(4-Fluorphenyl)-2-hydroxymethyl-4-(4-methylsulfonylphenyl)-1H-imidazol;
2-Benzylthio-5-(4-fluorphenyl)-4-[4-(methylsulfonyl)phenyl]imidazol;
5-(3-Fluor-4-methoxyphenyl)-4-[4-(methytsulfonyl)phenyl]-2-(trifluormethyl)-1H-imidazol;
5-(4-Chlorphenyl)-4-[4-(methylsulfonyl)phenyl]-2-(trifluormethyl)-1H-imidazol;
5-(2-Chlorphenyl)-4-[4-(methylsulfonyl)phenyl]-2-(trifluormethyl)-1H-imidazol;
5-(3,4-Dichlorphenyl)-4-[4-(methylsulfonyl)phenyl]-2-(trifluormethyl)-1H-imidazol;
4-[4-(Methylsulfonyl)phenyl]-5-(2-naphthyl)-2-(trifluormethyl)-1H-imidazol;
5-(4-Methoxyphenyl)-4-[4-(methylsulfonyl)phenyl]-2-(trifluormethyl)-1H-imidazol;
5-(4-Methylphenyl)-4-[4-(methylsulfonyl)phenyl]-2-(trifluormethyl)-1H-imidazol;
5-(2,4-Difluorphenyl)-4-[4-(methylsulfonyl)phenyl]-2-(trifluormethyl)-1H-imidazol;
5-(3,4-Difluorphenyl)-4-[4-(methylsulfonyl)phenyl]-2-(trifluormethyl)-1H-imidazol;
4-[4-(Methylsulfonyl)phenyl]-5-phenyl-2-(trifluormethyl)-1H-imidazol;
5-(4-Fluorphenyl)-2-(3-furyl)-4-(4-methylsulfonylphenyl)-1H-imidazol;
5-(3,5-Dimethylphenyl)-4-[4-(methylsulfonyl)phenyl]-2-(trifluormethyl)-1H-imidazol;
5-(5-Bromthien-2-yl)-4-[4-(methylsulfonyl)phenyl]-2-(trifluormethyl)-1H-imidazol;
N,N-Dimethyl-4-[4-[4-(methylsulfonyl)phenyl]-2-(trifluormethyl)-1H-imidazol-5-yl]benzolamin;
5-(3,5-Dimethyl-4-methoxyphenyl)-4-[4-(methylsulfonyl)phenyl]-2-(trifluormethyl)-1H-imidazol;
5-[3-Fluor-4-(methylthio)phenyl]-4-[4-(methylsulfonyl)phenyl]-2-(trifluormethyl)-1H-imidazol;
5-(3-Chlor-5-methylphenyl)-4-[4-(methylsulfonyl)phenyl]-2-(trifluormethyl)-1H-imidazol;
5-[3-Chlor-4-(methylthio)phenyl]-4-[4-(methylsulfonyl)phenyl]-2-(trifluormethyl)-1H-imidazol;
5-(3-Fluor-4-methylphenyl)-4-[4-(methylsulfonyl)phenyl]-2-(trifluormethyl)-1H-imidazol;
5-(3-Methylphenyl)-4-[4-(methylsulfonyl)phenyl]-2-trifluormethyl-1H-imidazol;
4-[4-(Methylsulfonyl)phenyl]-5-(3-pyridyl)-2-(trifluormethyl)-1H-imidazol;
5-(2-Fluorphenyl)-4-[4-(methylsulfonyl)phenyl]-2-(trifluormethyl)-1H-imidazol;
5-(3-Methoxyphenyl)-4-[4-(methylsulfonyl)phenyl]-2-(trifluormethyl)-1H-imidazol;
5-(2-Methylphenyl)-4-[4-(methylsulfonyl)phenyl]-2-(trifluormethyl)-1H-imidazol;
5-(Cyclohexyl)-4-[4-(methylsulfonyl)phenyl]-2-(trifluormethyl)-1H-imidazol;
4-[4-(Methylsulfonyl)phenyl]-5-(2-thiophenyl]-2-(trifluormethyl)-1H-imidazol;
5-(4-Chlor-3-methylphenyl)-4-[4-(methylsulfonyl)phenyl]-2-(trifluormethyl)-1H-imidazol;
5-(4-Cycloheptyl)-4-[4-(methylsulfonyl)phenyl]-2-(trifluormethyl)-1H-imidazol;
5-(4-Fluor-3-methylphenyl)-4-[4-(methylsulfonyl)phenyl]-2-(trifluormethyl)-1H-imidazol;
5-(Cyclopentyl)-4-[4-(methylsulfonyl)phenyl]-2-(trifluormethyl)-1H-imidazol;
4-[4-(Methylsulfonyl)phenyl]-5-(3-thiophenyl)-2-(trifluormethyl)-1H-imidazol;
5-(3-Fluor-2-methylphenyl)-4-[4-(methylsulfonyl)phenyl]-2-(trifluormethyl)-1H-imidazol;
5-(4-Chlor-3-methoxyphenyl)-4-[4-(methylsulfonyl)phenyl]-2-(trifluormethyl)-1H-imidazol;
5-(4-Fluorphenyl)-2-(methoxymethyl)-4-[4-(methylsulfonyl)-phenyl]-1H-imidazol.

5. Verbindung des Anspruchs 1 ausgewählt aus Verbindungen und deren pharmazeutisch verträglichen Salzen der Gruppe bestehend aus
4-[5-(4-Fluorphenyl)-2-(trifluormethyl)-1H-imidazol-4-yl]benzolsulfonamid;
4-[5-(4-Fluorphenyl)-2-phenoxymethyl-1H-imidazol-4-yl]benzolsulfonamid;
4-[5-(4-Fluorphenyl)-2-(2-phenyl-*trans*-eth-1-en)-1Himidazol-4-yl]benzolsulfonamid;
4-[2-(2-Benzofuryl)-5-(4-fluorphenyl)-1H-imidazol-4-yl]benzolsulfonamid;
4-[5-(4-Fluorphenyl)-3-isopropyl-1H-imidazol-4-yl]benzolsulfonamid;
4-[5-(4-Fluorphenyl)-2-methyl-1H-imidazol-4-yl]benzolsulfonamid;
4-[5-(4-Fluorphenyl)-2-(2-furyl)-1H-imidazol-4-yl]benzolsulfonamid;
4-[2-Benzylthio-5-(4-fluorphenyl)-1H-imidazol-4-yl]benzolsulfonamid;
4-[5-(4-Fluorphenyl)-2-hydroxymethyl-1H-imidazol-4-yl]benzolsulfonamid;
4-[5-(3-Fluor-4-methoxyphenyl)-2-(trifluormethyl)-1Himidazol-4-yl]benzolsulfonamid;
4-[5-(4-Chlorphenyl)-2-(trifluormethyl)-1H-imidazol-4-yl]benzolsulfonamid;
4-[5-(2-Chlorphenyl)-3-(trifluormethyl)-1H-imidazol-4-yl]benzolsulfonamid;
4-[5-(3,4-Dichlorphenyl)-2-(trifluormethyl)-1Himidazol-4-yl]benzolsulfonamid;
4-[5-(2-Naphthyl)-2-(trifluormethyl)-1H-imidazol-4-yl]benzolsulfonamid;
4-[5-(4-Methoxyphenyl)-2-(trifluormethyl)-1H-imidazol-4-yl]benzolsulfonamid;
4-[5-(4-Methylphenyl)-2-(trifluormethyl)-1H-imidazol-4-yl]benzolsulfonamid;
4-[5-(2,4-Difluorphenyl)-2-(trifluormethyl)-1H-imidazol-4-yl]benzolsulfonamid;
4-[5-(3,4-Difluorphenyl)-2-(trifluormethyl)-1H-imidazol-4-yl]benzolsulfonamid;
4-[5-Phenyl-2-(trifluormethyl)-1H-imidazol-4-yl]benzolsulfonamid und
4-[5-(4-Fluorphenyl)-2-(3-furyl)-1H-imidazol-4-yl]benzolsulfonamid.

6. Eine pharmazeutische Zusammensetzung, enthaltend eine therapeutisch wirksame Menge einer Verbindung, wobei diese Verbindung ausgewählt ist aus einer Familie von Verbindungen gemäß einem der Ansprüche 1 - 5 oder ein pharmazeutisch verträgliches Salz davon.

7. Verwendung einer Verbindung einer der Ansprüche 1, 2, 3, 4 oder 5 oder ein pharmazeutisch verträgliches Salz davon zur Herstellung eines Medikamentes zur Behandlung von Entzündung oder einer entzündungsbedingten Störung in einem Subjekt.

8. Verwendung gemäß Anspruch 7, worin die entzündungsbedingte Störung Arthritis ist.

9. Verwendung gemäß Anspruch 7, worin die entzündungsbedingte Störung Schmerz ist.

10. Verwendung gemäß Anspruch 7, worin die entzündungsbedingte Störung Fieber ist.

## Revendications

1. Composé de formule I : 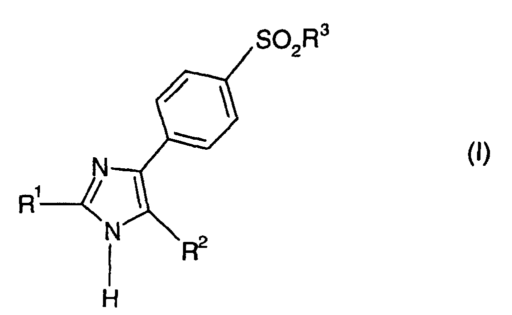 dans laquelle R¹ est choisi parmi alkyle en C₁-C₁₀, haloalkyle en C₁-C₆, (aryl)alkyle en C₁-C₆, (hétérocyclyl)alkyle en C₁-C₆, (hétéroaryl)alkyle en C₁-C₆, formyle, cyano, mercapto, alkoxy en C₁-C₆, alkylthio en C₁-C₆, (alkylthio en C₁-C₆)alkyle en C₁-C₆, alkylsulfonyle en C₁-C₆, haloalkylsulfonyle en C₁-C₆, halo, hydroxyalkyle en C₁-C₆, (alkoxy en C₁-C₆)alkyle en C₁-C₆, (alcényloxy en C₂-C₆)alkyle en C₁-C₆, (alkyl en C₁-C₆)carbonyle, arylcarbonyle, aryl(alkyl en C₁-C₆)carbonyle, cyanoalkyle en C₁-C₆, aryl(alcényle en C₂-C₆), aminoalkyle en C₁-C₆, (alkyl en C₁-C₆)aminoalkyle en C₁-C₆, N-arylaminoalkyle en C₁-C₆, N-(alkyl en C₁-C₆)-N-arylaminoalkyle en C₁-C₆, carboxy(alkyle en C₁-C₆), (alkoxy en C₁-C₆)carbonylalkyle en C₁-C₆, (alkoxy en C₁-C₆)carbonyle, (haloalkyl en C₁-C₆)carbonyle, carboxyle, aminocarbonyle, (alkyl en C₁-C₆)aminocarbonyle, mono- ou di(alkyl en C₁-C₆)aminocarbonylalkyle en C₁-C₆, aryl(alkoxy en C₁-C₆), aryl (alkyl en C₁-C₆)thio, phénylsulfonyle, hétéroaryl(alkoxy en C₁-C₆), hétéroaryl(alkyl en C₁-C₆)thio, hétéroaryl(alkoxy en C₁-C₆)alkyle en C₁-C₆, hétéroaryloxy(alkyle en C₁-C₆), hétéroarylthio(alkyle en C₁-C₆), hétéroaryl(alkylthio en C₁-C₆)alkyle en C₁-C₆, hétéroaryloxy, hétéroarylthio, aryl(thioalkyle en C₁-C₆), aryloxy(alkyle en C₁-C₆), arylthio, aryloxy, aryl(alkylthio en C₁-C₆)alkyle en C₁-C₆, aryl(alkoxy en C₁-C₆)alkyle en C₁-C₆, (alkoxy en C₁-C₆)aryl(alkoxy en C₁-C₆)alkyle en C₁-C₆, aryle et hétéroaryle, où les radicaux aryle et hétéroaryle sont le cas échéant, substitués en une position substituable avec un ou plusieurs radicaux choisis parmi halo, alkylthio en C₁-C₆, alkylsulfinyle en C₁-C₆, alkyle en C₁-C₁₀, cyano, haloalkyle en C₁-C₆, hydroxyle, alkoxy en C₁-C₆, hydroxyalkyle en C₁-C₆ et haloalkoxy en C₁-C₆ ; dans laquelle R² est choisi parmi hétéroaryle et aryle, où les radicaux aryle et hétéroaryle sont substitués avec un ou plusieurs radicaux choisis parmi hydrido, halo, alkylthio en C₁-C₆, alkylsulfinyle en C₁-C₆, alkyle en C₁-C₆, cyano, carboxyle, (alkoxy en C₁-C₆)carbonyle, haloalkyle en C₁-C₆, hydroxyle, alkoxy en C₁-C₆, hydroxyalkyle en C₁-C₆, (alkoxy en C₁-C₆)alkyle en C₁-C₆, haloalkoxy en C₁-C₆, amino, mono- et di(alkyl en C₁-C₆)amino, mono- et diarylamino et nitro ; et dans laquelle R³ est choisi parmi alkyle en C₁-C₁₀ et amino ;
dans laquelle aryle signifie phényle ou naphtyle ;
dans laquelle hétérocyclyl/hétérocyclo signifie des radicaux saturés, partiellement saturés ou insaturés à 5 ou 6 membres, contenant un hétéroatome, où l'hétéroatome est choisi parmi l'azote, le soufre et l'oxygène ;
dans laquelle hétéroaryle est un radical hétérocyclique complètement insaturé ;
pourvu que R¹ ne soit pas alkyle en C₁-C₁₀ lorsque R³ est méthyle et lorsque R² est phényle ou phényle substitué par méthyle, méthoxy ou chloro ; et pourvu que R¹ ne soit pas propylsulfonyle ou trifluoroacétyle lorsque R³ est méthyle ;
ou un sel pharmaceutiquement acceptable de celui-ci.

2. Composé selon la revendication 1, dans lequel R¹ est choisi parmi (hétérocyclyl)alkyle en C₁-C₆, (hétéroaryl)alkyle en C₁-C₆, formyle, cyano, mercapto, alkoxy en C₁-C₆, alkylthio en C₁-C₆, (alkylthio en C₁-C₆)-alkyle en C₁-C₆, alkylsulfonyle en C₁-C₆, haloalkylsulfonyle en C₁-C₆, arylsulfonyle, halo, (alcényle en C₂-C₆)alkyle en C₁-C₆, (alkyl en C₁-C₆) carbonyle, benzoyle, aryl(alkyl en C₁-C₆)carbonyle, cyanoalkyle en C₁-C₆, aryl(alcényle en C₂-C₆), aminoalkyle en C₁-C₆, (alkyl en C₁-C₆)aminoalkyle en C₁-C₆, N-arylaminoalkyle en C₁-C₆, N-(alkyl en C₁-C₆)-N-arylaminoalkyle, (alkoxy en C₁-C₆)carbonyle, (haloalkyl en C₁-C₆)carbonyle, carboxyle, (alkyl en C₁-C₆)aminocarbonyle, (alkyl en C₁-C₆)aminocarbonylalkyle en C₁-C₆, aryl(alkoxy en C₁-C₆), aryl(alkyl en C₁-C₆)thio, phénylsulfonyle, hétéroaryl(alkoxy en C₁-C₆), hétéroaryloxy, hétéroarylthio, arylthio(alkyle en C₁-C₆), aryloxy(alkyle en C₁-C₆), arylthio, aryloxy, aryl(alkylthio en C₁-C₆)alkyle en C₁-C₆, aryl(alkoxy en C₁-C₆)alkyle en C₁-C₆, (alkoxy en C₁-C₆)aryl(alkoxy en C₁-C₆)alkyle en C₁-C₆, aryle et hétéroaryle, où les radicaux aryle et hétéroaryle sont le cas échéant, substitués en une position substituable avec un ou plusieurs radicaux choisis parmi halo, alkylthio en C₁-C₆, alkylsulfinyle en C₁-C₆, alkyle en C₁-C₆, cyano, haloalkyle en C₁-C₆, hydroxyle, alkoxy en C₁-C₆, hydroxyalkyle en C₁-C₆, et haloalkoxy en C₁-C₆ ; dans laquelle R² et R³ sont tels que définis à la revendication 1 ;
ou un sel pharmaceutiquement acceptable de celui-ci.

3. Composé selon la revendication 1, dans lequel R¹ est choisi parmi alkyle en C₁-C₆, haloalkyle en C₁-C₆, hydroxyalkyle en C₁-C₆, (alkoxy en C₁-C₆)alkyle en C₁-C₆, aryl(alkylsulfonyle en C₁-C₆), arylthioalkyle en C₁-C₆, hétéroaryl(alkoxy en C₁-C₁₀)alkyle en C₁-C₆, hétéroaryloxy(alkyle en C₁-C₆), hétéroarylthio(alkyle en C₁-C₆), hétéroaryl(alkylthio en C₁-C₆)alkyle en C₁-C₆, aryl(alkylthio en C₁-C₆) et aryl(alkoxy en C₁-C₆) ; dans laquelle R² est choisi parmi hétéroaryle et aryle, où les radicaux aryle et hétéroaryle sont substitués en une position substituable avec un ou plusieurs radicaux choisis parmi hydrido, halo, alkylthio en C₁-C₆, alkylsulfinyle en C₁-C₆, alkyle en C₁-C₆, cyano, carboxyle, (alkoxy en C₁-C₆)carbonyle, haloalkyle en C₁-C₆, hydroxyle, alkoxy en C₁-C₆, hydroxyalkyle en C₁-C₆, (alkoxy en C₁-C₆)alkyle en C₁-C₆, haloalkoxy en C₁-C₆, amino, (alkyl en C₁-C₆)amino, phénylamino et nitro ; et dans laquelle R³ est tel que défini à la revendication 2 ;
ou un sel pharmaceutiquement acceptable de celui-ci.

4. Composé selon la revendication 1, choisi parmi les composés et leurs sels pharmaceutiquement acceptables, du groupe consistant en :
le 5-(3-chlorophényl)-4-(4-méthylsulfonylphényl)-2-trifluorométhyl-1H-imidazole ;
le 5-(3-chloro-4-méthylphényl)-4-(4-méthylsulfonylphényl)-2-trifluorométhyl-1H-imidazole ;
le 5-(3-fluorophényl)-4-(4-méthylsulfonylphényl)-2-trifluorométhyl-1H-imidazole ;
le 5-(3-chloro-4-méthoxyphényl)-4-(4-méthylsulfonylphényl)-2-trifluorométhyl-1H-imidazole ;
le 5-(4-fluorophényl)-4-(4-méthylsulfonylphényl)-2-trifluorométhyl-1H-imidazole ;
le 5-(4-fluorophényl)-4-(4-méthylsulfonylphényl)-2-phénoxyméthyl-1H-imidazole ;
le 5-(4-fluorophényl)-4-(4-méthylsulfonylphényl)-2-(2-phényl-trans-éth-1-ène)-1H-imidazole ;
le 2-(2-benzofuryl)-5-(4-fluorophényl)-4-(4-méthylsulfonylphényl)-1H-imidazole ;
le 5-(4-fluorophényl)-2-(2-furyl)-4-(4-méthylsulfonylphényl)-1H-imidazole ;
le 5-(4-fluorophényl)-2-méthyl-4-(4-méthylsulfonylphényl)-1H-imidazole ;
le 5-(4-fluorophényl)-2-isopropyl-4-(4-méthylsulfonylphényl)-1H-imidazole ;
le 5-(4-fluorophényl)-2-hydroxyméthyl-4-(4-méthylsulfonylphényl)-1H-imidazole ;
le 2-benzylthio-5-(4-fluorophényl)-4-(4-méthylsulfonylphényl)-1H-imidazole ;
le 5-(3-fluoro-4-méthoxyphényl)-4-(4-méthylsulfonylphényl)-2-trifluorométhyl-1H-imidazole ;
le 5-(4-chlorophényl)-4-(4-méthylsulfonylphényl)-2-trifluorométhyl-1H-imidazole ;
le 5-(2-chlorophényl)-4-(4-méthylsulfonylphényl)-2-trifluorométhyl-1H-imidazole ;
le 5-(3,4-dichlorophényl)-4-(4-méthylsulfonylphényl)-2-trifluorométhyl-1H-imidazole ;
le 4-(4-méthylsulfonylphényl)-5-(2-naphtyl)-2-trifluorométhyl-1H-imidazole ;
le 5-(4-méthoxyphényl)-4-(4-méthylsulfonylphényl)-2-trifluorométhyl-1H-imidazole ;
le 5-(4-méthylphényl)-4-(4-méthylsulfonylphényl)-2-trifluorométhyl-1H-imidazole ;
le 5- (2,4-difluorophényl)-4-(4-méthylsulfonylphényl)-2-trifluorométhyl-1H-imidazole ;
le 5-(3,4-difluorophényl)-4-(4-méthylsulfonylphényl)-2-trifluorométhyl-1H-imidazole ;
le 4-(4-méthylsulfonylphényl)-5-phényl-2-trifluorométhyl-1H-imidazole ;
le 5-(4-fluorophényl)-2-(3-furyl)-4-(4-méthylsulfonylphényl)-1H-imidazole ;
le 5-(3,5-diméthylphényl)-4-(4-méthylsulfonylphényl)-2-trifluorométhyl-1H-imidazole ;
le 5-(5-bromothièn-2-yl)-4-(4-méthylsulfonylphényl)-2-trifluorométhyl-1H-imidazole ;
la N,N-diméthyl-4-[4-(4-méthylsulfonylphényl)-2-trifluorométhyl-1H-imidazol-5-yl]benzanamine ;
le 5-(3,5-diméthyl-4-méthoxyphényl)-4-(4-méthylsulfonylphényl)-2-trifluorométhyl-1H-imidazole ;
le 5-[3-fluoro-4-(méthylthio)phényl]-4-(4-méthylsulfonylphényl)-2-trifluorométhyl-1H-imidazole ;
le 5-(3-chloro-5-méthylphényl)-4-(4-méthylsulfonylphényl)-2-trifluorométhyl-1H-imidazole ;
le 5-[3-chloro-4-(méthylthio)phényl]-4-(4-méthylsulfonylphényl)-2-trifluorométhyl-1H-imidazole ;
le 5-(3-fluoro-4-méthylphényl)-4-(4-méthylsulfonylphényl) -2-trifluorométhyl-1H-imidazole ;
le 5-(3-méthylphényl)-4-(4-méthylsulfonylphényl)-2-trifluorométhyl-1H-imidazole ;
le 4-(4-méthylsulfonylphényl)-5-(3-pyridyl)-2-trifluorométhyl-1H-imidazole ;
le 5-(2-fluorophényl)-4-(4-méthylsulfonylphényl)-2-trifluorométhyl-1H-imidazole ;
le 5-(3-méthoxyphényl)-4-(4-méthylsulfonylphényl)-2-trifluorométhyl-1H-imidazole ;
le 5-(2-méthylphényl)-4-(4-méthylsulfonylphényl)-2-trifluorométhyl-1H-imidazole ;
le 5-(cyclohexyl)-4-(4-méthylsulfonylphényl)-2-trifluorométhyl-1H-imidazole ;
le 4-(4-méthylsulfonylphényl)-5-(2-thiophényl)-2-trifluorométhyl-1H-imidazole ;
le 5-(4-chloro-3-méthylphényl)-4-(4-méthylsulfonylphényl)-2-trifluorométhyl-1H-imidazole ;
le 5-(4-cycloheptyl)-4-(4-méthylsulfonylphényl)-2-trifluorométhyl-1H-imidazole ;
le 5-(4-fluoro-3-méthylphényl)-4-(4-méthylsulfonylphényl)-2-trifluorométhyl-1H-imidazole ;
le 5-(cyclopentyl)-4-(4-méthylsulfonylphényl)-2-trifluorométhyl-1H-imidazole ;
le 4-(4-méthylsulfonylphényl)-5-(3-thiophényl)-2-trifluorométhyl-1H-imidazole ;
le 5-(3-fluoro-2-méthylphényl)-4-(4-méthylsulfonylphényl)-2-trifluorométhyl-1H-imidazole ;
le 5-(4-chloro-3-méthoxyphényl)-4-(4-méthylsulfonylphényl)-2-trifluorométhyl-1H-imidazole ;
le 5-(4-fluorophényl)-2-méthoxyméthyl-4-(4-méthylsulfonylphényl)-1H-imidazole.

5. Composé selon la revendication 1, choisi parmi les composés et leurs sels pharmaceutiquement acceptables, du groupe consistant en :
le 4-[5- (4-fluorophényl)-2-(trifluorométhyl)-1H-imidazol-4-yl]benzènesulfonamide ;
le 4-[5-(4-fluorophényl)-2-(phénoxyméthyl)-1H-imidazol-4-yl]benzènesulfonamide ;
le 4-[5-(4-fluorophényl)-2-(2-phényl-trans-éth-1-ène)-1H-imidazol-4-yl]benzènesulfonamide ;
le 4-[2-(2-benzofuryl) -5-(4-fluorophényl)-1H-imidazol-4-yl]benzènesulfonamide ;
le 4-[5-(4-fluorophényl)-2-isopropyl-1H-imidazol-4-yl]benzènesulfonamide ;
le 4-[5-(4-fluorophényl)-3-méthyl-1H-imidazol-4-yl]benzènesulfonamide;
le 4-[5-(4-fluorophényl)-2-(2-furyl)-1H-imidazol-4-yl]benzènesulfonamide ;
le 4-[2-benzylthio-5-(4-fluorophényl)-1H-imidazol-4-yl]benzènesulfonamide ;
le 4-[5-(4-fluorophényl)-2-(hydroxyméthyl)-1H-imidazol-4-yl]benzènesulfonamide ;
le 4-[5-(3-fluoro-4-méthoxyphényl)-2-(trifluorométhyl)-1H-imidazol-4-yl]benzènesulfonamide ;
le 4-[5-(4-chlorophényl)-2-(trifluorométhyl)-1H-imidazol-4-yl]benzènesulfonamide ;
le 4-[5-(2-chlorophényl)-3-(trifluorométhyl)-1H-imidazol-4-yl]benzènesulfonamide ;
le 4-[5- (3,4-dichlorophényl)-2-(trifluorométhyl)-1H-imidazol-4-yl]benzènesulfonamide ;
le 4-[5- (2-naphtyl)-2-(trifluorométhyl)-1H-imidazol-4-yl]benzènesulfonamide ;
le 4-[5- (4-méthoxyphényl)-2-(trifluorométhyl)-1H-imidazol-4-yl]benzènesulfonamide ;
le 4-[5-(4-méthylphényl)-2-(trifluorométhyl)-1H-imidazol-4-yl]benzènesulfonamide ;
le 4-[5-(2,4-difluorophényl)-2-(trifluorométhyl)-1H-imidazol-4-yl]benzènesulfonamide ;
le 4-[5-(3,4-difluorophényl)-2-(trifluorométhyl)-1H-imidazol-4-yl]benzènesulfonamide ;
le 4-[5-phényl-2-(trifluorométhyl)-1H-imidazol-4-yl]benzènesulfonamide, et
le 4-[5-(4-fluorophényl)-2-(3-furyl)-1H-imidazol-4-yl]benzènesulfonamide.

6. Composition pharmaceutique comprenant une quantité thérapeutiquement efficace d'un composé, ledit composé étant choisi parmi une famille de composés selon l'une quelconque des revendications 1 à 5, ou un sel pharmaceutiquement acceptable de ceux-ci.

7. Utilisation d'un composé selon l'une quelconque des revendications 1, 2, 3, 4 ou 5, ou d'un sel pharmaceutiquement acceptable de celui-ci, pour préparer un médicament pour traiter l'inflammation ou un trouble associé à l'inflammation chez un patient.

8. Utilisation selon la revendication 7, dans laquelle le trouble associé à l'inflammation est l'arthrite.

9. Utilisation selon la revendication 7, dans laquelle le trouble associé à l'inflammation est la douleur.

10. Utilisation selon la revendication 7, dans laquelle le trouble associé à l'inflammation est la fièvre.
